# EUROPEAN PATENT APPLICATION

(11) **EP 4 609 882 A1**
(43) Date of publication of application: **03.09.2025**
(21) Application number: 23877312.1
(22) Date of filing: 11.10.2023
(51) Int. Cl.: A61K 48/00, C12N 15/35, C12N 15/864, C07K 14/015

(54) **GENE INTRODUCTION METHOD, GENE THERAPY METHOD, AND TISSUE REGENERATION METHOD**

(30) Priority: 12.10.2022 US 202263415320 P; 20.07.2023 US 202363514586 P
(71) Applicant: CPC Corporation, Tokyo 101-0062 (JP)
(72) Inventor: KURITA, Masakazu, Tokyo 1130022 (JP); KATO, Motoi, Okayama 7000913 (JP); SHEN, Qi, Tokyo 1130033 (JP); HIRABAYASHI, Yusuke, Tokyo 1140015 (JP); SUGA, Shogo, Tokyo 1120001 (JP); SUZUKI, Keiichiro, Osaka 5630032 (JP); DU, Zening, Tokyo 1130031 (JP); HOJO, Hironori, Tokyo 1150043 (JP); OKADA, Hiroyuki, Tokyo 1130033 (JP)
(74) Representative: Grünecker Patent- und Rechtsanwälte PartG mbB
(86) International application number: PCT/JP2023/036900
(87) International publication number: WO 2024/080311

(57) **Abstract**

[Problem] There is no gene transduction method with high efficiency and high specificity for skin, subcutaneous tissue, skin ulcer surfaces, and their constituent cells. More generally, achieving high gene transduction efficiency in vivo is challenging. Additionally, it is difficult to morphologically and functionally regenerate skin appendages and to regenerate lost peripheral tissues of the upper and lower limbs (upper arm, forearm, hand, fingers, thigh, lower leg, foot, toes).

[Solution]This invention provides solutions to these challenges through a novel AAV (adeno-associated virus) capsid with high gene transduction efficiency and high specificity for skin, subcutaneous tissue, and skin ulcer surface cells, as well as a novel AAV capsid with high gene transduction efficiency and high specificity for epidermal tissue and its constituent keratinocytes. Additionally, the invention provides a novel tissue-specific promoter with high gene expression efficiency and high specificity for epidermal tissue and its constituent keratinocytes, and a pharmaceutical composition that enhances gene transduction efficiency by co-administering steroids alongside in vivo gene transduction. The invention further includes a pharmaceutical composition that promotes the regeneration, neogenesis, and functional enhancement of skin appendages through in vivo gene transduction, thereby compensating for functional impairments of skin appendages. Additionally, it provides a research and development platform for developing methods to regenerate peripheral and missing tissues of the upper and lower limbs, as well as a pharmaceutical composition for achieving tissue regeneration. Thus, the present invention provides methods for overcoming these challenges.

## Description

### Field of the Invention

The present invention relates to gene therapy methods, gene transduction methods, tissue regeneration induction methods, rejuvenation induction methods, and genome editing methods. Furthermore, it pertains to their use as therapeutic methods by applying the aforementioned gene therapy methods, gene transduction methods, tissue regeneration induction methods, tissue rejuvenation induction methods, and genome editing methods to clinical pathological conditions or diseases, as well as their utilization as research and development platforms for therapeutic methods and biological research.

### Background Art

Gene transduction technologies that transduce nucleic acids such as DNA and RNA into cells in vivo or in vitro are widely utilized as methods for treating pathological conditions or genetic disorders that are challenging to address with conventional treatments, as well as for tissue regeneration in regenerative medicine. Additionally, these technologies are extensively employed as tools in drug discovery and biological research.

To use gene transduction for gene therapy, its development, drug discovery, or as a method in biological research, achieving higher gene expression in a greater number of cells-i.e., attaining high gene transduction efficiency-is often required.

Methods for gene transduction into animal cells include viral vector methods using adeno-associated virus (AAV) vectors, adenovirus vectors, retrovirus vectors, lentivirus vectors, Sendai virus vectors, and others. Additionally, methods using plasmids or minicircle DNA in their naked state, calcium phosphate methods, lipofection methods, and electroporation methods are utilized. Lipid nanoparticles (LNPs) are employed for RNA delivery.

In particular, achieving high gene transduction efficiency into target organs, tissues, or cells in vivo is often challenging. Therefore, achieving higher efficiency in gene transduction is a critical requirement for utilizing gene transduction as a therapeutic method and for its development.

Among others, no outstanding gene transduction methods are available for the skin and skin ulcer regions. While research and development efforts are progressing for treatments for genetic skin disorders (refer to Non-Patent Document 1) and tissue regeneration therapies for skin ulcers (refer to Non-Patent Documents 2 and 3), there remains a need for methods that enable high gene transduction efficiency and tissue-specific gene expression.

Skin appendages, including hair follicles, sebaceous glands, and sweat glands, play roles such as protection from mechanical damage, insulation, moisturizing, and temperature regulation. Skin appendages are formed during the fetal stage through interactions between epithelial tissues and mesenchymal tissues during organogenesis (refer to Non-Patent Document 4).

Representative pathological conditions caused by defects, damage, or dysfunction of skin appendages include alopecia and sebaceous deficiency. These conditions can lead to thermoregulatory disorders and itching due to skin dryness, as well as cosmetic impairments.

Clinically, regenerating lost skin appendages is challenging, often requiring transplantation of existing appendages through skin grafting or flap surgery from other parts of the body.

In contrast, a method exists to produce cells with skin appendage-inducing ability by transducing a transgene containing at least one gene relatively strongly expressed in cells with such ability into somatic cells lacking this capability. These cells can then be used as a source for treating defects, damage, or dysfunction of skin appendages (refer to Patent Document 1). Additionally, a method has been reported for inducing skin without appendages by introducing a gene into somatic cells in vivo (refer to Non-Patent Document 1). However, no reports describe a method for inducing skin tissue with appendages from somatic cells in vivo by gene transduction.

The upper limbs, consisting of the upper arm, forearm, hand, and fingers, and the lower limbs, consisting of the thigh, leg, foot, and toes, are complex tissues composed of bones, cartilage, muscles, tendons, fat, peripheral nerves, blood vessels, and skin.

In mammals, including humans, it is clinically difficult to regenerate tissue loss at any level. As a result, there are no better therapeutic options than substituting functionality and aesthetics by using artificial devices such as prosthetic limbs, prosthetic legs, or prostheses.

Furthermore, even in cases where individual tissues such as bone, cartilage, muscle, or fat are missing within the structures of the upper or lower limbs, clinical regeneration remains challenging, and tissue transplantation from other parts of the body is often required.

### Prior Art Documents

### Patent Document

Patent Document 1. International Publication No. 2022/244502

### Non-Patent Document

Non-Patent Document 1. Ain QU, Campos EVR, Huynh A, Witzigmann D, Hedtrich S. Gene Delivery to the Skin - How Far Have We Come? "Trends Biotechnol", 2021, 39(5), p474-487.
Non-Patent Document 2. Kurita M, Araoka T, Hishida T, O'Keefe DD, Takahashi Y, Sakamoto A, Sakurai M, Suzuki K, Wu J, Yamamoto M, Hernandez-Benitez R, Ocampo A, Reddy P, Shokhirev MN, Magistretti P, Núnez Delicado E, Eto H, Harii K, Izpisua Belmonte JC. "Nature", 2018, 561(7722), p243-247.
Non-Patent Document 3. Kato M, Ishikawa S, Shen Q, Du Z, Katashima T, Naito M, Numahata T, Okazaki M, Sakai T, Kurita M. "Commun Biol", 2023, 6(1), p508.
Non-Patent Document 4. Lee J, Böscke R, Tang PC, Hartman BH, Heller S, Koehler KR. "Cell Research", 2018, 22(1), p242-254.

### Summary of the Invention

The present invention comprises methods for gene transduction with high efficiency and specificity for the skin, subcutaneous tissues, and their constituent cells; methods for improving gene transduction efficiency in vivo; methods for regenerating skin appendages using gene transduction; and methods for regenerating missing distal portion of limb extremities. These methods can be utilized as therapeutic approaches aimed at disease treatment, condition improvement, and health promotion through gene transduction. Additionally, they can serve as tools for researching and developing gene transduction-based therapies. Furthermore, they can be used as research tools in drug discovery and biological studies unrelated to the development of gene transduction therapies.

### Problems to Be Solved by the Invention

The first problem to be solved is the absence of a gene transduction method with high efficiency and specificity for the skin, subcutaneous tissues, ulcerated skin surfaces, and their constituent cells.

The second problem is the general difficulty in achieving high gene transduction efficiency and high expression of target genes during in vivo gene transduction.

The third problem is the difficulty of morphologically and functionally regenerating lost skin appendages in conditions caused by defects, damage, dysfunction, or aging-related physiological changes.

The fourth problem is the difficulty of morphologically and functionally regenerating missing peripheral tissues of upper and lower limbs (such as the arm, forearm, hand, fingers, thigh, leg, foot, and toes).

### Solution to Problem

The present invention is characterized primarily by novel AAV capsids with high gene transduction efficiency and specificity for the skin, subcutaneous tissues, ulcerated skin surfaces, and their constituent cells. These include: novel AAV capsids with high gene transduction efficiency and specificity for cells in the skin, subcutaneous tissues, and ulcerated skin surfaces; novel AAV capsids with high gene transduction efficiency and specificity for epidermal tissues and their constituent cells, keratinocytes; novel tissue-specific promoters with high gene expression and specificity for epidermal tissues and their constituent cells, keratinocytes.

The present invention is also characterized by methods for improving gene transduction efficiency and achieving high gene expression across various organs, tissues, and cells in the body. These methods involve the systemic or local administration of steroids before, simultaneously with, or shortly after introducing various gene delivery systems, including AAV, retroviral vectors, lentiviral vectors, adenoviral vectors, Sendai viral vectors, naked DNA such as plasmid DNA or mini-circle DNA, and more recently, mRNA (including those carried by lipid nanoparticles (LNPs)).

The present invention is also characterized by methods for inducing the regeneration, creation, and functional enhancement of skin appendages through in vivo gene transduction. These methods are aimed at addressing conditions such as the loss of skin and skin appendages in pathological states, age-related changes, and various conditions associated with the decline of skin appendage functions, thereby promoting the restoration and fulfillment of skin appendage functions.

The present invention is also characterized by methods for morphologically and functionally regenerating missing peripheral tissues of the upper and lower limbs (such as the upper arm, forearm, hand, fingers, thigh, leg, foot, and toes) and by research and development platforms for developing such regeneration methods.

### Effects of the Invention

AAVDJ1, an AAV with excellent gene transduction efficiency for cells in ulcerated skin surfaces in the present invention, offers advantages of higher gene transduction efficiency and superior specificity compared to AAVDJ and other capsids. These advantages are observed in cells of ulcerated skin surfaces, subcutaneous tissues in vivo, and constituent cells under cultured conditions.

AAVDJ2, an AAV with excellent gene transduction efficiency for keratinocytes and epidermal tissues in the present invention, demonstrates higher gene transduction efficiency and superior specificity compared to AAV2, AAV6, and AAVDJ, which were previously known for their efficiency in keratinocytes. AAVDJ2 offers the advantage of high gene transduction efficiency, superior tropism, and high selectivity for epidermal tissues in vivo and for keratinocytes under cultured conditions.

The K14SCP3 promoter, K16SCP3 promoter, and K16P5 short promoter, which are highly specific promoters for keratinocytes and epidermal tissues in the present invention, provide higher or comparable levels of gene expression compared to the widely known CAG promoter, which is recognized for its high gene expression across various tissues and cell types including keratinocytes and epidermal tissues. However, these promoters exhibit relatively low gene expression in non-keratinocyte or non-epidermal tissues and cells, such as mesenchymal cells near the epidermis. As a result, they offer advantages as tissue-specific promoters for epidermal tissues and cultured keratinocytes.

The method of the present invention, which induces the regeneration, creation, and functional enhancement of skin appendages in vivo through gene transduction, has the advantage of enabling the regeneration, creation, and functional enhancement of skin appendages lost due to defects, damage, dysfunction, or physiological changes such as aging.

The method of the present invention for regenerating peripheral tissues of the upper and lower limbs enables the morphological and functional regeneration of parts of the peripheral tissues in the upper and lower limbs, which has not been achievable in mammals, including humans. This provides the advantage of serving as a research and development platform for developing methods aimed at the complete regeneration of missing tissues, a concept that has not previously existed.

### Brief Description of the Drawings

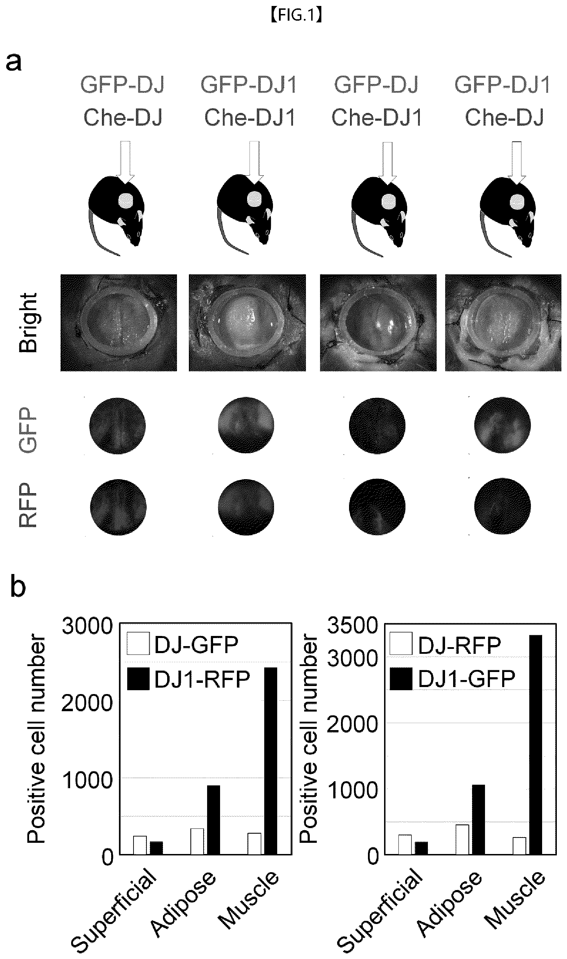

[FIG. 1] This figure presents the results of a comparative study on the gene transduction efficiency of AAVDJ and AAVDJ1 for ulcerated skin surfaces on the backs of mice (Example 1). It demonstrates that AAVDJ1 exhibits higher gene transduction efficiency compared to AAVDJ, particularly in deep tissues such as the fat and muscle layers. Panel (a) provides a schematic of the combinations of transduced genes and capsids administered to each group of mice in the upper section, along with representative photographs captured under visible light and fluorescence microscopy. Panel (b) includes two graphs. The graph on the left shows the number of gene transduction-positive cells in the superficial layer, fat layer, and muscle layer, counted from tissue observations after co-administration of AAVDJ encoding GFPNLS and AAVDJ1 encoding mCherryNLS. The graph on the right presents the results of image analysis to quantify gene transduction-positive cells in the same tissue layers after co-administration of AAVDJ encoding mCherryNLS and AAVDJ1 encoding GFPNLS.

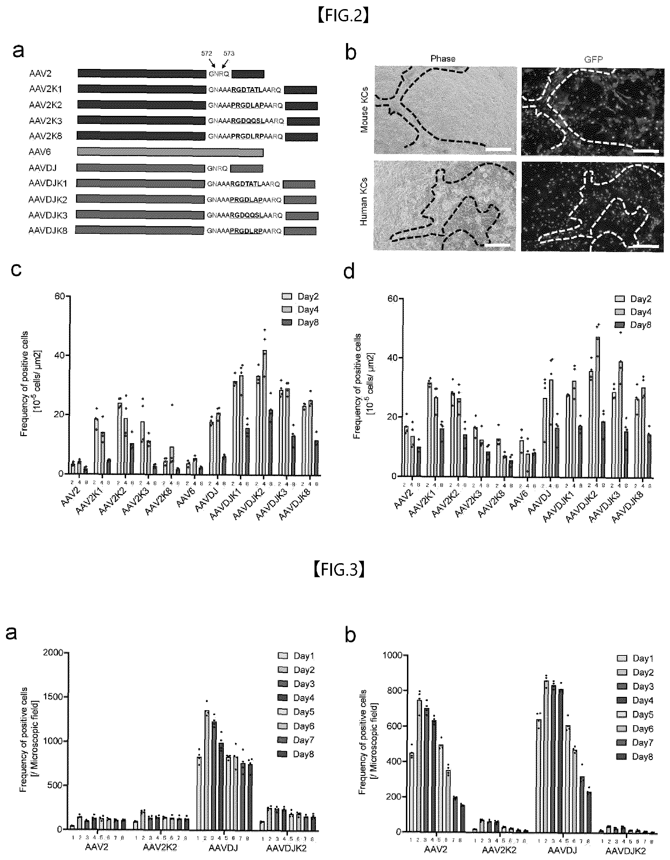

[FIG. 2] This figure presents the results of a comparative study on the gene transduction efficiency of novel AAVDJ variants and known AAVs for mouse and human cultured keratinocytes (Example 2). The novel AAVDJ variants AAVDJK1, AAVDJK2, AAVDJK3, and AAVDJK8 demonstrate higher gene transduction efficiency, with AAVDJK2 exhibiting superior efficiency compared to known AAVs. Panel (a) shows a schematic of the amino acid sequences of known AAVs with high gene transduction efficiency for keratinocytes (AAV2, AAV2K1, AAV2K2, AAV2K3, AAV2K8, AAV6, and AAVDJ) and the novel AAVDJ variants (AAVDJK1, AAVDJK2, AAVDJK3, and AAVDJK8). Panel (b) displays phase-contrast microscopy images of colonies of mouse and human keratinocytes under feeder culture conditions (dotted lines indicate colony boundaries). Panel (c) shows the results of gene transduction experiments in mouse keratinocytes using GFPNLS-expressing vectors prepared with each capsid. The novel AAVDJ variants AAVDJK1, AAVDJK2, AAVDJK3, and AAVDJK8, particularly AAVDJK2, demonstrated higher gene transduction efficiency compared to known AAVs. Panel (d) shows the results of gene transduction experiments in human keratinocytes using GFPNLS-expressing vectors prepared with each capsid. The novel AAVDJ variants AAVDJK1, AAVDJK2, AAVDJK3, and AAVDJK8, particularly AAVDJK2, demonstrated higher gene transduction efficiency compared to known AAVs.
[FIG. 3] This figure presents the results of a comparative study on the gene transduction efficiency of known AAV2, AAV2K2, AAVDJ, and the novel AAVDJK2 for mouse and human cultured mesenchymal cells (Example 3). The study shows that the novel AAVDJK2 does not exhibit the high gene transduction efficiency for mesenchymal cells observed with AAVDJ or AAV2. Panel (a) shows the results of gene transduction experiments in mouse adipose-derived mesenchymal cells using GFPNLS-expressing vectors prepared with each capsid. The novel AAVDJK2 does not exhibit the high gene transduction efficiency for mesenchymal cells observed with AAVDJ. Panel (b) shows the results of gene transduction experiments in human fibroblasts using GFPNLS-expressing vectors prepared with each capsid. The novel AAVDJK2 does not exhibit the high gene transduction efficiency for mesenchymal cells observed with AAVDJ.

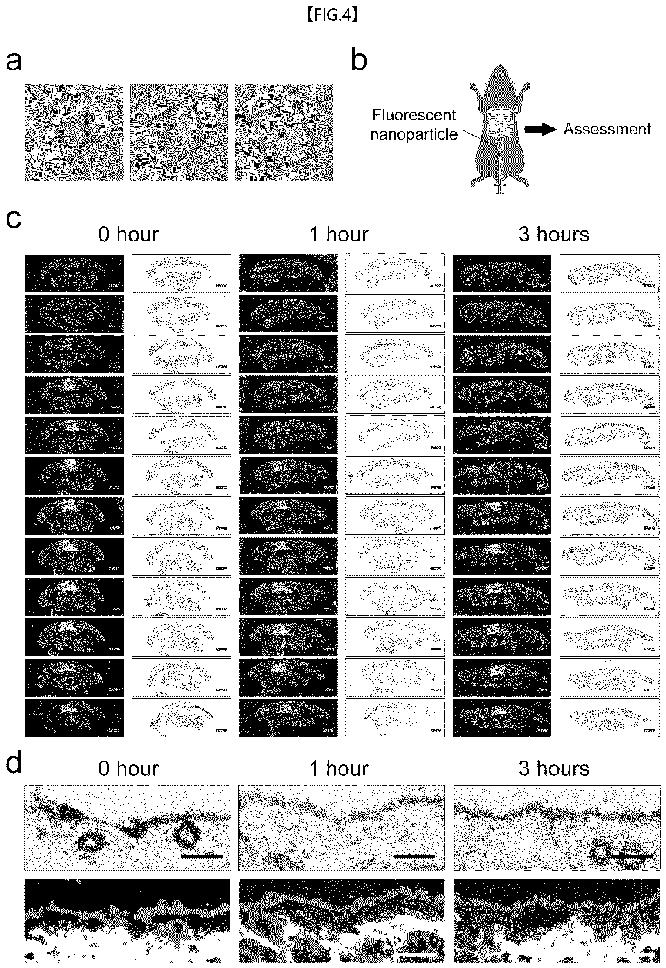

[FIG. 4] This figure shows the results of an injection test of fluorescent silica particles into the dorsal skin of mice (Reference Example 1), demonstrating that most of the injected particles do not distribute within the epidermis following intradermal injection. Panel (a) shows the process of intradermal injection using a surgical microscope. Panel (b) shows a schematic of the injection test of fluorescent silica particles into the dorsal skin of mice. Panel (c) shows the distribution of fluorescent silica particles observed in tissue sections immediately after injection, and 1 hour and 3 hours post-injection, following collection of skin tissues. It is evident that most of the injected particles are distributed from the dermis to the subcutaneous layer. Panel (d) shows a magnified view near the epidermis of the same specimen. It is evident that most of the injected fluorescent silica particles are not distributed within the epidermis.

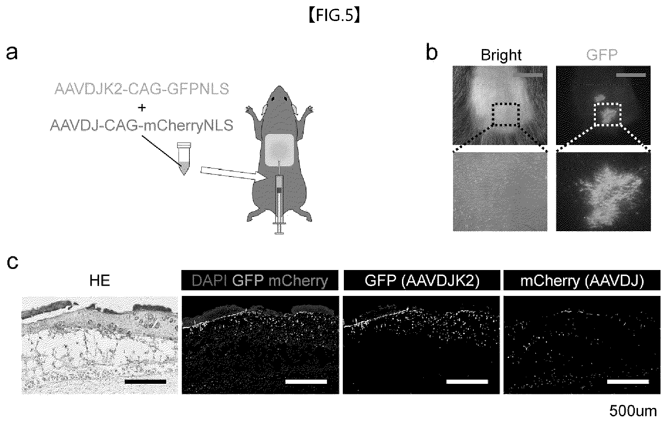

[FIG. 5] This figure presents the results of a comparative study on the gene transduction efficiency of AAVDJK2 and AAVDJ in the dorsal skin of mice, demonstrating that AAVDJK2 exhibits higher gene transduction efficiency and specificity for epidermal tissues compared to AAVDJ. Panel (a) shows a schematic of the experiment. AAVDJK2 expressing GFPNLS and AAVDJ expressing mCherryNLS, both under the control of a CAG promoter, were adjusted to the same titer (10¹¹ GC (gene copies)/35 µl) and combined, which was then injected intradermally into the dorsal skin of mice. Panel (b) shows fluorescence stereomicroscopy images of the injection site two days after the injection, taken during tissue sampling. Panel (c) shows histological observations indicating that GFPNLS transduced by AAVDJK2 is strongly expressed in the epidermis and weakly expressed in the subcutaneous tissue, whereas mCherry transduced by AAVDJ is weakly expressed in the epidermis and strongly expressed in the subcutaneous tissue.

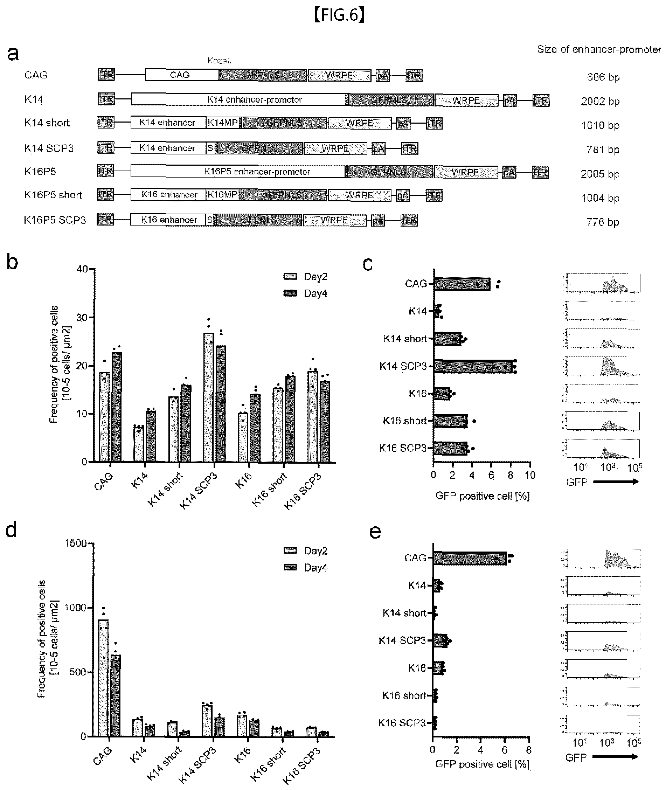

[FIG. 6] This figure presents the results of a comparative study on gene expression conducted using AAV vectors with AAVDJK2 capsids expressing GFPNLS under the control of the novel K14SCP3 promoter, K16SCP3 promoter, K16P5 short promoter, and the known CAG promoter, K14 promoter, K14 short promoter, and K16P5 promoter in mouse and human cultured keratinocytes and mesenchymal cells (Example 5). The results demonstrate that the novel promoters exhibit higher gene transduction efficiency and/or higher specificity for keratinocytes. Panel (a) shows a schematic of the GAPNLS-expressing AAV plasmid sequences constructed with the novel K14SCP3 promoter, K16SCP3 promoter, K16P5 short promoter, and the known CAG promoter, K14 promoter, K14 short promoter, and K16P5 promoter. Panel (b) shows the results of gene transduction experiments in mouse keratinocytes using AAVDJK2 capsid vectors expressing GFPNLS under the control of each promoter. The results demonstrate that the K14SCP3 promoter outperforms the CAG promoter, K14 promoter, and K14 short promoter, and that the K16SCP3 promoter and K16P5 short promoter exhibit higher gene transduction efficiency compared to the K14 promoter, K14 short promoter, and K16P5 promoter. Panel (c) shows the results of gene transduction efficiency analysis conducted using flow cytometry after gene transduction into mouse keratinocytes with AAVDJK2 capsid vectors expressing GFPNLS under the control of each promoter. The results demonstrate that the K14SCP3 promoter outperforms the CAG promoter, K14 promoter, and K14 short promoter. Additionally, the K16SCP3 promoter and K16P5 short promoter exhibit higher gene transduction efficiency compared to the K14 promoter, K14 short promoter, and K16P5 promoter. Panel (d) shows the results of gene transduction experiments in mouse adipose-derived mesenchymal cells using AAVDJK2 capsid vectors expressing GFPNLS under the control of each promoter. The results demonstrate that the gene transduction efficiency of keratin-specific promoters, including the novel K14SCP3 promoter, K16SCP3 promoter, and K16P5 short promoter, is significantly lower than that of the CAG promoter. Panel (e) shows the results of gene transduction efficiency analysis conducted using flow cytometry after gene transduction into mouse adipose-derived mesenchymal cells with AAVDJK2 capsid vectors expressing GFPNLS under the control of each promoter. The results demonstrate that the gene transduction efficiency of keratin-specific promoters, including the novel K14SCP3 promoter, K16SCP3 promoter, and K16P5 short promoter, is significantly lower than that of the CAG promoter.

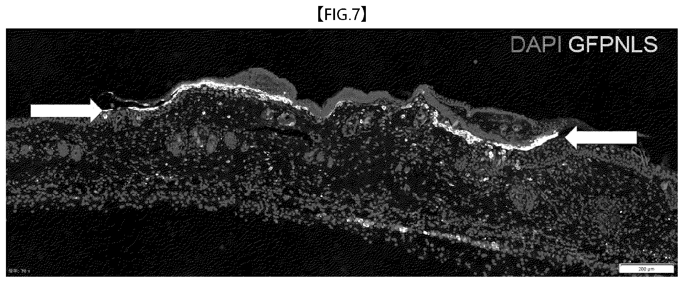

[FIG. 7] This figure shows the results of a gene transduction experiment in which AAVDJK2 expressing GFPNLS under the control of the K14SCP3 promoter was intradermally injected into the dorsal skin of mice. The results demonstrate that the vector achieves high efficiency and specificity in gene transduction to epidermal tissues. On the next day following a subcutaneous injection of 10 µl of steroid (Kenacort injection), AAVDJK2-K14SCP3-GFPNLS was intradermally injected into the dorsal skin. Two days later, tissue observations of the dorsal skin showed strong GFPNLS expression localized to the epidermal tissue (indicated by white arrows). Excluding the epidermal tissue of hair follicles, gene transduction to the dermis and subcutaneous tissue was observed at a low frequency.

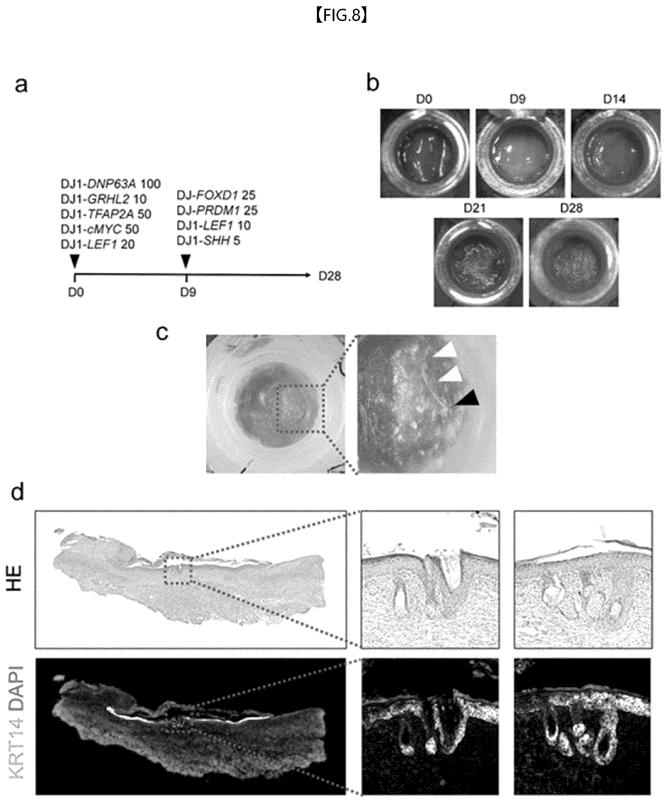

[FIG. 8] This figure depicts an experiment in which gene transduction was performed using AAV on an isolated skin ulcer created by attaching a chamber over the dorsal fascia of a mouse, resulting in the induction of skin with appendages. The results demonstrate that skin with appendages was successfully induced in the ulcer isolated from the surrounding skin. Panel (a) shows a schematic of the experiment. The timeline for gene transduction using AAV is indicated, with the day of chamber attachment and ulcer creation defined as D0. DJ1 and DJ represent the capsids of the AAV used (AAVDJ1 and AAVDJ, respectively). The genes transduced are shown in italics, and the numbers to the right of each virus (5, 10, 20, 25, 50, 100) indicate the titer of the administered AAV (×10¹⁰ GC, gene copies). Panel (b) shows the chronological appearance of the ulcerated area. From D21, the induction of epidermal tissue in the ulcerated area becomes evident. Panel (c) shows a stereomicroscopic image of the ulcerated area on D28. Hair growth is observed (indicated by arrows). Panel (d) shows histological observations. HE represents hematoxylin staining, KRT14 indicates keratin 14 as an epidermal marker, and DAPI stains cell nuclei. The epidermal areas induced in the isolated ulcerated region exhibit the formation of skin appendage structures, including hair follicles and sebaceous glands.

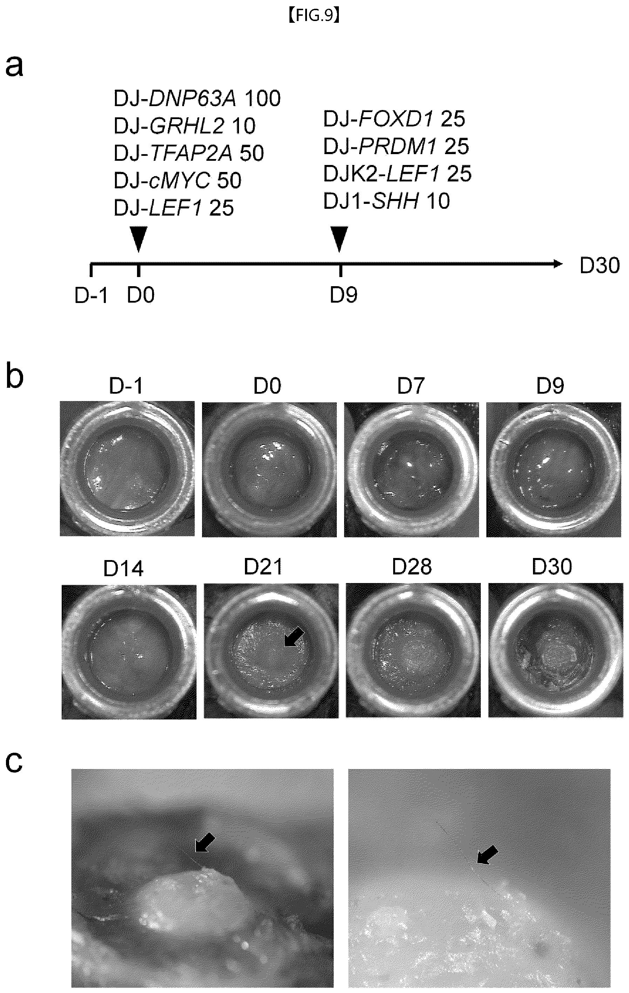

[FIG. 9] This figure illustrates an experiment in which gene transduction was performed using AAV on an isolated skin ulcer created by attaching a chamber over the dorsal fascia of a mouse, leading to the induction of skin with appendages. Black hair growth is observed in the skin ulcer isolated from the surrounding skin. Panel (a) shows a schematic of the experiment. The timeline for gene transduction using AAV is indicated, with the day of chamber attachment and ulcer creation defined as D-1. DJ1, DJ, and DJK2 represent the capsids of the AAV used (AAVDJ1, AAVDJ, and AAVDJK2, respectively). The genes transduced are shown in italics, and the numbers to the right of each virus (10, 25, 50, 100) indicate the titer of the administered AAV (×10¹⁰ GC, gene copies). Panel (b) shows the chronological appearance of the ulcerated area. From D21, the induction of epidermal tissue in the ulcerated area becomes evident (indicated by arrows). Panel (c) shows a stereomicroscopic image of the skin tissue induced in the ulcerated area on D30. Black hair growth is observed (indicated by arrows).

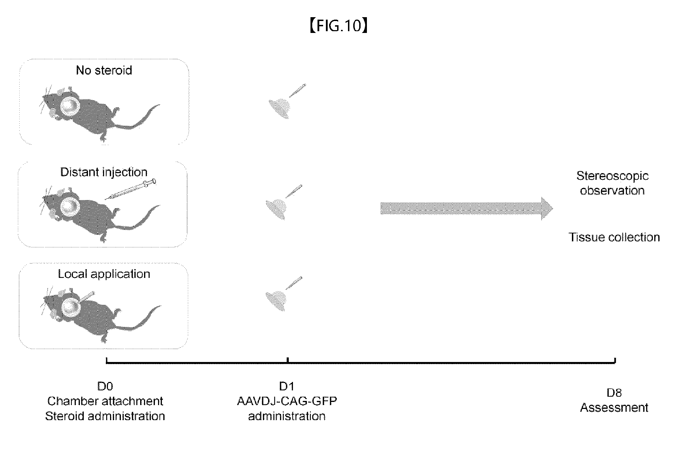

[FIG. 10] This figure shows a schematic of an experiment evaluating the effect of steroids on the gene transduction efficiency of AAV in skin ulcers. On the day before the administration of AAVDJ-CAG-GFP (10¹¹ GC/100 µl), skin ulcers were created on the dorsal skin of mice, and silicone chambers were attached. Three groups were prepared: a no-steroid group, a remote administration group in which 10 µl of Kenacort A (40 mg/ml) was subcutaneously injected into the abdominal region, and a local administration group in which 10 µl of Kenacort was injected into the chamber. On the seventh day after AAV administration, the skin ulcer areas were observed using fluorescence stereomicroscopy, and tissues, including the ulcerated areas, were collected.

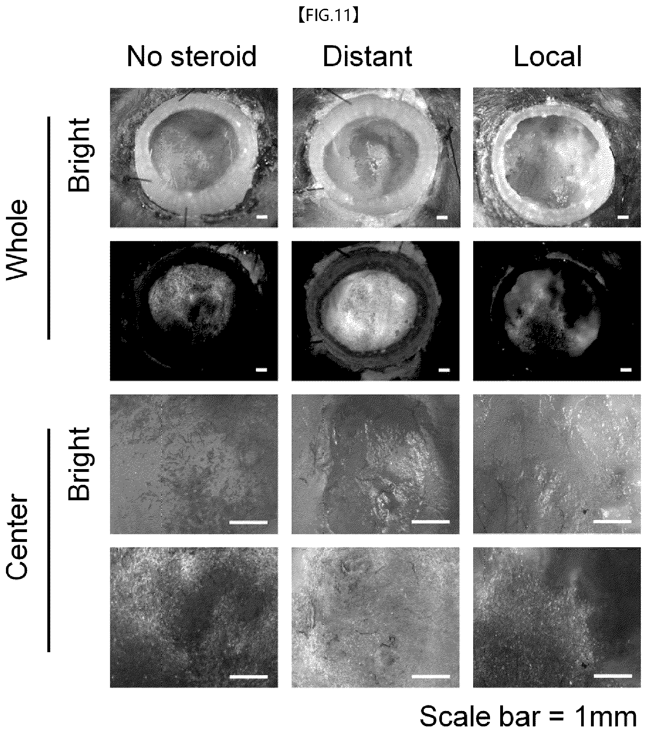

[FIG. 11] This figure shows stereomicroscopic observations of the ulcerated area under visible light and fluorescence on the seventh day after AAV administration, demonstrating strong GFP expression in the ulcerated area in the systemic steroid administration group.

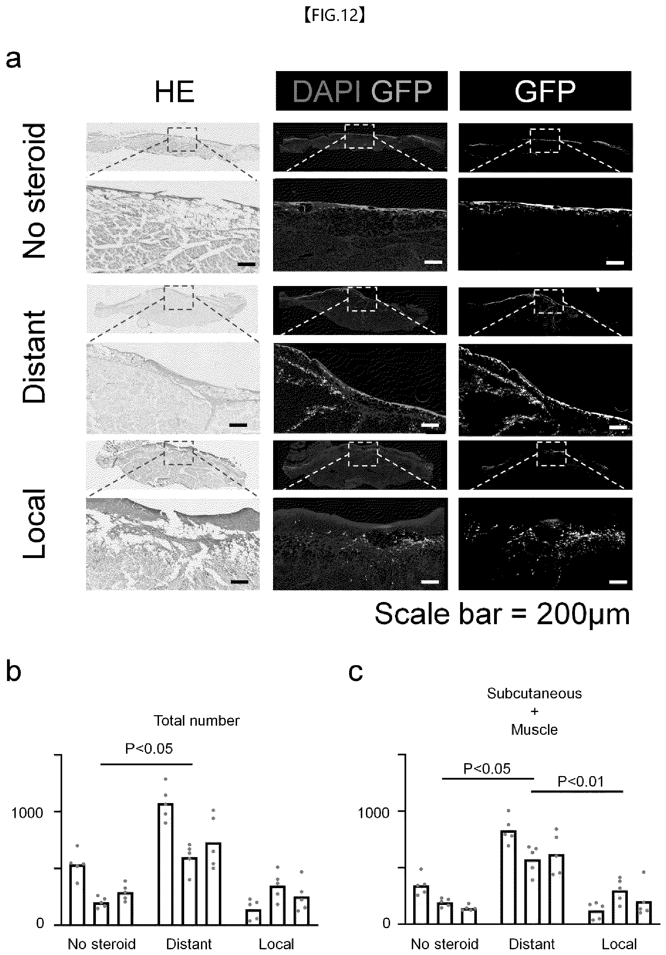

[FIG. 12] This figure shows histological observations on the seventh day after AAV administration, demonstrating that a large number of GFP-positive cells were observed, particularly in the deep layers, in the remote administration group. (a) shows representative findings of the ulcerated tissues from each group. In the no-steroid group, the number of GFP-positive cells in the subcutaneous and deep layers was low. In contrast, the remote administration group exhibited a large number of GFP-positive cells, particularly in the deep layers, while the local administration group showed a low number of positive cells in both the superficial and deep layers. (b) shows the count of GFP-positive cells in the central region of the ulcer based on histological findings . The results indicate that the remote administration group exhibited a significantly higher number of GFP-positive cells compared to the no-steroid group, considering both superficial and deep layers combined. (c) shows that in the deep layers (subcutaneous + muscle layers), the remote administration group exhibited a significantly higher number of GFP-positive cells compared to the no-steroid group and the local administration group.

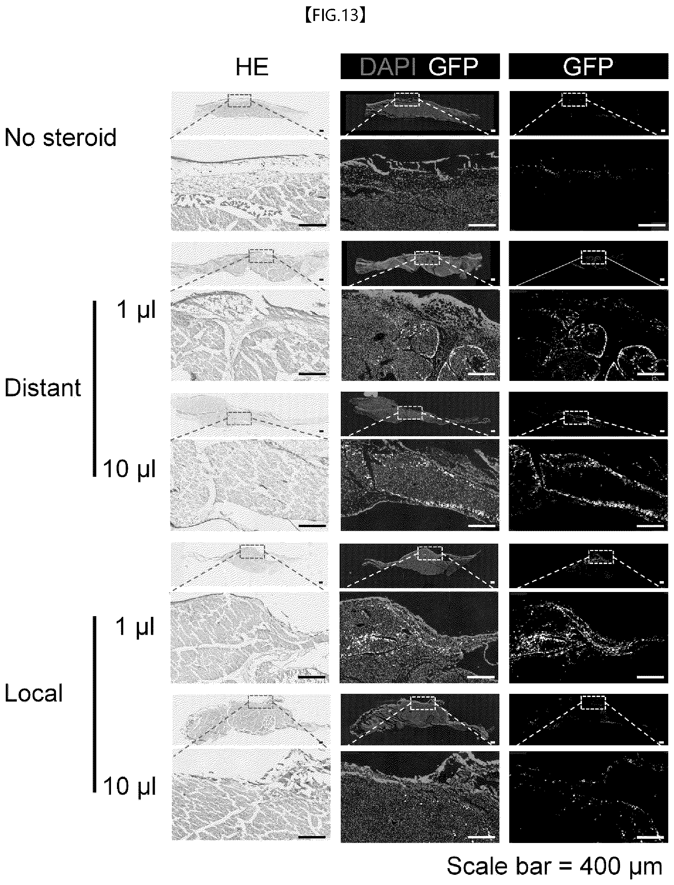

[FIG. 13] This figure shows the results of an experiment evaluating the effect of different steroid concentrations on the gene transduction efficiency of AAV in skin ulcers. On the day before administering AAVDJ-CAG-GFP (10¹¹ GC/100 µl), skin ulcers were created on the dorsal skin of mice, and silicone chambers were attached. Groups were prepared as follows: a no-steroid group; a remote administration group in which 1 µl or 10 µl of Kenacort A (40 mg/ml) was subcutaneously injected into the abdominal region; and a local administration group in which 1 µl or 10 µl of Kenacort was injected into the chamber. On the sixth day after AAV administration, the skin ulcer areas were observed using fluorescence stereomicroscopy, and tissues, including the ulcerated areas, were collected. The results show that in the remote administration group, a higher number of positive cells was observed with the 10 µl dose, whereas in the local administration group, the 1 µl dose resulted in a higher number of positive cells.

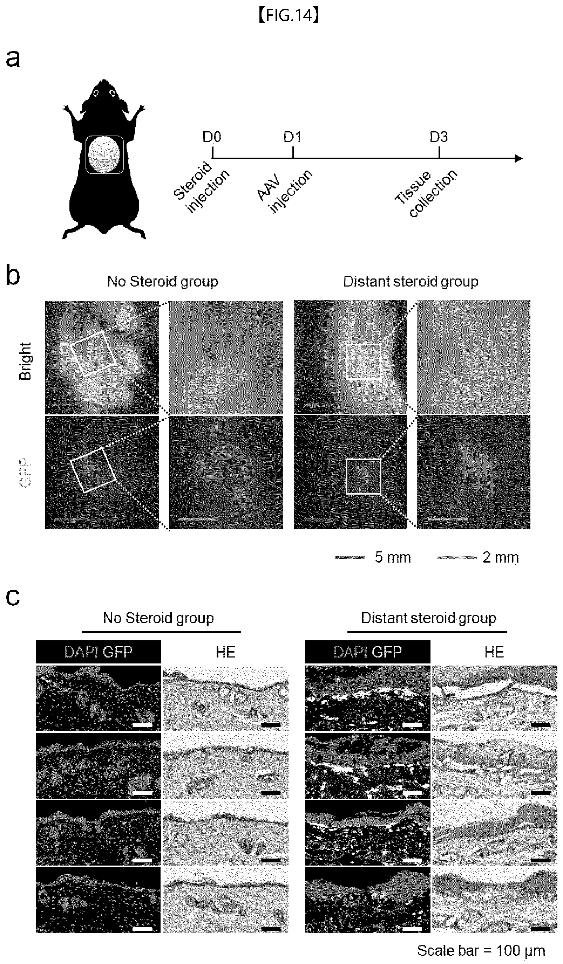

[FIG. 14] This figure presents the results of an experiment evaluating the effect of steroid administration on the gene transduction efficiency of AAV in the dorsal skin of mice, demonstrating that steroid administration improves gene transduction efficiency. (a) shows a schematic of the experiment. Two groups were prepared: a remote administration group in which 10 µl of Kenacort A (40 mg/ml) was subcutaneously injected into the abdominal region, and a no-steroid group. One day later, after shaving the dorsal skin, 35 µl of AAVDJK2-K14SCP3-GFPNLS (10¹¹ GC) was intradermally injected into the dorsal skin. Observations and tissue sampling were performed on the second day after AAV administration. (b) shows stereomicroscopic observations, indicating stronger GFP expression in animals that received steroid administration compared to those that did not. (c) shows histological observations, demonstrating high-efficiency and continuous GFP-positive cells centered in the epidermal tissue in steroid-administered animals. In contrast, GFP-positive cells were observed at low frequency and sporadically in animals without steroid administration.

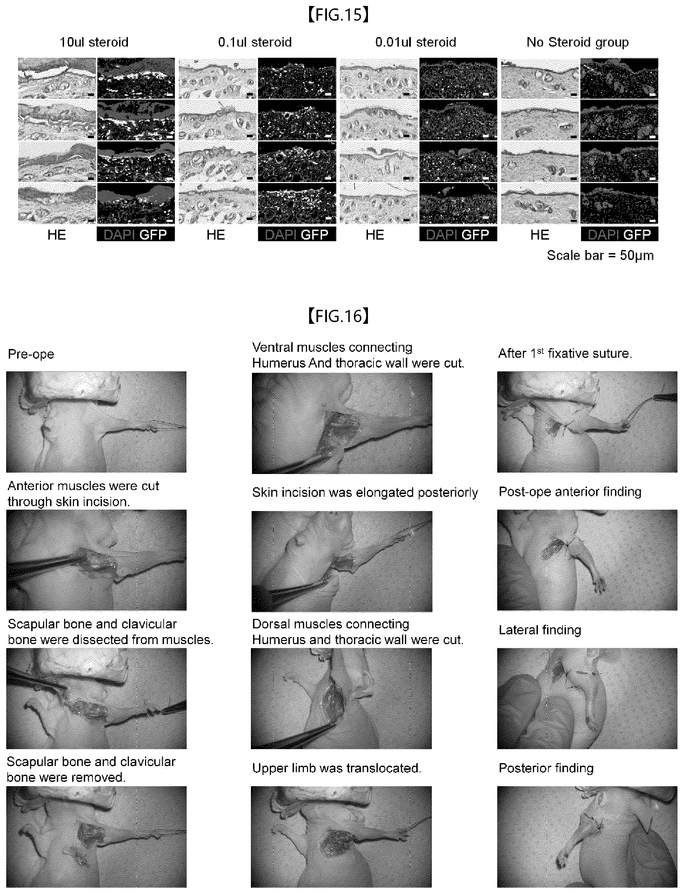

[FIG. 15] This figure shows the results of an experiment evaluating the effect of different concentrations of steroids on the gene transduction efficiency of AAV in the skin. The results demonstrate that when the amount of steroid administered subcutaneously to the abdominal region for remote administration is drastically reduced (0.01 µl), the enhancement effect of steroid on gene transduction efficiency decreases.
[FIG. 16] This figure illustrates the surgical procedure performed to reposition the left upper limb to the dorsal side following amputation in a mouse. The purpose of the procedure is to prevent self-inflicted trauma to the stump and to maintain local stability as much as possible. The surgical steps are shown sequentially from top to bottom in the left column, middle column, and right column. During the procedure, the brachial plexus and the main arteries and veins supplying the upper limb are preserved. This surgical technique allows the left upper limb to be repositioned to the dorsal side.

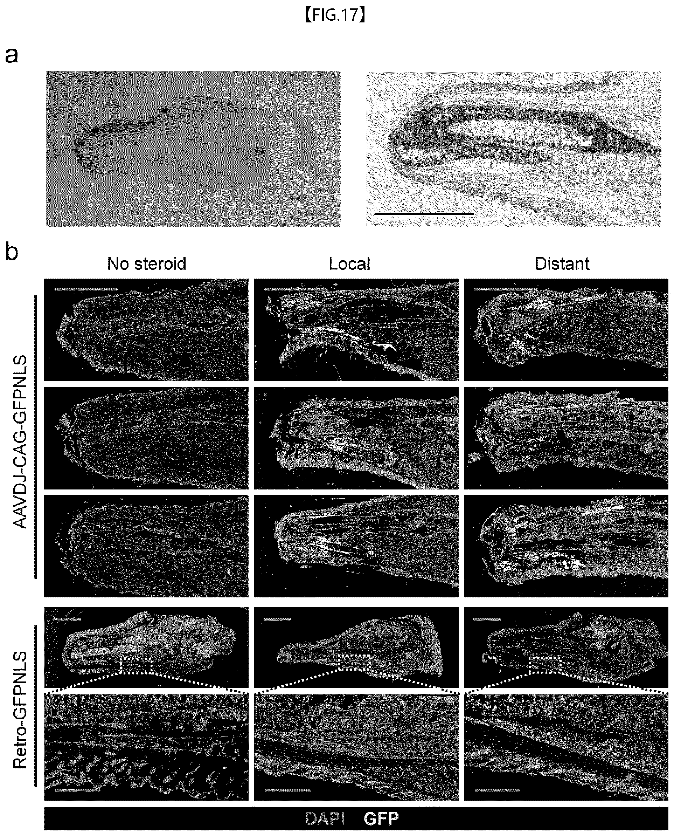

[FIG. 17] This figure presents the results of an experiment evaluating the effect of steroid administration on the gene transduction efficiency of AAV and retrovirus at the amputation site of the mouse forearm. The results demonstrate that steroid administration enhances gene transduction efficiency. (a) shows the macroscopic view of the collected forearm amputation tissue and representative histological observations. The central region contains bone, surrounded by muscle, with skin and subcutaneous fat covering the outer layer. The black scale bar represents 2 mm. (b) shows histological observations following gene transduction with GFPNLS using AAV and retroviral vectors after no steroid administration, local administration, or systemic administration. In both AAV and retroviral vectors, gene transduction efficiency was significantly enhanced by local or systemic steroid administration. Notably, in the case of AAV, a remarkable improvement in gene transduction efficiency was observed in a wide range of tissues constituting the limbs, including the subcutaneous tissue, fascia, muscle, and periosteum. The scale bar for the low-magnification image represents 2 mm, while the scale bar for the high-magnification image represents 500 µm.

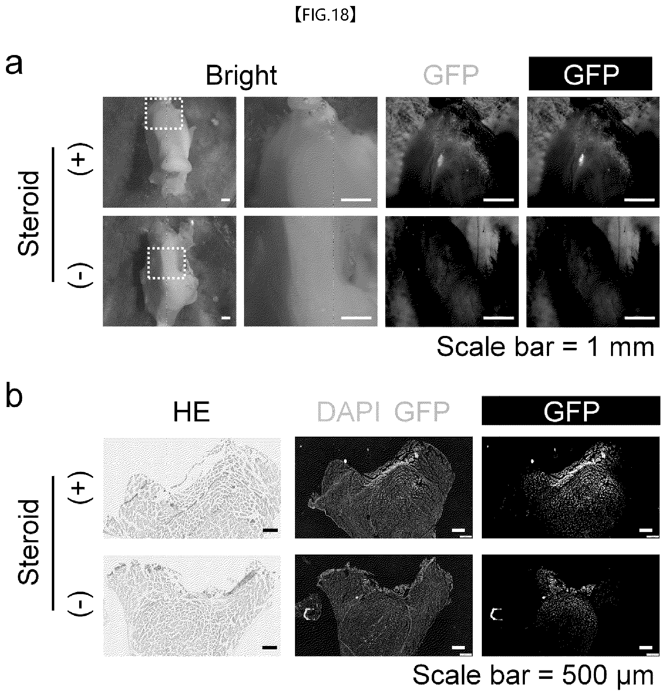

[FIG. 18] This figure shows the results of an experiment evaluating the effect of steroid administration on the gene transduction efficiency of mRNA in the anterior thigh muscles of mice. The results demonstrate that steroid administration enhances gene transduction efficiency. (a) shows stereomicroscopic observations 12 hours after intramuscular injection of mRNA encoding EGFP, processed using Lipofectamine 2000, into the anterior thigh muscles of nude mice. The injection was performed one day after systemic steroid administration via subcutaneous injection into the abdominal region. Fluorescent signals visible under stereomicroscopy were observed on the muscle surface in the systemic steroid administration group. (b) shows histological observations of the muscle tissue, demonstrating stronger GFP expression in the steroid-administered group.

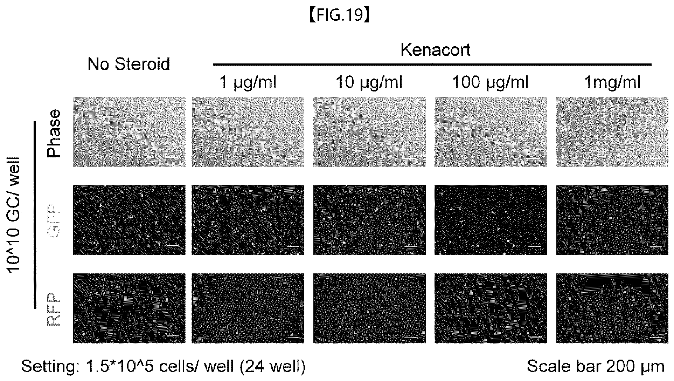

[FIG. 19] This figure shows the results of an experiment evaluating the effect of steroid administration on the gene transduction efficiency of AAV in in vitro cultured cells. The results demonstrate that higher gene transduction efficiency is achieved with appropriate concentrations of steroids. In the presence of 1 or 10 µg/ml steroids, a greater number of GFP-positive cells were observed compared to the no-steroid condition. In contrast, at higher concentrations of steroids (100 and 1000 µg/ml), the number of cells and GFP-positive cells decreased due to the effects of steroids on the cells.

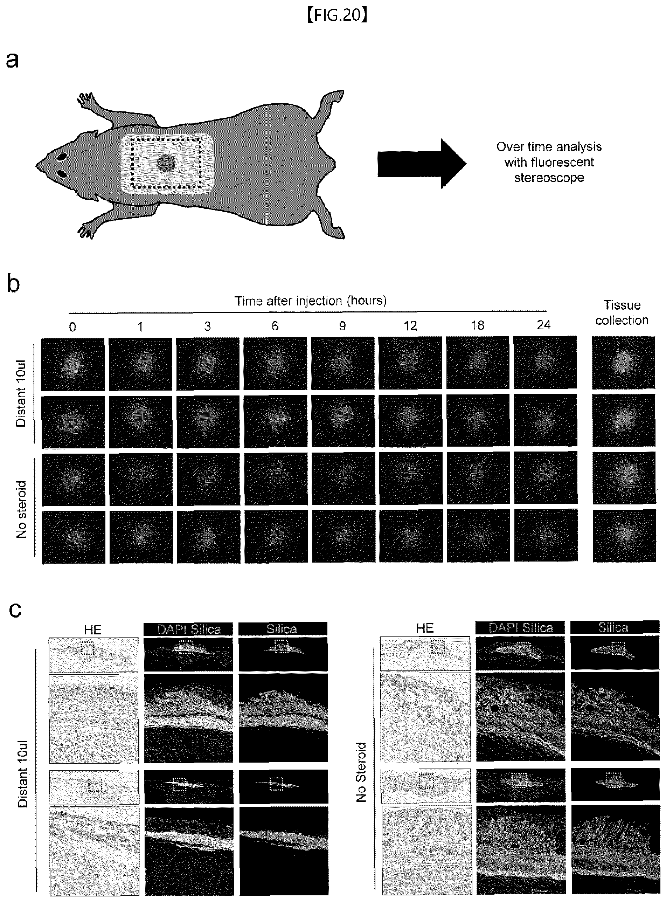

[FIG. 20] This figure shows the results of an experiment evaluating the effect of steroid administration on the dynamics of subcutaneously injected fluorescent silica particles. The results demonstrate that steroid administration reduced the clearance of locally injected fluorescent silica particles from the injection site. (a) shows a schematic of the experiment. Two groups were prepared: a no-steroid group and a remote administration group, in which 10 µl of Kenacort A (40 mg/ml) was subcutaneously injected into the abdominal region one day prior. Fluorescent silica particles of similar size to AAV (sicastar^{®}-redF, plain, 30 nm, 25 mg/ml) were subcutaneously injected into the dorsal skin of mice (30 µl). Observations of the injection site were conducted immediately and at 1, 3, 6, 9, 12, 18, and 24 hours after injection using fluorescence stereomicroscopy to analyze the effect of steroids on the local retention of fluorescent silica particles. (b) The left side shows fluorescence microscopy images taken at consistent exposure times (30 msec) over time, while the right side shows images taken at longer exposure times during tissue collection after 24 hours. The results indicate that in animals administered steroids, the silica particles strongly remained at the injection site after administration. Observations at the 24-hour mark also revealed that in animals without steroid administration, the particles had dispersed more broadly into the surrounding areas. (c) shows histological observations of tissue sections collected 24 hours after administration. The images include hematoxylin and eosin (HE) staining, fluorescence observations of nuclear staining with DAPI and silica, and fluorescence observations of silica particles alone. The histological findings also demonstrate that in animals administered steroids, the diffusion of silica particles into the surrounding tissue was suppressed compared to animals without steroid administration, leading to stronger retention of the particles at the local injection site.

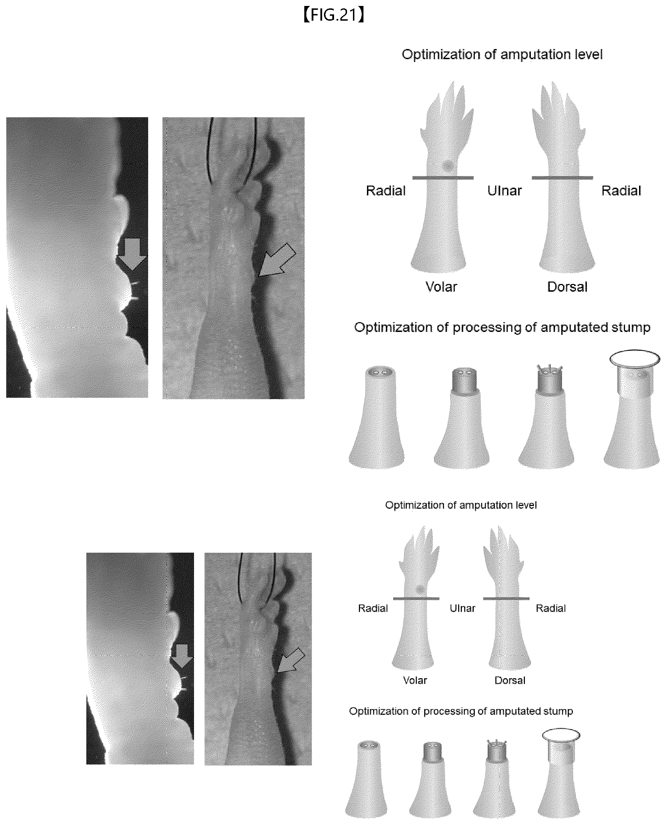

[FIG. 21] This figure illustrates a schematic of the stump processing method for forearm amputation in nude mice. On the skin surface of the forearm proximal to the wrist joint, there is a structure with a slight protrusion and redness (indicated by the arrow in the right photograph). In neonatal mice, this area also features a protrusion, which is characterized by hair growth from birth (indicated by the arrow in the left photograph), making it an anatomically consistent structure. By setting the amputation level proximal to this structure, the forearm can be reliably amputated proximal to the wrist joint. The amputation can be performed as a simple transverse cut, or additional steps can be taken, such as excising the surrounding skin of the stump or leaving the nerves longer during the amputation. After the amputation, the wound can either be left as an open wound or closed by compressing the surrounding skin manually or by suturing. If significant bleeding occurs at the stump, hemostasis can be promoted by attaching a silicone cap.

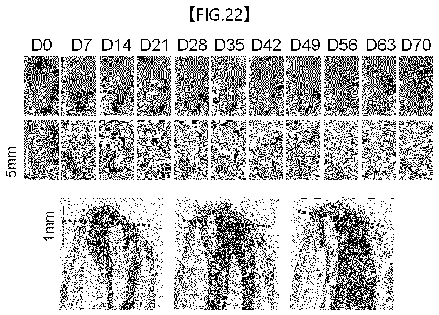

[FIG. 22] This figure shows the results of an experiment in which the left upper limb of a nude mouse was repositioned to the dorsal side, followed by transverse amputation at the forearm, leaving the cross-section as an open wound. The figure presents the progression of the stump's external appearance and histological observations. No significant morphological changes in the stump were observed externally over the 70 days following amputation. In the histological sections, the bone stump was closed with cortical bone, and the soft tissue had healed through contraction and closure of the surrounding skin. No notable tissue regeneration was observed. The upper scale bar represents 5 mm, and the lower scale bar represents 1 mm. The dotted lines indicate the amputation site, determined based on the morphology of the bone.

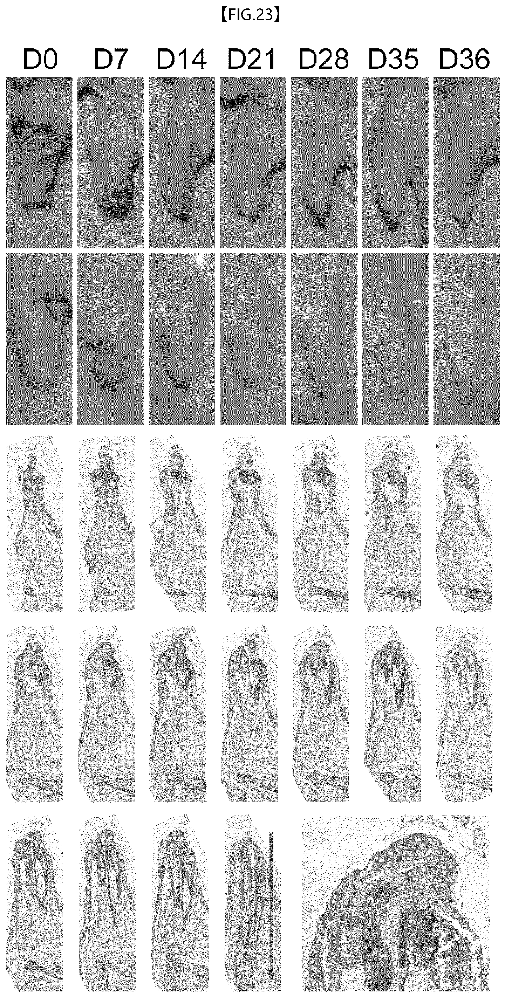

[FIG. 23] This figure shows the results of an experiment in which the left upper limb of a nude mouse was repositioned to the dorsal side, followed by transverse amputation at the forearm, leaving the cross-section as an open wound, and gene transduction was performed. The figure illustrates the progression of the stump's external appearance and histological observations. Gene transduction was conducted using retroviruses to deliver POLE4, NFIB, PPARD, FGF10, FGF20, and FGF2; AAVDJ1 to deliver PRRX1 and HDAC2; and AAVDJ to deliver LEF1 and SHH. The retroviral vectors were locally injected 10 times from immediately before amputation to the fifth day after amputation. AAVDJ-SHH was locally injected once immediately after amputation. The other AAV vectors were mixed and applied to the wound surface twice on the day of amputation, followed by two injections on the next day and three injections on the second day. Approximately four weeks after amputation, axial elongation of tissue at the stump was observed. Histological observations of the tissue collected on day 36 revealed axial elongation of bone within the stump. Furthermore, the distal part of the stump extended beyond the bone tip and formed a mass of soft tissue (enlarged image). The scale bar in the histological images represents 10 mm. From top to bottom: first row, dorsal frontal view; second row, lateral view; third to fifth rows, serial histological sections and enlarged views.

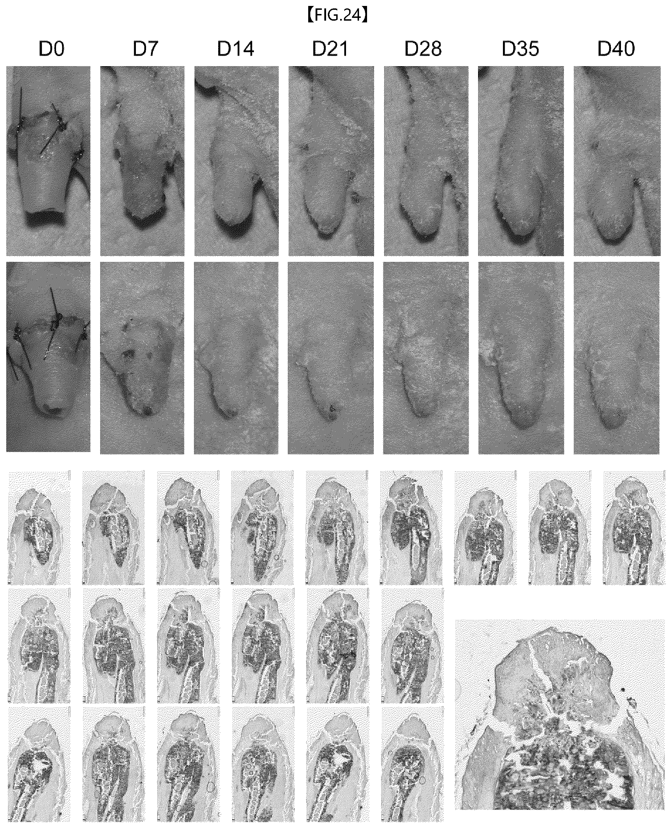

[FIG. 24] This figure shows the results of an experiment in which the left upper limb of a nude mouse was repositioned to the dorsal side, followed by transverse amputation at the forearm, leaving the cross-section as an open wound, and gene transduction was performed. The figure illustrates the progression of the stump's external appearance and histological observations. Gene transduction was conducted using retroviruses to deliver PRDX2, POLE4, NFIB, PPARD, FGF10, FGF20, FGF2, BMP5, and SHH; AAVDJ1 to deliver PRRX1 and HDAC2; and AAVDJ to deliver LEF1. The retroviral vectors were locally injected five times immediately before amputation and from the second to fifth days after amputation. The three types of AAV vectors were mixed and injected twice immediately before amputation and twice the following day. Approximately three weeks after amputation, axial elongation of tissue at the stump was observed. Histological observations of the tissue collected on day 40 revealed the formation of a mass of soft tissue distal to the bone cut surface. Within this soft tissue mass, new bone formation accompanied by trabecular structures was observed (enlarged image). From top to bottom: first row, dorsal frontal view; second row, lateral view; third to fifth rows, serial histological sections and enlarged views.

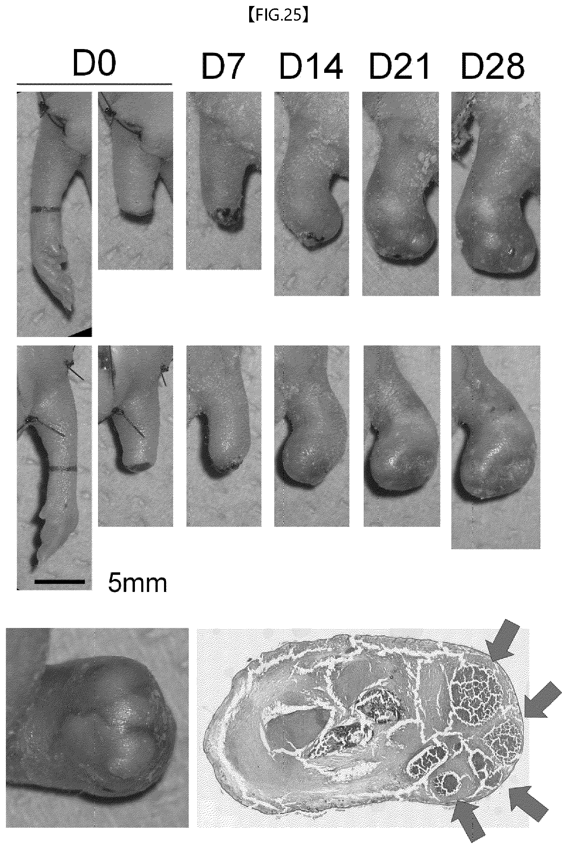

[FIG. 25] This figure shows the results of an experiment in which the left upper limb of a nude mouse was repositioned to the dorsal side, followed by transverse amputation at the forearm, leaving the cross-section as an open wound, and gene transduction was performed. The figure illustrates the progression of the stump's external appearance and histological observations. Gene transduction was conducted at the stump using retroviruses to deliver FGF10, FGF20, FGF2, NFIB, PPARD, and POLE4; retroviruses to deliver SHH to the lateral region of the stump; and AAVDJK2 to deliver FGF8 to the stump. The retroviruses expressing six genes were locally injected 18 times from immediately before amputation to the fifth day after amputation. The retrovirus expressing SHH was locally injected 11 times from immediately after amputation to the third day. AAVDJK2 expressing FGF8 was locally injected 7 times from the fifth to the 23rd day after amputation. Three weeks after amputation, the distal stump became flattened, and white branched structures became visible when observed from the palmar side. Histological observations of tissue collected on the 28th day revealed clusters of bone marrow cells corresponding to the white structures within the soft tissue mass (indicated by arrows). From top to bottom: first row, dorsal frontal view; second row, ventral frontal view; third row left, palmar view on day 28; third row right, histological observations.

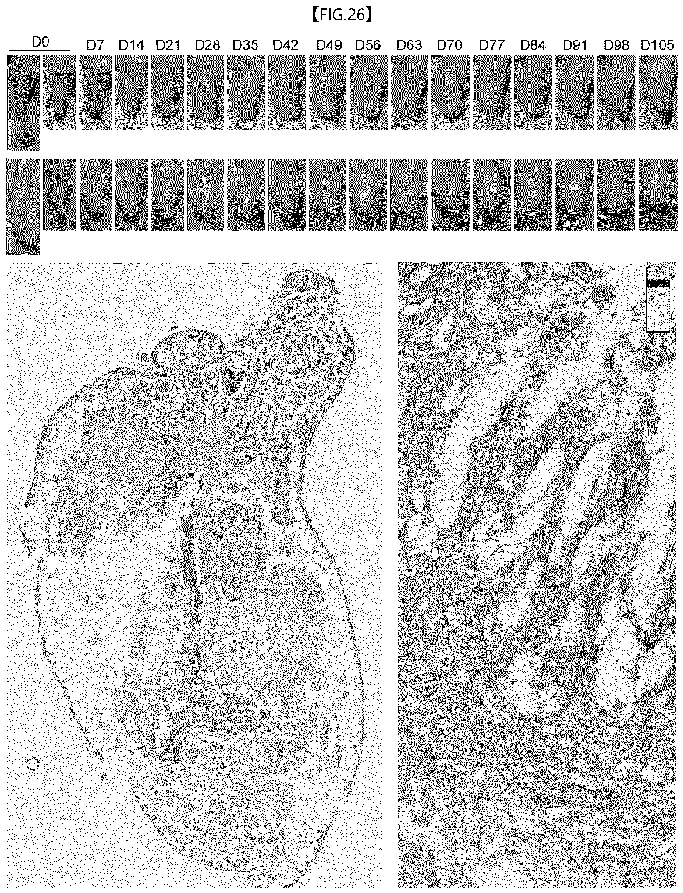

[FIG. 26] This figure shows the results of an experiment in which the left upper limb of a nude mouse was repositioned to the dorsal side, followed by transverse amputation at the forearm. The cross-section was pressed and adhered using surrounding skin, and gene transduction was performed. The figure illustrates the progression of the external appearance of the stump, representative histological observations, and magnified images. Gene transduction at the stump was performed using retroviruses to deliver FGF10, FGF20, FGF2, and OCT4. SHH was transduced into the lateral side of the stump using AAVDJ, while FGF8 was transduced from the medial side of the stump to the distal tip using AAVDJ. AAVDJ expressing SHH and FGF8 was locally injected four times from the day before amputation and every seven days until the 20th day after amputation. Retroviruses expressing FGF10, FGF20, FGF2, and OCT4 were locally injected 20 times between the time of amputation and the fourth day after amputation. Subsequently, retroviruses expressing OCT4 were locally injected 16 times between the fifth and tenth days after amputation. For the initial injection of retrovirus just before amputation, Kenacort A was mixed into the retrovirus solution. Around 10 weeks after amputation, a protruding structure began to appear at the distal stump and continued to elongate. Histological observations of the tissue collected on the 105th day revealed axially oriented cellular structures within the elongated tissue. From top to bottom: first row, dorsal frontal view; second row, lateral view; third row left, histological observations including the protruding structure; third row right, magnified image of the protruding structure.

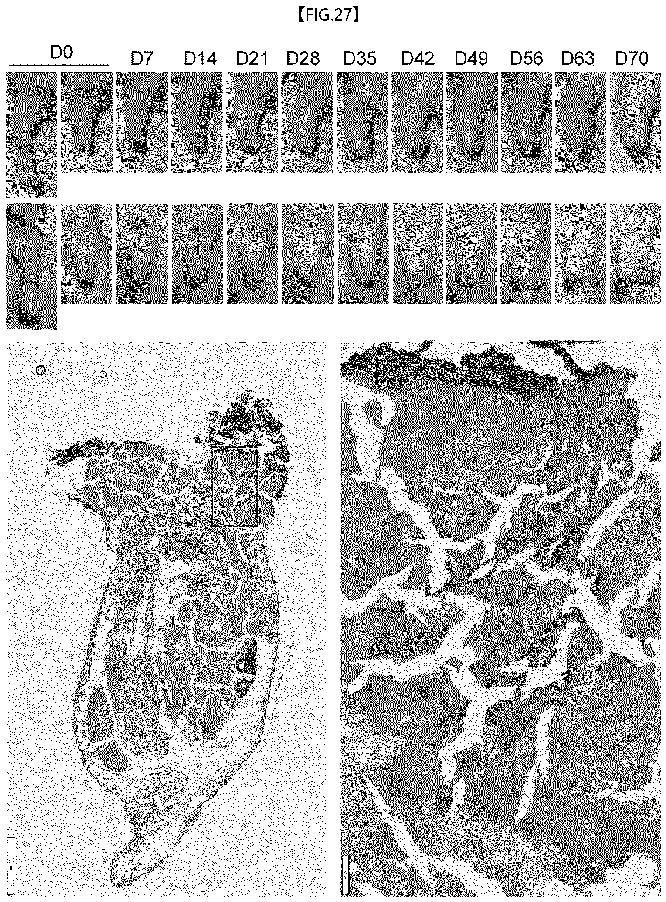

[FIG. 27] This figure shows the results of an experiment in which the left upper limb of a nude mouse was repositioned to the dorsal side, followed by transverse amputation at the forearm. The cross-section was pressed and adhered using surrounding skin, and gene transduction was performed. The figure illustrates the progression of the external appearance of the stump, representative histological observations, and magnified images. Gene transduction at the stump was performed using retroviruses to deliver FGF10, FGF20, FGF2, OCT4, and SOX2. SHH was transduced into the lateral side of the stump using AAVDJ, while FGF8 was transduced from the medial side of the stump to the distal tip using AAVDJ. AAVDJ expressing SHH and FGF8 was locally injected four times from the day before amputation and every seven days until the 20th day after amputation. Retroviruses expressing FGF10, FGF20, FGF2, OCT4, and SOX2 were locally injected 20 times between the time of amputation and the fourth day after amputation. Subsequently, retroviruses expressing OCT4 and SOX2 were locally injected 16 times between the fifth and tenth days after amputation. For the initial injection of retrovirus just before amputation, Kenacort A was mixed into the retrovirus solution. Around 8 weeks after amputation, a protruding structure began to appear at the distal stump, which later continued to elongate in a branched form. Histological observations of tissue collected on the 70th day revealed axially oriented cellular structures within the elongated branched region. From top to bottom: first row, dorsal frontal view; second row, lateral view; third row left, histological observations including the branched elongated structure; third row right, magnified image of the branched elongated structure.

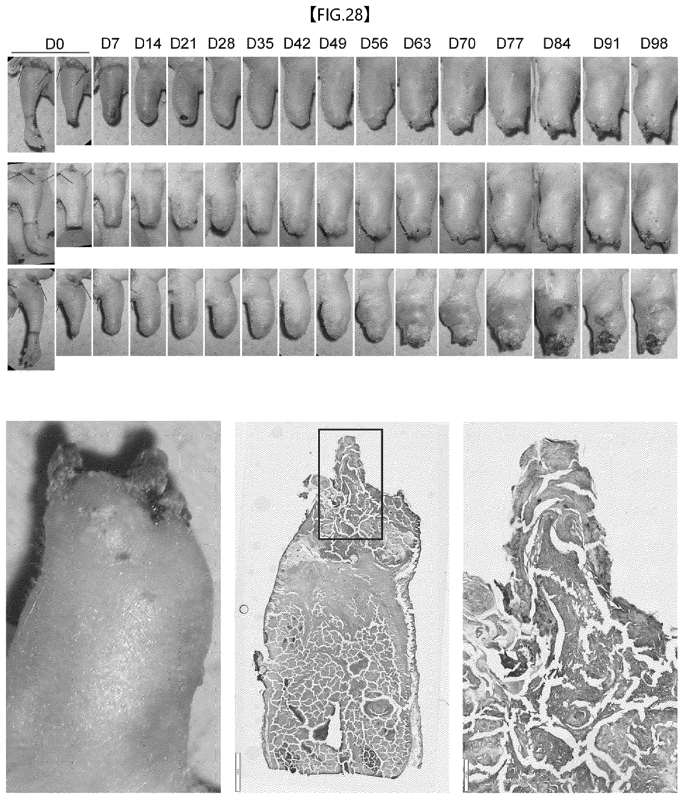

[FIG. 28] This figure shows the results of an experiment in which the left upper limb of a nude mouse was repositioned to the dorsal side, followed by transverse amputation at the forearm. The cross-section was pressed and adhered using surrounding skin, and gene transduction was performed. The figure illustrates the progression of the external appearance of the stump, representative histological observations, and magnified images. Gene transduction at the stump was performed using retroviruses to deliver FGF10, FGF20, FGF2, OCT4, SOX2, and TBX6. SHH was transduced into the lateral side of the stump using AAVDJ, while FGF8 was transduced from the medial side of the stump to the distal tip using AAVDJ. AAVDJ expressing SHH and FGF8 was locally injected four times from the day before amputation and every seven days until the 20th day after amputation. Retroviruses expressing FGF10, FGF20, FGF2, OCT4, SOX2, and TBX6 were locally injected 20 times between the time of amputation and the fourth day after amputation. Subsequently, retroviruses expressing OCT4, SOX2, and TBX6 were locally injected 16 times between the fifth and tenth days after amputation. For the initial injection of retrovirus just before amputation, Kenacort A was mixed into the retrovirus solution. Around 8 weeks after amputation, a protruding structure began to appear at the distal stump, which later continued to elongate in a branched form. Histological observations of tissue collected on the 98th day revealed axially oriented tissue structures within the elongated branched region, consisting of cartilage-like cells resembling the distal phalanges of fingers and trabecular bone structures indicative of new bone formation. From top to bottom: first row, dorsal frontal view; second row, lateral view; third row, ventral frontal view; fourth row left, magnified image of the distal portion on day 98; fourth row center, histological observations including the branched elongated structure; fourth row right, magnified histological observations of the branched elongated structure.

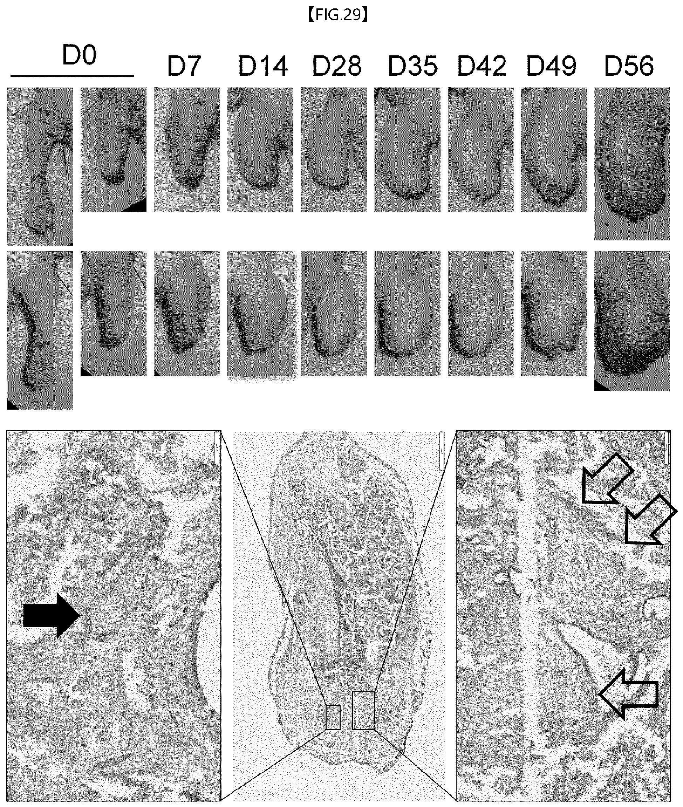

[FIG. 29] This figure shows the results of an experiment in which the left upper limb of a nude mouse was repositioned to the dorsal side, followed by transverse amputation at the forearm. The cross-section was pressed and adhered using surrounding skin, and gene transduction was performed. The figure illustrates the progression of the external appearance of the stump, representative histological observations, and magnified images. Gene transduction at the stump was performed using retroviruses to deliver FGF10, FGF20, FGF2, OCT4, SOX2, TBX6, and SHH. FGF8 was transduced into the distal tip of the stump using AAVDJ. AAVDJ expressing FGF8 was locally injected four times from the day before amputation and every seven days until the 20th day after amputation. Retroviruses expressing FGF10, FGF20, FGF2, OCT4, SOX2, and TBX6 were locally injected 16 times between the time of amputation and the fifth day after amputation. Retroviruses expressing SHH were locally injected four times from the time of amputation to the third day. Subsequently, retroviruses expressing OCT4, SOX2, and TBX6 were locally injected seven times between the sixth and eighth days after amputation. For the retrovirus injections administered after amputation, Kenacort A was mixed into the retrovirus solution. Histological observations of the tissue collected on the 56th day after amputation revealed the formation of a pouch-like structure distal to the bone stump. Within this structure, newly formed cartilage tissue and muscle bundles independent of the surrounding muscles were observed. From top to bottom: first row, dorsal frontal view; second row, ventral frontal view; third row center, overall histological observations; third row left, magnified view of the pouch-like structure distal to the bone stump, with arrows indicating cartilage tissue; third row right, magnified view of the pouch-like structure distal to the bone stump, with arrows indicating newly formed muscle tissue.

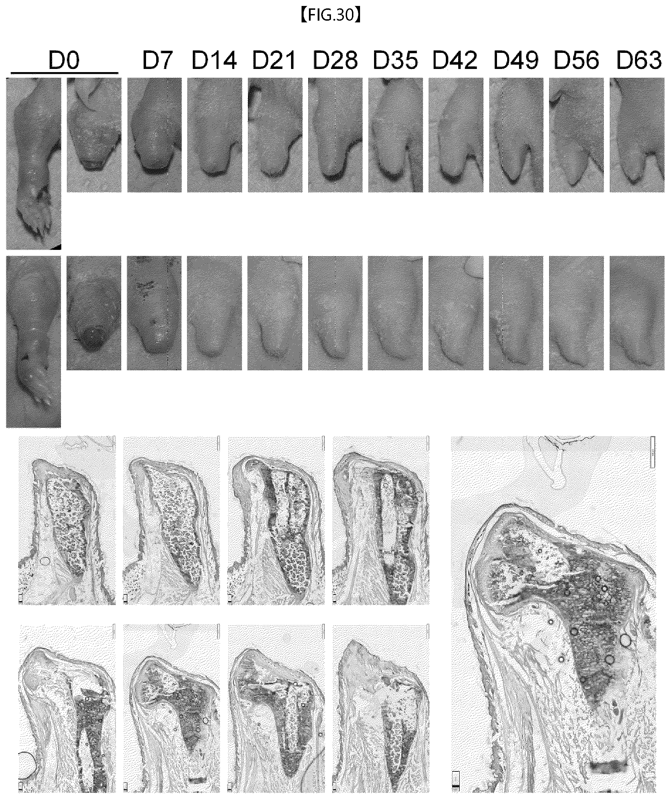

[FIG. 30] This figure shows the results of an experiment in which the left upper limb of a nude mouse was repositioned to the dorsal side. Gene transduction was performed at the planned amputation site by injecting FGF2 and viral solutions, followed by transverse amputation at the forearm. The cross-section was left as an open wound, and gene transduction was continued after amputation. The figure illustrates the progression of the external appearance of the stump and histological observations. Before amputation, gene transduction was performed over three days, with one injection per day, using retroviruses expressing HMGB3, DMNT1, HDAC2, PRDX2, and FGF10, mixed with FGF2. Just before amputation, retroviral solutions expressing the same genes mixed with FGF2 were locally injected. After amputation, SHH was transduced into the external side of the stump using AAVDJ. On the first and second days after amputation, retroviral solutions were applied once daily to the cross-section. Over time, the distal stump began to protrude and elongate. Histologically, cartilage tissue resembling intra-articular cartilage, accompanied by endochondral ossification-like histological structures, was induced at the bone stump. From top to bottom: first row, dorsal frontal view; second row, lateral view; third and fourth rows, serial histological sections and magnified images.

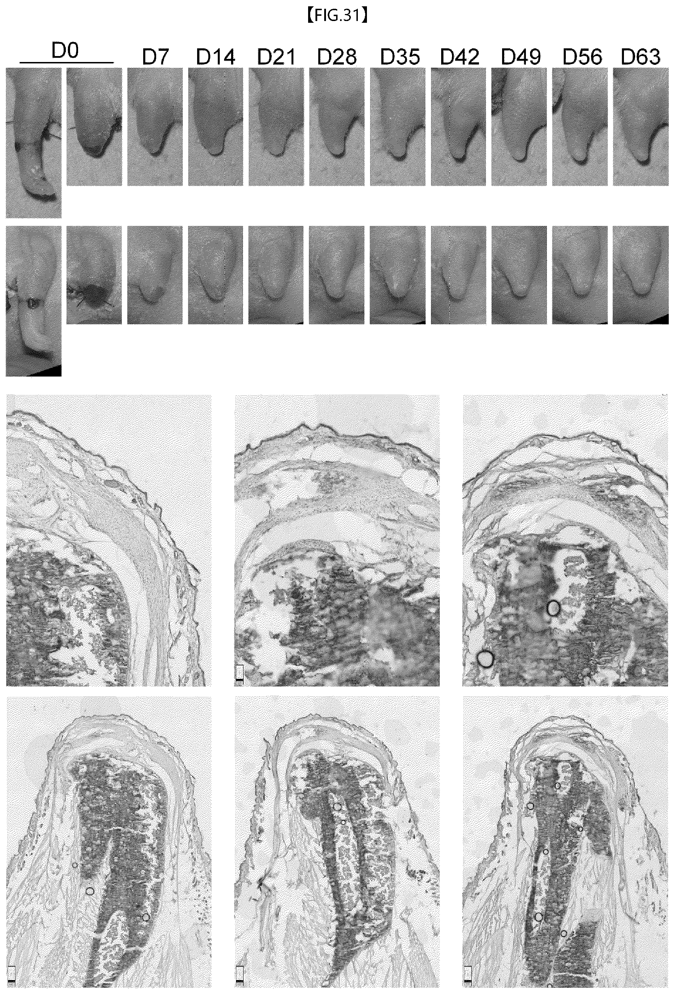

[FIG. 31] This figure shows the results of an experiment in which the left upper limb of a nude mouse was repositioned to the dorsal side. Gene transduction was performed at the planned amputation site by injecting FGF2 and viral solutions, followed by transverse amputation at the forearm. The cross-section was left as an open wound, and gene transduction was continued after amputation. The figure illustrates the progression of the external appearance of the stump and histological observations. From four to two days before amputation, gene transduction with retroviruses expressing FGF10 and FGF2 injections were performed. During the amputation, the nerve stump was left longer, and the surrounding skin of the stump was excised, followed by reinjection of viral solutions. One week after amputation, SHH was transduced into the external side of the stump using AAVDJ1. Histological observations revealed continuous induction of cartilage tissue from cutaneous muscle layers near the stump. From top to bottom: first row, dorsal frontal view; second row, lateral view; third row, magnified image of the distal region of the tissue section; fourth row, histological observations including the distal region.

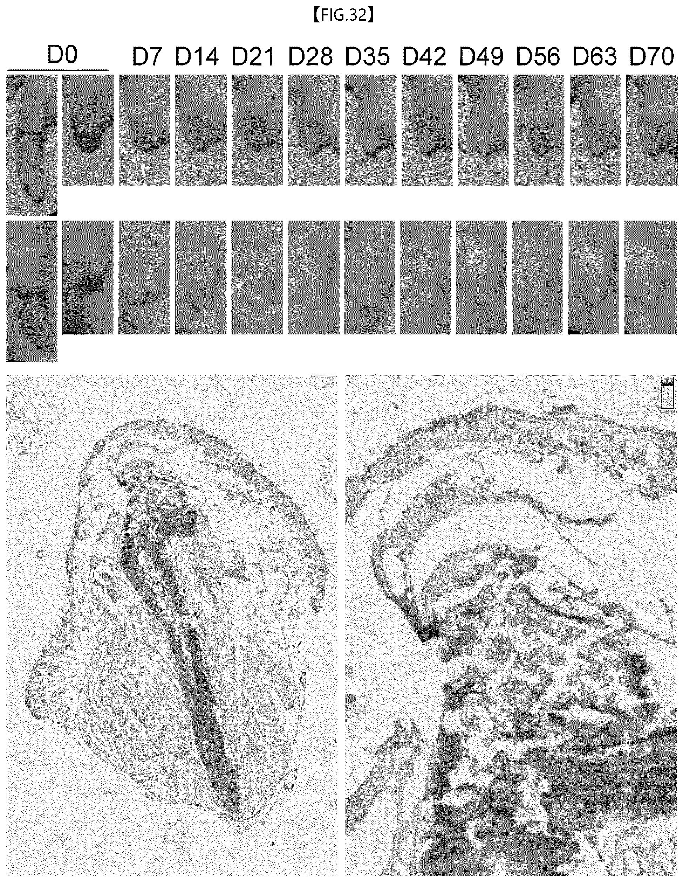

[FIG. 32] This figure shows the results of an experiment in which the left upper limb of a nude mouse was repositioned to the dorsal side. Gene transduction was performed at the planned amputation site by injecting FGF2 and viral solutions, followed by transverse amputation at the forearm. The cross-section was left as an open wound, and gene transduction was continued after amputation. The figure illustrates the progression of the external appearance of the stump and histological observations. From four to two days before amputation, gene transduction was performed using retroviruses expressing HMGB3, DMNT1, HDAC2, and FGF10, combined with FGF2 injections. During the amputation, the nerve stump was left longer, and the surrounding skin of the stump was excised, followed by reinjection of viral solutions. One week after amputation, SHH was transduced into the external side of the stump using AAVDJ1. Histological observations revealed continuous induction of cartilage tissue from cutaneous muscle layers near the stump. The bone stump was covered with cartilage-like tissue. From top to bottom: first row, dorsal frontal view; second row, lateral view; third row left, histological observations of tissue sections including the distal region; third row right, magnified histological observations of the distal region.

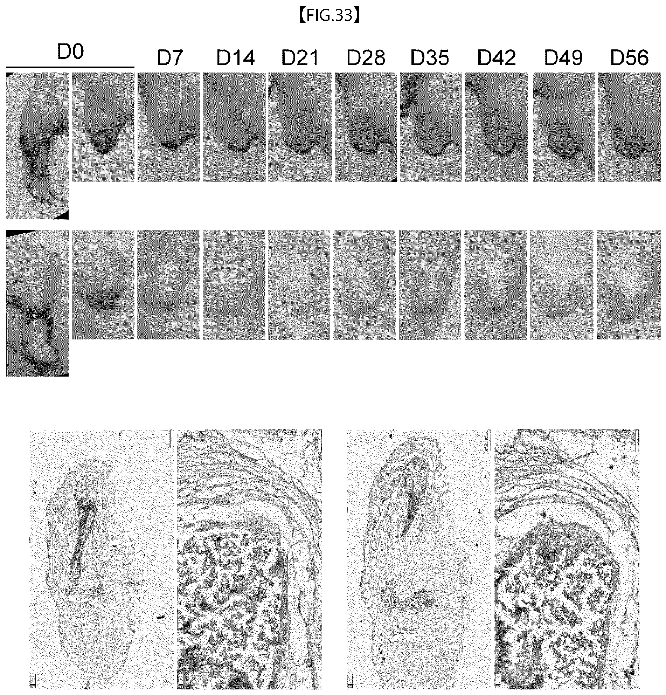

[FIG. 33] This figure shows the results of an experiment in which the left upper limb of a nude mouse was repositioned to the dorsal side. Gene transduction was performed at the planned amputation site by injecting FGF2 and viral solutions, followed by transverse amputation at the forearm. The cross-section was left as an open wound, and gene transduction was continued after amputation. The figure illustrates the progression of the external appearance of the stump and histological observations. From eight to one day before amputation, gene transduction was performed using retroviruses expressing MSX1, MSX2, LIN28A, MEIS1, and FGF10, combined with FGF2 injections. During the amputation, the nerve stump was left longer, and the surrounding skin of the stump was excised, followed by reinjection of viral solutions. On the 11th day after amputation, SHH was transduced into the external side of the stump using AAVDJ1. On the 14th day after amputation, LEF1 and FGF8were transduced into the distal region of the stump using AAVDJK2. Histological observations revealed axial elongation of the bone stump and cartilage tissue induced at the bone stump, accompanied by endochondral ossification-like structures resembling intra-articular cartilage. From top to bottom: first row, dorsal frontal view; second row, lateral view; third row, observations of adjacent tissue sections including the distal region (1st and 3rd from the left), and magnified images of the distal region (2nd and 4th from the left).

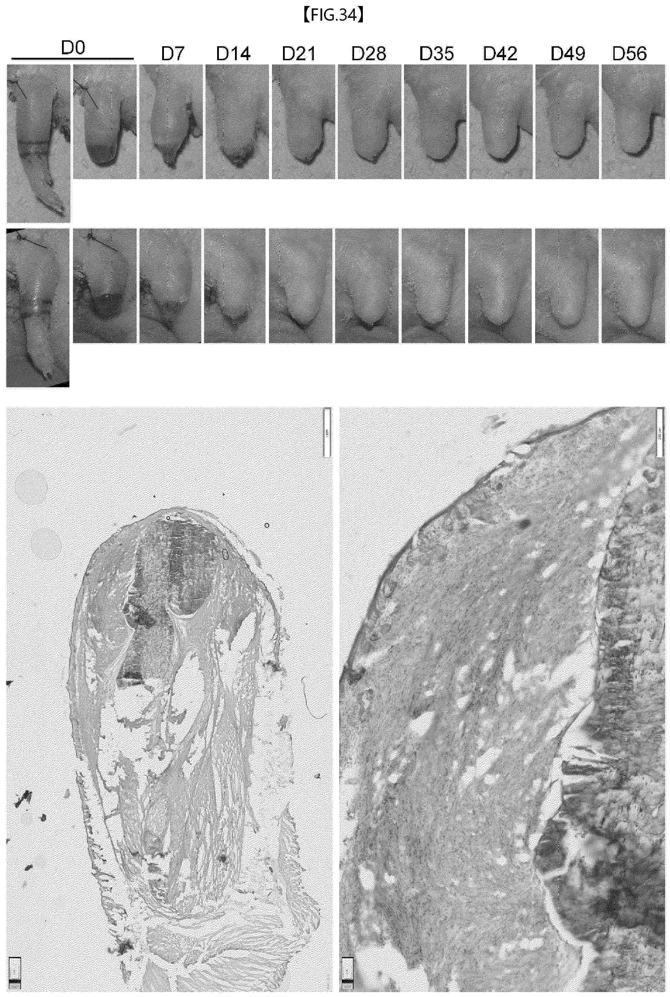

[FIG. 34] This figure shows the results of an experiment in which the left upper limb of a nude mouse was repositioned to the dorsal side. Gene transduction was performed at the planned amputation site by injecting FGF2 and viral solutions, followed by transverse amputation at the forearm. The cross-section was left as an open wound, and gene transduction was continued after amputation. The figure illustrates the progression of the external appearance of the stump and histological observations. From five to two days before amputation, gene transduction was performed using retroviruses expressing MSX1, MSX2, LIN28A, WNT7A, and FGF10, combined with FGF2 injections. On the day before amputation, SHH was transduced using AAVDJ1, and LEF1 was transduced using AAVDJK2. Following retrovirus injection, transverse amputation was performed, and the surrounding skin of the stump was excised. On the day after amputation, retroviral solutions were injected again. Histological observations revealed that muscle tissue was induced in the subcutaneous layer surrounding the amputation site. From top to bottom: first row, dorsal frontal view; second row, lateral view; third row left, histological observations of tissue sections including the distal region; third row right, magnified histological observations of the distal region.

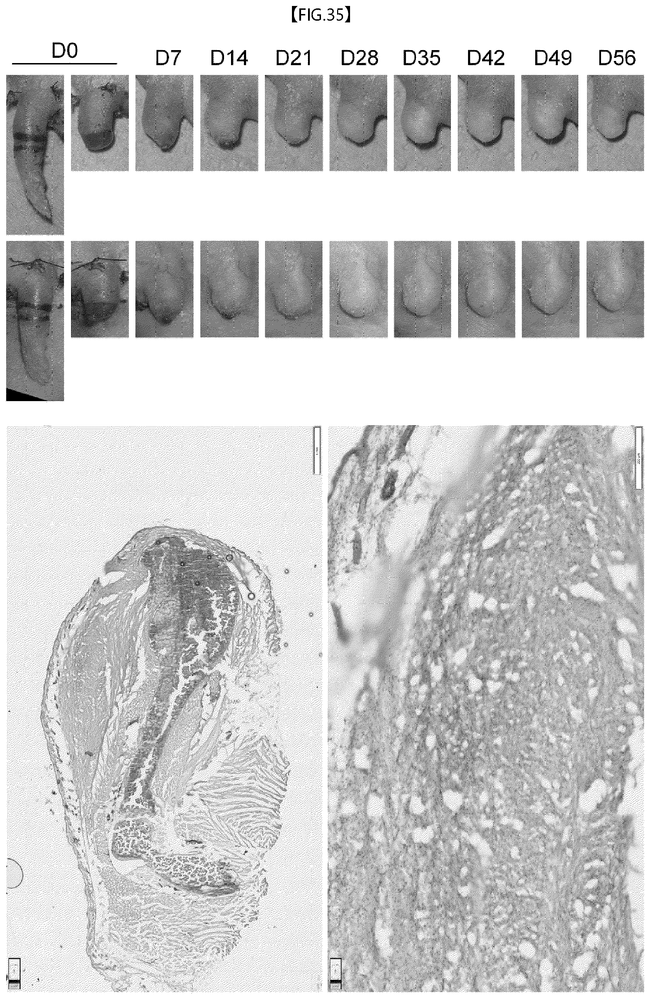

[FIG. 35] This figure shows the results of an experiment in which the left upper limb of a nude mouse was repositioned to the dorsal side. Gene transduction was performed at the planned amputation site by injecting FGF2 and viral solutions, followed by transverse amputation at the forearm. The cross-section was left as an open wound, and gene transduction was continued after amputation. The figure illustrates the progression of the external appearance of the stump and histological observations. From five to two days before amputation, gene transduction was performed using retroviruses expressing LEF1, HDAC2, PRDX2, WNT7A, and FGF10, combined with FGF2 injections. On the day before amputation, SHH was transduced using AAVDJ1, and LEF1was transduced using AAVDJK2. After the retroviral injection, transverse amputation was performed, and the surrounding skin of the stump was excised. On the day after amputation, retroviral solutions were injected again. Histological observations revealed the induction of muscle tissue in the subcutaneous layer surrounding the amputation site. From top to bottom: first row, dorsal frontal view; second row, lateral view; third row left, histological observations of tissue sections including the distal region; third row right, magnified histological observations of the distal region.

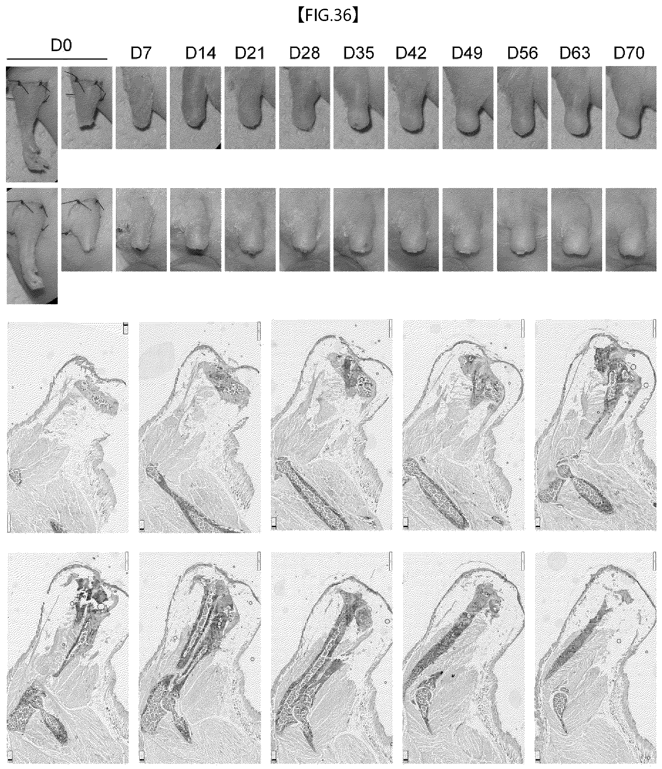

[FIG. 36] This figure shows the results of an experiment in which the left upper limb of a nude mouse was repositioned to the dorsal side, followed by transverse amputation at the forearm. The cross-section was left as an open wound, and gene transduction was performed using AAV after amputation. The figure illustrates the progression of the external appearance of the stump and histological observations. From the day of amputation to the second day, gene transduction was performed at the stump using AAVDJ to deliver LEF1, PRRX1, HDAC2, PRDX2, PPARD, FGF10, FGF20, FGF2, BMP5, and SHH. The distal stump became rounded and enlarged, and histological observations revealed the proliferation of adipose tissue within the muscle tissue beneath the subcutaneous and deep fascia layers. From top to bottom: first row, dorsal frontal view; second row, lateral view; third and fourth rows, serial histological sections.

### Detailed Description of the Invention

### 1. Applications of AAV with Superior Gene Transduction Efficiency for Cells in Skin Ulcers

In the present invention, "cells in skin ulcers" refer to cells present in skin ulcerated areas resulting from skin defects. These include dermis-derived fibroblasts, adipose-derived mesenchymal stem cells from the stromal components of adipose tissue, adipocytes, cells present in the fascia, stromal cells within muscle tissue, muscle cells, and tissue macrophages, which are physiologically present in the skin and soft tissues. Additionally, they include circulating blood-derived cells, such as mononuclear cells and macrophages, which accumulate in skin ulcers in response to wound signals.

AAV is known to exhibit high gene transduction efficiency, or tropism, for specific cell types or tissues depending on the structure of its capsid. Notably, the artificially developed AAVDJ capsid has been shown to exhibit significantly superior tropism compared to other capsids for cells in mouse skin, soft tissues, and skin ulcers (see Non-Patent Document 2, Grimm D, Lee JS, Wang L, Desai T, Akache B, Storm TA, Kay MA. In vitro and in vivo gene therapy vector evolution via multispecies interbreeding and retargeting of adeno-associated viruses. J Virol. 2008 Jun;82(12):5887-911).

In the present invention, "gene therapy" refers not only to therapeutic methods aimed at compensating for the functional loss of target genes by inducing their expression but also to therapies that promote tissue regeneration by expressing target genes. It further includes therapies involving the transduction of artificial genes with specific functions, such as micro RNA and siRNA, therapies that involve gene transduction for the purpose of genome modification or genome editing, and other treatments involving gene transduction for therapeutic purposes, such as disease treatment, physiological function enhancement, aging prevention, and anti-aging improvements.

In the present invention, "gene transduction vector" refers to a medium or carrier used to deliver DNA or RNA into the cells of plants or animals for the purpose of controlling gene expression or manipulating the genes themselves. Representative examples include viral vectors such as AAV, adenoviral vectors, lentiviral vectors, retroviral vectors, and Sendai viral vectors; plasmid vectors; and mRNA, as well as lipid nanoparticles composed of mRNA.

In the present invention, "gene transduction pharmaceutical" refers to a substance used for the treatment or prevention of conditions requiring recovery or improvement from functional decline due to diseases, pathological disorders, or physiological states, or for achieving further improvements from a normal state (e.g., rejuvenation to counter age-related changes). This term also includes items intended for therapeutic or preventive purposes to restore functions affected by diseases, pathological disorders, or physiological conditions, or to enhance a normal state. It further encompasses quasi-drugs and cosmetics designed to influence the structure or function of humans or animals.

In the present invention, "usable as a research and development tool for gene therapy" does not merely refer to its use as a tool for proof-of-concept experiments in experimental animals for gene therapy. It also encompasses the use of gene transduction to verify the efficacy of therapeutic approaches that ultimately aim to induce gene expression through other methods, such as various viral vectors, plasmid DNA delivery, or the administration of micro RNA, siRNA, or mRNA. This definition specifically includes its application as part of screening methods, such as CRISPR screening.

In the present invention, "usable as a research tool for drug discovery or biological research other than for the development of gene therapy methods" refers to the use of gene transduction as a research and development tool for purposes such as creating disease models or regulating molecular biological signaling pathways. This includes its application in the development of therapeutic approaches other than gene therapy, such as the development of small-molecule compounds, medium- and high-molecular compounds, protein therapeutics, antibody drugs, and cell-based medicines, as well as its use as a tool for biological research and development.

The AAVDJ1, an AAV with superior gene transduction efficiency for cells in skin ulcers in the present invention, demonstrates higher gene transduction efficiency and superior tropism for cells in skin ulcers compared to other capsids, including AAVDJ. As such, it can be used as a gene transduction vector in gene therapy for skin ulcers in vivo and as a research and development tool for gene therapy methods targeting skin ulcers. Additionally, it can be utilized as a research tool for drug discovery and biological studies not aimed at the development of gene therapy methods. Because AAVDJ1 also exhibits high tropism when used with various cultured cells derived from skin ulcers, it can serve as a gene transduction vector for cultured cells, including in gene therapy using cultured cells, as well as a research and development tool for gene therapy methods. Furthermore, it can also be employed as a research tool for drug discovery and biological studies beyond the development of gene therapy methods.

The AAV in the present invention, which exhibits superior gene transduction efficiency for cells in skin ulcers, was developed using directed evolution on a capsid-modified AAVDJ library. This library was created by modifying part of the nucleotide sequence of the AAVDJ Rep-Cap capsid to encode QRG, followed by seven random amino acids, and then 11 amino acids of A (QRGXXXXXXXA (SEQ ID NO: 1), where X represents random amino acids) at the NR site corresponding to the 589th and 590th amino acids of the AAVDJ capsid. (See Müller OJ, Kaul F, Weitzman MD, Pasqualini R, Arap W, Kleinschmidt JA, Trepel M. Random peptide libraries displayed on adeno-associated virus to select for targeted gene therapy vectors. Nat Biotechnol. 2003;21(9):1040-6.) The capsid-modified AAVDJ library was infected into the skin ulcers on the dorsal side of mice. After infection, the ulcerated tissue was collected, and primary mesenchymal cells were isolated and cultured. DNA containing viral genomes was extracted from the cells, and the extracted DNA underwent PCR amplification to amplify fragments containing randomized nucleotide sequences. These fragments were cloned into a backbone vector, and AAV was regenerated through repeated cycles. After four independent cycles, the remaining fragments were sequenced, and capsid-modified AAVDJs with multiple candidate sequences in the randomized sequence region were identified. AAVs expressing GFPNLS were prepared for each candidate. Gene transduction efficiency was evaluated in mouse adipose-derived mesenchymal cells and in the skin ulcers created on the dorsal side of mice. Among the candidates, **EPKARAP (SEQ ID NO: 2)** exhibited the highest gene transduction efficiency. Therefore, the AAV containing this peptide was designated as **AAVDJ1,** which exhibits superior tropism for cells in skin ulcers. Notably, the EPKARAP (SEQ ID NO: 2) sequence within the substituted peptides of AAVDJ1 plays a particularly important role in its infectivity.

### 2. Method for Producing AAV with Superior Gene Transduction Efficiency for Cells in Skin Ulcers

The AAVDJ1, which exhibits superior tropism for cells in skin ulcers, is an AAV whose capsid (SEQ ID NO: 5; amino acid sequence 2) has been modified from the known AAVDJ capsid (SEQ ID NO: 3; amino acid sequence 1) by replacing the NR region at the 589th and 590th amino acid positions with QRGEPKARAPA (SEQ ID NO: 4). AAVDJ1 can be produced as an AAV capable of expressing a desired gene by general methods for AAV production, such as co-transfection of a Rep-Cap plasmid encoding the AAVDJ1 capsid, a vector plasmid carrying the target gene, and a helper plasmid, or by a baculovirus-based AAV production method. The nucleotide sequence encoding AAVDJ1 (SEQ ID NO: 6; capsid nucleotide sequence 1) and the nucleotide sequence of the Rep-Cap plasmid (SEQ ID NO: 7; plasmid nucleotide sequence 1) are provided.

The known amino acid sequence of the AAVDJ capsid (SEQ ID NO: 3) is as follows. The NR region corresponding to the 589th and 590th amino acid positions is underlined.

MAADGYLPDWLEDTLSEGIRQWWKLKPGPPPPKPAERHKDDSRGLVLPGYKYLGPFNG
LDKGEPVNEADAAALEHDKAYDRQLDSGDNPYLKYNHADAEFQERLKEDTSFGGNLG
RAVFQAKKRLLEPLGLVEEAAKTAPGKKRPVEHSPVEPDSSSGTGKAGQQPARKRLNF
GQTGDADSVPDPQPIGEPPAAPSGVGSLTMAAGGGAPMADNNEGADGVGNSSGNWHC
DSTWMGDRVITTSTRTWALPTYNNHLYKQISNSTSGGSSNDNAYFGYSTPWGYFDFNR
FHCHFSPRDWQRLINNNWGFRPKRLSFKLFNIQVKEVTQNEGTKTIANNLTSTIQVFTDS
EYQLPYVLGSAHQGCLPPFPADVFMIPQYGYLTLNNGSQAVGRSSFYCLEYFPSQMLRT
GNNFQFTYTFEDVPFHSSYAHSQSLDRLMNPLIDQYLYYLSRTQTTGGTTNTQTLGFSQ
GGPNTMANQAKNWLPGPCYRQQRVSKTSADNNNSEYSWTGATKYHLNGRDSLVNPG
PAMASHKDDEEKFFPQSGVLIFGKQGSEKTNVDIEKVMITDEEEIRTTNPVATEQYGSVS
TNLQRGNRQAATADVNTQGVLPGMVWQDRDVYLQGPIWAKIPHTDGHFHPSPLMGGF
GLKHPPPQILIKNTPVPADPPTTFNQSKLNSFITQYSTGQVSVEIEWELQKENSKRWNPEI
QYTSNYYKSTSVDFAVNTEGVYSEPRPIGTRYLTRNL

The amino acid sequence of the AAVDJ1 capsid in the present invention (SEQ ID NO: 5) is as follows. The substituted region, QRGEPKARAPA (SEQ ID NO: 4), is underlined.

MAADGYLPDWLEDTLSEGIRQWWKLKPGPPPPKPAERHKDDSRGLVLPGYKYLGPFNG
LDKGEPVNEADAAALEHDKAYDRQLDSGDNPYLKYNHADAEFQERLKEDTSFGGNLG
RAVFQAKKRLLEPLGLVEEAAKTAPGKKRPVEHSPVEPDSSSGTGKAGQQPARKRLNFG
QTGDADSVPDPQPIGEPPAAPSGVGSLTMAAGGGAPMADNNEGADGVGNSSGNWHCD
STWMGDRVITTSTRTWALPTYNNHLYKQISNSTSGGSSNDNAYFGYSTPWGYFDFNRFH
CHFSPRDWQRLINNNWGFRPKRLSFKLFNIQVKEVTQNEGTKTIANNLTSTIQVFTDSEY
QLPYVLGSAHQGCLPPFPADVFMIPQYGYLTLNNGSQAVGRSSFYCLEYFPSQMLRTGN
NFQFTYTFEDVPFHSSYAHSQSLDRLMNPLIDQYLYYLSRTQTTGGTTNTQTLGFSQGGP
NTMANQAKNWLPGPCYRQQRVSKTSADNNNSEYSWTGATKYHLNGRDSLVNPGPAMA
SHKDDEEKFFPQSGVLIFGKQGSEKTNVDIEKVMITDEEEIRTTNPVATEQYGSVSTNLQR
GQRGEPKARAPAQAATADVNTQGVLPGMVWQDRDVYLQGPIWAKIPHTDGHFHPSPL

MGGFGLKHPPPQILIKNTPVPADPPTTFNQSKLNSFITQYSTGQVSVEIEWELQKENSKRW
NPEIQYTSNYYKSTSVDFAVNTEGVYSEPRPIGTRYLTRNL*

An example of the nucleotide sequence encoding the AAVDJ1 capsid in the present invention (SEQ ID NO: 6) is as follows. The modified region is underlined.

ATGGCTGCCGATGGTTATCTTCCAGATTGGCTCGAGGACACTCTCTCTGAAGGAATAA
GACAGTGGTGGAAGCTCAAACCTGGCCCACCACCAAAGCCCGCAGAGCGGCAT
AAGGACGACAGCAGGGGTCTTGTGCTTCCTGGGTACAAGTACCTCGGACCCTTCAAC
GGACTCGACAAGGGAGAGCCGGTCAACGAGGCAGACGCCGCGGCCCTCGAGCACG
ACAAAGCCTACGACCGGCAGCTCGACAGCGGAGACAACCCGTACCTCAAGTACAAC
CACGCCGACGCCGAGTTCCAGGAGCGGCTCAAAGAAGATACGTCTTTTGGGGGCAA
CCTCGGGCGAGCAGTCTTCCAGGCCAAAAAGAGGCTTCTTGAACCTCTTGGTCTGGT
TGAGGAAGCGGCTAAGACGGCTCCTGGAAAGAAGAGGCCTGTAGAGCACTCTCCTG
TGGAGCCAGACTCCTCCTCGGGAACCGGAAAGGCGGGCCAGCAGCCTGCAAGAAAA
AGATTGAATTTTGGTCAGACTGGAGACGCAGACTCAGTCCCAGACCCTCAACCAATC
GGAGAACCTCCCGCAGCCCCCTCAGGTGTGGGATCTCTTACAATGGCTGCAGGCGGT
GGCGCACCAATGGCAGACAATAACGAGGGCGCCGACGGAGTGGGTAATTCCTCGGG
AAATTGGCATTGCGATTCCACATGGATGGGCGACAGAGTCATCACCACCAGCACCCG
AACCTGGGCCCTGCCCACCTACAACAACCACCTCTACAAGCAAATCTCCAACAGCAC
ATCTGGAGGATCTTCAAATGACAACGCCTACTTCGGCTACAGCACCCCCTGGGGGTAT
TTTGACTTTAACAGATTCCACTGCCACTTTTCACCACGTGACTGGCAGCGACTCATCA
ACAACAACTGGGGATTCCGGCCCAAGAGACTCAGCTTCAAGCTCTTCAACATCCAGG
TCAAGGAGGTCACGCAGAATGAAGGCACCAAGACCATCGCCAATAACCTCACCAGC
ACCATCCAGGTGTTTACGGACTCGGAGTACCAGCTGCCGTACGTTCTCGGCTCTGCCC
ACCAGGGCTGCCTGCCTCCGTTCCCGGCGGACGTGTTCATGATTCCCCAGTACGGCTA
CCTAACACTCAACAACGGTAGTCAGGCCGTGGGACGCTCCTCCTTCTACTGCCTGGA
ATACTTTCCTTCGCAGATGCTGAGAACCGGCAACAACTTCCAGTTTACTTACACCTTC
GAGGACGTGCCTTTCCACAGCAGCTACGCCCACAGCCAGAGCTTGGACCGGCTGATG
AATCCTCTGATTGACCAGTACCTGTACTACTTGTCTCGGACTCAAACAACAGGAGGCA
CGACAAATACGCAGACTCTGGGCTTCAGCCAAGGTGGGCCTAATACAATGGCCAATC

AGGCAAAGAACTGGCTGCCAGGACCCTGTTACCGCCAGCAGCGAGTATCAAAGACA
TCTGCGGATAACAACAACAGTGAATACTCGTGGACTGGAGCTACCAAGTACCACCTC
AATGGCAGAGACTCTCTGGTGAATCCGGGCCCGGCCATGGCAAGCCACAAGGACGAT
GAAGAAAAGTTTTTTCCTCAGAGCGGGGTTCTCATCTTTGGGAAGCAAGGCTCAGAG
AAAACAAATGTGGACATTGAAAAGGTCATGATTACAGACGAAGAGGAAATCAGGAC
AACCAATCCCGTGGCTACGGAGCAGTATGGTTCTGTATCTACCAACCTCCAGAGAGGC
CAGAGAGGCGAACCAAAAGCACGTGCACCAGCCCAGGCGGCCACCGCAGATGTCAA
CACACAAGGCGTTCTTCCAGGCATGGTCTGGCAGGACAGAGATGTGTACCTTCAGGG
GCCCATCTGGGCAAAGATTCCACACACGGACGGACATTTTCACCCCTCTCCCCTCATG
GGTGGATTCGGACTTAAACACCCTCCGCCTCAGATCCTGATCAAGAACACGCCTGTA
CCTGCGGATCCTCCGACCACCTTCAACCAGTCAAAGCTGAACTCTTTCATCACCCAGT
ATTCTACTGGCCAAGTCAGCGTGGAGATCGAGTGGGAGCTGCAGAAGGAAAACAGC
AAGCGCTGGAACCCCGAGATCCAGTACACCTCCAACTACTACAAATCTACAAGTGTG
GACTTTGCTGTTAATACAGAAGGCGTGTACTCTGAACCCCGCCCCATTGGCACCCGTT
ACCTCACCCGTAATCTGTAA

An example of the Rep-Cap plasmid nucleotide sequence encoding the AAVDJ1 capsid in the present invention (SEQ ID NO: 7) is as follows. The underlined region corresponds to the AAVDJ1 capsid.

CCGCCATGCCGGGGTTTTACGAGATTGTGATTAAGGTCCCCAGCGACCTTGACGAGC
ATCTGCCCGGCATTTCTGACAGCTTTGTGAACTGGGTGGCCGAGAAGGAATGGGAGT
TGCCGCCAGATTCTGACATGGATCTGAATCTGATTGAGCAGGCACCCCTGACCGTGGC
CGAGAAGCTGCAGCGCGACTTTCTGACGGAATGGCGCCGTGTGAGTAAGGCCCCGG
AGGCCCTTTTCTTTGTGCAATTTGAGAAGGGAGAGAGCTACTTCCACATGCACGTGCT
CGTGGAAACCACCGGGGTGAAATCCATGGTTTTGGGACGTTTCCTGAGTCAGATTCG
CGAAAAACTGATTCAGAGAATTTACCGCGGGATCGAGCCGACTTTGCCAAACTGGTT
CGCGGTCACAAAGACCAGAAATGGCGCCGGAGGCGGGAACAAGGTGGTGGATGAGT
GCTACATCCCCAATTACTTGCTCCCCAAAACCCAGCCTGAGCTCCAGTGGGCGTGGA
CTAATATGGAACAGTATTTAAGCGCCTGTTTGAATCTCACGGAGCGTAAACGGTTGGT
GGCGCAGCATCTGACGCACGTGTCGCAGACGCAGGAGCAGAACAAAGAGAATCAGA

ATCCCAATTCTGATGCGCCGGTGATCAGATCAAAAACTTCAGCCAGGTACATGGAGCT
GGTCGGGTGGCTCGTGGACAAGGGGATTACCTCGGAGAAGCAGTGGATCCAGGAGG
ACCAGGCCTCATACATCTCCTTCAATGCGGCCTCCAACTCGCGGTCCCAAATCAAGGC
TGCCTTGGACAATGCGGGAAAGATTATGAGCCTGACTAAAACCGCCCCCGACTACCT
GGTGGGCCAGCAGCCCGTGGAGGACATTTCCAGCAATCGGATTTATAAAATTTTGGA
ACTAAACGGGTACGATCCCCAATATGCGGCTTCCGTCTTTCTGGGATGGGCCACGAAA
AAGTTCGGCAAGAGGAACACCATCTGGCTGTTTGGGCCTGCAACTACCGGGAAGAC
CAACATCGCGGAGGCCATAGCCCACACTGTGCCCTTCTACGGGTGCGTAAACTGGAC
CAATGAGAACTTTCCCTTCAACGACTGTGTCGACAAGATGGTGATCTGGTGGGAGGA
GGGGAAGATGACCGCCAAGGTCGTGGAGTCGGCCAAAGCCATTCTCGGAGGAAGCA
AGGTGCGCGTGGACCAGAAATGCAAGTCCTCGGCCCAGATAGACCCGACTCCCGTGA
TCGTCACCTCCAACACCAACATGTGCGCCGTGATTGACGGGAACTCAACGACCTTCG
AACACCAGCAGCCGTTGCAAGACCGGATGTTCAAATTTGAACTCACCCGCCGTCTGG
ATCATGACTTTGGGAAGGTCACCAAGCAGGAAGTCAAAGACTTTTTCCGGTGGGCAA
AGGATCACGTGGTTGAGGTGGAGCATGAATTCTACGTCAAAAAGGGTGGAGCCAAG
AAAAGACCCGCCCCCAGTGACGCAGATATAAGTGAGCCCAAACGGGTGCGCGAGTC
AGTTGCGCAGCCATCGACGTCAGACGCGGAAGCTTCGATCAACTACGCAGACAGGTA
CCAAAACAAATGTTCTCGTCACGTGGGCATGAATCTGATGCTGTTTCCCTGCAGACAA
TGCGAGAGAATGAATCAGAATTCAAATATCTGCTTCACTCACGGACAGAAAGACTGT
TTAGAGTGCTTTCCCGTGTCAGAATCTCAACCCGTTTCTGTCGTCAAAAAGGCGTATC
AGAAACTGTGCTACATTCATCATATCATGGGAAAGGTGCCAGACGCTTGCACTGCCTG
CGATCTGGTCAATGTGGATTTGGATGACTGCATCTTTGAACAATAAATGATTTAAATCA
GGTATGGCTGCCGATGGTTATCTTCCAGATTGGCTCGAGGACACTCTCTCTGAAGGAA
TAAGACAGTGGTGGAAGCTCAAACCTGGCCCACCACCACCAAAGCCCGCAGAGCGG
CATAAGGACGACAGCAGGGGTCTTGTGCTTCCTGGGTACAAGTACCTCGGACCCTTC
AACGGACTCGACAAGGGAGAGCCGGTCAACGAGGCAGACGCCGCGGCCCTCGAGC
ACGACAAAGCCTACGACCGGCAGCTCGACAGCGGAGACAACCCGTACCTCAAGTAC
AACCACGCCGACGCCGAGTTCCAGGAGCGGCTCAAAGAAGATACGTCTTTTGGGGG
CAACCTCGGGCGAGCAGTCTTCCAGGCCAAAAAGAGGCTTCTTGAACCTCTTGGTCT
GGTTGAGGAAGCGGCTAAGACGGCTCCTGGAAAGAAGAGGCCTGTAGAGCACTCTC

CTGTGGAGCCAGACTCCTCCTCGGGAACCGGAAAGGCGGGCCAGCAGCCTGCAAGA
AAAAGATTGAATTTTGGTCAGACTGGAGACGCAGACTCAGTCCCAGACCCTCAACCA
ATCGGAGAACCTCCCGCAGCCCCCTCAGGTGTGGGATCTCTTACAATGGCTGCAGGC
GGTGGCGCACCAATGGCAGACAATAACGAGGGCGCCGACGGAGTGGGTAATTCCTC
GGGAAATTGGCATTGCGATTCCACATGGATGGGCGACAGAGTCATCACCACCAGCAC
CCGAACCTGGGCCCTGCCCACCTACAACAACCACCTCTACAAGCAAATCTCCAACAG
CACATCTGGAGGATCTTCAAATGACAACGCCTACTTCGGCTACAGCACCCCCTGGGG
GTATTTTGACTTTAACAGATTCCACTGCCACTTTTCACCACGTGACTGGCAGCGACTC
ATCAACAACAACTGGGGATTCCGGCCCAAGAGACTCAGCTTCAAGCTCTTCAACATC
CAGGTCAAGGAGGTCACGCAGAATGAAGGCACCAAGACCATCGCCAATAACCTCAC
CAGCACCATCCAGGTGTTTACGGACTCGGAGTACCAGCTGCCGTACGTTCTCGGCTCT
GCCCACCAGGGCTGCCTGCCTCCGTTCCCGGCGGACGTGTTCATGATTCCCCAGTACG
GCTACCTAACACTCAACAACGGTAGTCAGGCCGTGGGACGCTCCTCCTTCTACTGCCT
GGAATACTTTCCTTCGCAGATGCTGAGAACCGGCAACAACTTCCAGTTTACTTACACC
TTCGAGGACGTGCCTTTCCACAGCAGCTACGCCCACAGCCAGAGCTTGGACCGGCTG
ATGAATCCTCTGATTGACCAGTACCTGTACTACTTGTCTCGGACTCAAACAACAGGAG
GCACGACAAATACGCAGACTCTGGGCTTCAGCCAAGGTGGGCCTAATACAATGGCCA
ATCAGGCAAAGAACTGGCTGCCAGGACCCTGTTACCGCCAGCAGCGAGTATCAAAG
ACATCTGCGGATAACAACAACAGTGAATACTCGTGGACTGGAGCTACCAAGTACCAC
CTCAATGGCAGAGACTCTCTGGTGAATCCGGGCCCGGCCATGGCAAGCCACAAGGAC
GATGAAGAAAAGTTTTTTCCTCAGAGCGGGGTTCTCATCTTTGGGAAGCAAGGCTCA
GAGAAAACAAATGTGGACATTGAAAAGGTCATGATTACAGACGAAGAGGAAATCAG
GACAACCAATCCCGTGGCTACGGAGCAGTATGGTTCTGTATCTACCAACCTCCAGAGA
GGCCAGAGAGGCGAACCAAAAGCACGTGCACCAGCCCAGGCGGCCACCGCAGATGT
CAACACACAAGGCGTTCTTCCAGGCATGGTCTGGCAGGACAGAGATGTGTACCTTCA
GGGGCCCATCTGGGCAAAGATTCCACACACGGACGGACATTTTCACCCCTCTCCCCT
CATGGGTGGATTCGGACTTAAACACCCTCCGCCTCAGATCCTGATCAAGAACACGCCT
GTACCTGCGGATCCTCCGACCACCTTCAACCAGTCAAAGCTGAACTCTTTCATCACCC
AGTATTCTACTGGCCAAGTCAGCGTGGAGATCGAGTGGGAGCTGCAGAAGGAAAAC
AGCAAGCGCTGGAACCCCGAGATCCAGTACACCTCCAACTACTACAAATCTACAAGT

GTGGACTTTGCTGTTAATACAGAAGGCGTGTACTCTGAACCCCGCCCCATTGGCACCC
GTTACCTCACCCGTAATCTGTAATTGCCTGTTAATCAATAAACCGGTTGATTCGTTTCA
GTTGAACTTTGGTCTCTGCGAAGGGCGAATTCGTTTAAACCTGCAGGACTAGAGGTC
CTGTATTAGAGGTCACGTGAGTGTTTTGCGACATTTTGCGACACCATGTGGTCACGCT
GGGTATTTAAGCCCGAGTGAGCACGCAGGGTCTCCATTTTGAAGCGGGAGGTTTGAA
CGCGCAGCCGCCAAGCCGAATTCTGCAGATATCCATCACACTGGCGGCCGCTCGACT
AGAGCGGCCGCCACCGCGGTGGAGCTCCAGCTTTTGTTCCCTTTAGTGAGGGTTAAT
TGCGCGCTTGGCGTAATCATGGTCATAGCTGTTTCCTGTGTGAAATTGTTATCCGCTCA
CAATTCCACACAACATACGAGCCGGAAGCATAAAGTGTAAAGCCTGGGGTGCCTAAT
GAGTGAGCTAACTCACATTAATTGCGTTGCGCTCACTGCCCGCTTTCCAGTCGGGAAA
CCTGTCGTGCCAGCTGCATTAATGAATCGGCCAACGCGCGGGGAGAGGCGGTTTGCG
TATTGGGCGCTCTTCCGCTTCCTCGCTCACTGACTCGCTGCGCTCGGTCGTTCGGCTG
CGGCGAGCGGTATCAGCTCACTCAAAGGCGGTAATACGGTTATCCACAGAATCAGGG
GATAACGCAGGAAAGAACATGTGAGCAAAAGGCCAGCAAAAGGCCAGGAACCGTA
AAAAGGCCGCGTTGCTGGCGTTTTTCCATAGGCTCCGCCCCCCTGACGAGCATCACA
AAAATCGACGCTCAAGTCAGAGGTGGCGAAACCCGACAGGACTATAAAGATACCAG
GCGTTTCCCCCTGGAAGCTCCCTCGTGCGCTCTCCTGTTCCGACCCTGCCGCTTACCG
GATACCTGTCCGCCTTTCTCCCTTCGGGAAGCGTGGCGCTTTCTCATAGCTCACGCTG
TAGGTATCTCAGTTCGGTGTAGGTCGTTCGCTCCAAGCTGGGCTGTGTGCACGAACCC
CCCGTTCAGCCCGACCGCTGCGCCTTATCCGGTAACTATCGTCTTGAGTCCAACCCGG
TAAGACACGACTTATCGCCACTGGCAGCAGCCACTGGTAACAGGATTAGCAGAGCGA
GGTATGTAGGCGGTGCTACAGAGTTCTTGAAGTGGTGGCCTAACTACGGCTACACTAG
AAGAACAGTATTTGGTATCTGCGCTCTGCTGAAGCCAGTTACCTTCGGAAAAAGAGT
TGGTAGCTCTTGATCCGGCAAACAAACCACCGCTGGTAGCGGTGGTTTTTTTGTTTGC
AAGCAGCAGATTACGCGCAGAAAAAAAGGATCTCAAGAAGATCCTTTGATCTTTTCT
ACGGGGTCTGACGCTCAGTGGAACGAAAACTCACGTTAAGGGATTTTGGTCATGAGA
TTATCAAAAAGGATCTTCACCTAGATCCTTTTAAATTAAAAATGAAGTTTTAAATCAAT
CTAAAGTATATATGAGTAAACTTGGTCTGACAGTTACCAATGCTTAATCAGTGAGGCA
CCTATCTCAGCGATCTGTCTATTTCGTTCATCCATAGTTGCCTGACTCCCCGTCGTGTA
GATAACTACGATACGGGAGGGCTTACCATCTGGCCCCAGTGCTGCAATGATACCGCGA

GACCCACGCTCACCGGCTCCAGATTTATCAGCAATAAACCAGCCAGCCGGAAGGGCC
GAGCGCAGAAGTGGTCCTGCAACTTTATCCGCCTCCATCCAGTCTATTAATTGTTGCC
GGGAAGCTAGAGTAAGTAGTTCGCCAGTTAATAGTTTGCGCAACGTTGTTGCCATTGC
TACAGGCATCGTGGTGTCACGCTCGTCGTTTGGTATGGCTTCATTCAGCTCCGGTTCC
CAACGATCAAGGCGAGTTACATGATCCCCCATGTTGTGCAAAAAAGCGGTTAGCTCC
TTCGGTCCTCCGATCGTTGTCAGAAGTAAGTTGGCCGCAGTGTTATCACTCATGGTTAT
GGCAGCACTGCATAATTCTCTTACTGTCATGCCATCCGTAAGATGCTTTTCTGTGACTG
GTGAGTACTCAACCAAGTCATTCTGAGAATAGTGTATGCGGCGACCGAGTTGCTCTTG
CCCGGCGTCAATACGGGATAATACCGCGCCACATAGCAGAACTTTAAAAGTGCTCATC
ATTGGAAAACGTTCTTCGGGGCGAAAACTCTCAAGGATCTTACCGCTGTTGAGATCC
AGTTCGATGTAACCCACTCGTGCACCCAACTGATCTTCAGCATCTTTTACTTTCACCA
GCGTTTCTGGGTGAGCAAAAACAGGAAGGCAAAATGCCGCAAAAAAGGGAATAAGG
GCGACACGGAAATGTTGAATACTCATACTCTTCCTTTTTCAATATTATTGAAGCATTTAT
CAGGGTTATTGTCTCATGAGCGGATACATATTTGAATGTATTTAGAAAAATAAACAAAT
AGGGGTTCCGCGCACATTTCCCCGAAAAGTGCCACCTAAATTGTAAGCGTTAATATTT
TGTTAAAATTCGCGTTAAATTTTTGTTAAATCAGCTCATTTTTTAACCAATAGGCCGAA
ATCGGCAAAATCCCTTATAAATCAAAAGAATAGACCGAGATAGGGTTGAGTGTTGTTC
CAGTTTGGAACAAGAGTCCACTATTAAAGAACGTGGACTCCAACGTCAAAGGGCGA
AAAACCGTCTATCAGGGCGATGGCCCACTACGTGAACCATCACCCTAATCAAGTTTTT
TGGGGTCGAGGTGCCGTAAAGCACTAAATCGGAACCCTAAAGGGAGCCCCCGATTTA
GAGCTTGACGGGGAAAGCCGGCGAACGTGGCGAGAAAGGAAGGGAAGAAAGCGAA
AGGAGCGGGCGCTAGGGCGCTGGCAAGTGTAGCGGTCACGCTGCGCGTAACCACCA
CACCCGCCGCGCTTAATGCGCCGCTACAGGGCGCGTCCCATTCGCCATTCAGGCTGCG
CAACTGTTGGGAAGGGCGATCGGTGCGGGCCTCTTCGCTATTACGCCAGCTGGCGAA
AGGGGGATGTGCTGCAAGGCGATTAAGTTGGGTAACGCCAGGGTTTTCCCAGTCACG
ACGTTGTAAAACGACGGCCAGTGAGCGCGCGTAATACGACTCACTATAGGGCGAATT
GGGTACCGGGCCCCCCCTCGATCGAGGTCGACGGTATCGGGGGAGCTCGCAGGGTCT
CCATTTTGAAGCGGGAGGTTTGAACGCGCAG

### 3. Experimental Results 1

The present invention will be described in detail below based on examples and the like; however, the invention is not limited to these.

### [Example 1]

Figure 1 shows the results of a comparative study on the gene transduction efficiency of AAVDJ and AAVDJ1 in mouse dorsal skin ulcers. Capsid plasmids, along with vector plasmids expressing GFPNLS (NLS stands for nuclear localization signal, and the fluorescent protein with this sequence emits fluorescence localized to the cell nucleus) and mCherryNLS (SEQ ID NO: 8, plasmid nucleotide sequence 2; SEQ ID NO: 9, plasmid nucleotide sequence 3) under the control of the CAG promoter, were prepared. Additionally, pAD5 plasmid was used as a helper plasmid. AAVs were produced using calcium phosphate transfection in 293AAV cells and purified via ultracentrifugation according to standard methods (see Non-Patent Document 2). The following AAV vectors were prepared: AAVDJ-GFPNLS, AAVDJ-mCherryNLS, AAVDJ1-GFPNLS, and AAVDJ1-mCherryNLS. Four combinations of these vectors were prepared, each containing 10¹¹ GC in 50 µl of viral solution:
1. AAVDJ-GFPNLS + AAVDJ-mCherryNLS
2. AAVDJ1-GFPNLS + AAVDJ1-mCherryNLS
3. AAVDJ-GFPNLS + AAVDJ1-mCherryNLS
4. AAVDJ1-GFPNLS + AAVDJ-mCherryNLS
Each combination was applied into the ulcerated areas inside silicone chambers attached to the dorsal skin of five mice per group. Four days later, the ulcerated areas were imaged using a fluorescence stereomicroscope (Axio Zoom .V16, Zeiss). Subsequently, tissues including the ulcerated areas were collected, fixed in 4% paraformaldehyde, embedded in OCT compound, and processed into frozen sections. The sections stained with DAPI for nuclear staining, as well as adjacent sections stained with hematoxylin and eosin (HE), were scanned into image files using a fluorescence slide scanner (VS200, Olympus). During this process, the exposure times for green fluorescence imaging and red fluorescence imaging were adjusted using samples from groups in which gene transduction was performed with the same capsid virus. Using adjacent HE-stained serial sections as references, the regions of DAPI-stained sections were classified into the superficial layer (above the fascia of the subcutaneous adipose tissue), the fat layer (subcutaneous adipose tissue), and the muscle layer (muscles such as the trapezius). To segment the nuclei in each layer, a UNet++ model was created and trained using the RMSprop optimizer and a binary cross-entropy dice loss (BCE-dice-loss) function. To generate training data, images were cropped into 512×512 pixel sections and applied to the model training with 400 epochs, a batch size of 8, and a learning rate of 10⁻⁴. After training, the model was applied to the entire dataset to predict nuclear regions, and the probability of each pixel was calculated within the range [0, 1]. Pixels with probabilities in the range [0.5, 1] were extracted as nuclear regions, which were then segmented using the Watershed algorithm. Nuclei containing five or more fluorescence-positive pixels were defined as nuclei of gene transduction-positive cells. The number of gene transduction-positive cells was counted for each region and layer. For each sample, 10 to 16 sections (average: 14.4) were analyzed.

As a result, fluorescence stereomicroscopy observations of the ulcerated areas revealed that groups transduced with the same capsid showed similar signals under both green fluorescence and red fluorescence imaging. However, in groups transduced with different capsids, distinct signals were observed under green and red fluorescence imaging. In the analysis of the number of gene transduction-positive nuclei based on histological observations, higher numbers of gene transduction-positive cells were observed with AAVDJ1 capsid viruses compared to AAVDJ capsid viruses in both the fat layer and muscle layer in samples from groups such as AAVDJ-GFPNLS + AAVDJ1-mCherryNLS and AAVDJ1-GFPNLS + AAVDJ-mCherryNLS. These findings demonstrate that AAVDJ1 has significantly higher gene transduction efficiency than AAVDJ, particularly in deep tissues such as the fat layer and muscle layer.

The sequence of the vector plasmid pAAV-CAG-GFPNLS expressing GFPNLS (SEQ ID NO: 8) is as follows.

CTTCCGCTTCCTCGCTCACTGACTCGCTGCGCTCGGTCGTTCGGCTGCGGCGAGCGG
TATCAGCTCACTCAAAGGCGGTAATACGGTTATCCACAGAATCAGGGGATAACGCA
GGAAAGAACATGTGAGCAAAAGGCCAGCAAAAGGCCAGGAACCGTAAAAAGGCCG
CGTTGCTGGCGTTTTTCCATAGGCTCCGCCCCCCTGACGAGCATCACAAAAATCGAC
GCTCAAGTCAGAGGTGGCGAAACCCGACAGGACTATAAAGATACCAGGCGTTTCCC
CCTGGAAGCTCCCTCGTGCGCTCTCCTGTTCCGACCCTGCCGCTTACCGGATACCTGT

CCGCCTTTCTCCCTTCGGGAAGCGTGGCGCTTTCTCATAGCTCACGCTGTAGGTATCT
CAGTTCGGTGTAGGTCGTTCGCTCCAAGCTGGGCTGTGTGCACGAACCCCCCGTTCA
GCCCGACCGCTGCGCCTTATCCGGTAACTATCGTCTTGAGTCCAACCCGGTAAGACA
CGACTTATCGCCACTGGCAGCAGCCACTGGTAACAGGATTAGCAGAGCGAGGTATG
TAGGCGGTGCTACAGAGTTCTTGAAGTGGTGGCCTAACTACGGCTACACTAGAAGA
ACAGTATTTGGTATCTGCGCTCTGCTGAAGCCAGTTACCTTCGGAAAAAGAGTTGGT
AGCTCTTGATCCGGCAAACAAACCACCGCTGGTAGCGGTGGTTTTTTTGTTTGCAAG
CAGCAGATTACGCGCAGAAAAAAAGGATCTCAAGAAGATCCTTTGATCTTTTCTACG
GGGTCTGACGCTCAGTGGAACGAAAACTCACGTTAAGGGATTTTGGTCATGAGATTA
TCAAAAAGGATCTTCACCTAGATCCTTTTAAATTAAAAATGAAGTTTTAAATCAATC
TAAAGTATATATGAGTAAACTTGGTCTGACAGTTACCAATGCTTAATCAGTGAGGCA
CCTATCTCAGCGATCTGTCTATTTCGTTCATCCATAGTTGCCTGACTCCCCGTCGTGT
AGATAACTACGATACGGGAGGGCTTACCATCTGGCCCCAGTGCTGCAATGATACCG
CGAGACCCACGCTCACCGGCTCCAGATTTATCAGCAATAAACCAGCCAGCCGGAAG
GGCCGAGCGCAGAAGTGGTCCTGCAACTTTATCCGCCTCCATCCAGTCTATTAATTG
TTGCCGGGAAGCTAGAGTAAGTAGTTCGCCAGTTAATAGTTTGCGCAACGTTGTTGC
CATTGCTACAGGCATCGTGGTGTCACGCTCGTCGTTTGGTATGGCTTCATTCAGCTCC
GGTTCCCAACGATCAAGGCGAGTTACATGATCCCCCATGTTGTGCAAAAAAGCGGTT
AGCTCCTTCGGTCCTCCGATCGTTGTCAGAAGTAAGTTGGCCGCAGTGTTATCACTC
ATGGTTATGGCAGCACTGCATAATTCTCTTACTGTCATGCCATCCGTAAGATGCTTTT
CTGTGACTGGTGAGTACTCAACCAAGTCATTCTGAGAATAGTGTATGCGGCGACCGA
GTTGCTCTTGCCCGGCGTCAATACGGGATAATACCGCGCCACATAGCAGAACTTTAA
AAGTGCTCATCATTGGAAAACGTTCTTCGGGGCGAAAACTCTCAAGGATCTTACCGC
TGTTGAGATCCAGTTCGATGTAACCCACTCGTGCACCCAACTGATCTTCAGCATCTTT
TACTTTCACCAGCGTTTCTGGGTGAGCAAAAACAGGAAGGCAAAATGCCGCAAAAA
AGGGAATAAGGGCGACACGGAAATGTTGAATACTCATACTCTTCCTTTTTCAATATT
ATTGAAGCATTTATCAGGGTTATTGTCTCATGAGCGGATACATATTTGAATGTATTTA
GAAAAATAAACAAATAGGGGTTCCGCGCACATTTCCCCGAAAAGTGCCACCTAAAT
TGTAAGCGTTAATATTTTGTTAAAATTCGCGTTAAATTTTTGTTAAATCAGCTCATTT
TTTAACCAATAGGCCGAAATCGGCAAAATCCCTTATAAATCAAAAGAATAGACCGA

GATAGGGTTGAGTGTTGTTCCAGTTTGGAACAAGAGTCCACTATTAAAGAACGTGGA
CTCCAACGTCAAAGGGCGAAAAACCGTCTATCAGGGCGATGGCCCACTACGTGAAC
CATCACCCTAATCAAGTTTTTTGGGGTCGAGGTGCCGTAAAGCACTAAATCGGAACC
CTAAAGGGAGCCCCCGATTTAGAGCTTGACGGGGAAAGCCGGCGAACGTGGCGAGA
AAGGAAGGGAAGAAAGCGAAAGGAGCGGGCGCTAGGGCGCTGGCAAGTGTAGCGG
TCACGCTGCGCGTAACCACCACACCCGCCGCGCTTAATGCGCCGCTACAGGGCGCGT
CCCATTCGCCATTCAGGCTGCGCAACTGTTGGGAAGGGCGATCGGTGCGGGCCTCTT
CGCTATTACGCCAGCTGCGCGCTCGCTCGCTCACTGAGGCCGCCCGGGCAAAGCCCG
GGCGTCGGGCGACCTTTGGTCGCCCGGCCTCAGTGAGCGAGCGAGCGCGCAGAGAG
GGAGTGGCCAACTCCATCACTAGGGGTTCCTTGTAGTTAATGATTAACCCGCCATGC
TACTTATCTACGTAGCCATGCTCTAGGAAGAGTACCATTGACGTCAATAATGACGTA
TGTTCCCATAGTAACGCCAATAGGGACTTTCCATTGACGTCAATGGGTGGAGTATTT
ACGGTAAACTGCCCACTTGGCAGTACATCAAGTGTATCATATGCCAAGTACGCCCCC
TATTGACGTCAATGACGGTAAATGGCCCGCCTGGCATTATGCCCAGTACATGACCTT
ATGGGACTTTCCTACTTGGCAGTACATCTACGTATTAGTCATCGCTATTACCATGGTC
GAGGTGAGCCCCACGTTCTGCTTCACTCTCCCCATCTCCCCCCCCTCCCCACCCCCAA
TTTTGTATTTATTTATTTTTTAATTATTTTGTGCAGCGATGGGGGCGGGGGGGGGGG
GGGGGCGCGCGCCGGGCGGGGCGGGGCGGGGCGAGGGGCGGGGCGGGGCGAGGCG
GAGAGGTGCGGCGGCAGCCAATCAGAGCGGCGCGCTCCGAAAGTTTCCTTTTATGG
CGAGGCGGCGGCGGCGGCGGCCCTATAAAAAGCGAAGCGCGCGGCGGGCGGGAGT
CGCTGCGCGCTGCCTTCGCCCCGTGCCCCGCTCCGCCGCCGCCTCGCGCCGCCCGCC
CCGGCTCTGACTGACCGCGTTACTCCCACAGGTGAGCGGGCGGGACGGCCCTTCTCC
TCCGGGCTGTAATTAGCGCTTGGTTTAATGACGGCTTGTTTCTTTTCTGTGGCTGCGT
GAAAGCCTTGAGGGGCTCCGGGAGGGCCCTTTGTGCGGGGGGAGCGGCTCGGGGCT
GTCCGCGGGGGGACGGCTGCCTTCGGGGGGGACGGGGCAGGGCGGGGTTCGGCTTC
TGGCGTGTGACCGGCGGCTCTAGAGCCTCTGCTAACCATGTTCATGCCTTCTTCTTTT
TCCTACAGCTCCTGGGCAACGTGCTGGTTATTGTGCTGTCTCATCATTTTGGCAAAGA
ATTGGATCCGCCACCATGGTGAGCAAGGGCGAGGAGCTGTTCACCGGGGTGGTGCC
CATCCTGGTCGAGCTGGACGGCGACGTAAACGGCCACAAGTTCAGCGTGTCCGGCG
AGGGCGAGGGCGATGCCACCTACGGCAAGCTGACCCTGAAGTTCATTTGCACCACC

GGCAAGCTGCCCGTGCCCTGGCCCACCCTCGTGACCACCCTGACCTACGGCGTGCAG
TGCTTCAGCCGCTACCCCGACCACATGAAGCAGCACGACTTCTTCAAGTCCGCCATG
CCCGAAGGCTACGTCCAGGAGCGCACCATCTTCTTCAAGGACGACGGCAACTACAA
GACCCGCGCCGAGGTGAAGTTCGAGGGCGACACCCTGGTGAACCGCATCGAGCTGA
AGGGCATCGACTTCAAGGAGGACGGCAACATCCTGGGGCACAAGCTGGAGTACAAC
TACAACAGCCACAACGTCTATATCATGGCCGACAAGCAGAAGAACGGCATCAAGGT
GAACTTCAAGATCCGCCACAACATCGAGGACGGCAGCGTGCAGCTCGCCGACCACT
ACCAGCAGAACACCCCCATCGGCGACGGCCCCGTGCTGCTGCCCGACAACCACTAC
CTGAGCACCCAGTCCGCCCTGAGCAAAGACCCCAACGAGAAGCGCGATCACATGGT
CCTGCTGGAGTTCGTGACCGCCGCCGGGATCACTCTCGGCATGGACGAGCTGTACAA
GTCCGGACTCAGATCTGATCCAAAAAAGAAGAGAAAGGTAGATCCAAAAAAGAAG
AGAAAGGTAGATCCAAAAAAGAAGAGAAAGGTATAAGAATTCGATATCAAGCTTAT
CGATAATCAACCTCTGGATTACAAAATTTGTGAAAGATTGACTGGTATTCTTAACTA
TGTTGCTCCTTTTACGCTATGTGGATACGCTGCTTTAATGCCTTTGTATCATGCTATT
GCTTCCCGTATGGCTTTCATTTTCTCCTCCTTGTATAAATCCTGGTTGCTGTCTCTTTA
TGAGGAGTTGTGGCCCGTTGTCAGGCAACGTGGCGTGGTGTGCACTGTGTTTGCTGA
CGCAACCCCCACTGGTTGGGGCATTGCCACCACCTGTCAGCTCCTTTCCGGGACTTT
CGCTTTCCCCCTCCCTATTGCCACGGCGGAACTCATCGCCGCCTGCCTTGCCCGCTGC
TGGACAGGGGCTCGGCTGTTGGGCACTGACAATTCCGTGGTGTTGTCGGGGAAATCA
TCGTCCTTTCCTTGGCTGCTCGCCTGTGTTGCCACCTGGATTCTGCGCGGGACGTCCT
TCTGCTACGTCCCTTCGGCCCTCAATCCAGCGGACCTTCCTTCCCGCGGCCTGCTGCC
GGCTCTGCGGCCTCTTCCGCGTCTTCGCCTTCGCCCTCAGACGAGTCGGATCTCCCTT
TGGGCCGCCTCCCCGCATCGATACCGTCGACCCGGGCGGCCGCTTCGAGCAGACAT
GATAAGATACATTGATGAGTTTGGACAAACCACAACTAGAATGCAGTGAAAAAAAT
GCTTTATTTGTGAAATTTGTGATGCTATTGCTTTATTTGTAACCATTATAAGCTGCAA
TAAACAAGTTAACAACAACAATTGCATTCATTTTATGTTTCAGGTTCAGGGGGAGAT
GTGGGAGGTTTTTTAAAGCAAGTAAAACCTCTACAAATGTGGTAAAATCGATAAGG
ATCTTCCTAGAGCATGGCTACGTAGATAAGTAGCATGGCGGGTTAATCATTAACTAC
AAGGAACCCCTAGTGATGGAGTTGGCCACTCCCTCTCTGCGCGCTCGCTCGCTCACT
GAGGCCGGGCGACCAAAGGTCGCCCGACGCCCGGGCTTTGCCCGGGCGGCCTCAGT

GAGCGAGCGAGCGCGCAGCTGCATTAATGAATCGGCCAACGCGCGGGGAGAGGCG
GTTTGCGTATTGGGCGCT

The sequence of the vector plasmid pAAV-CAG-mCherryNLS expressing mCherryNLS (SEQ ID NO: 9) is as follows.

CTTCCGCTTCCTCGCTCACTGACTCGCTGCGCTCGGTCGTTCGGCTGCGGCGAGCGG
TATCAGCTCACTCAAAGGCGGTAATACGGTTATCCACAGAATCAGGGGATAACGCA
GGAAAGAACATGTGAGCAAAAGGCCAGCAAAAGGCCAGGAACCGTAAAAAGGCCG
CGTTGCTGGCGTTTTTCCATAGGCTCCGCCCCCCTGACGAGCATCACAAAAATCGAC
GCTCAAGTCAGAGGTGGCGAAACCCGACAGGACTATAAAGATACCAGGCGTTTCCC
CCTGGAAGCTCCCTCGTGCGCTCTCCTGTTCCGACCCTGCCGCTTACCGGATACCTGT
CCGCCTTTCTCCCTTCGGGAAGCGTGGCGCTTTCTCATAGCTCACGCTGTAGGTATCT
CAGTTCGGTGTAGGTCGTTCGCTCCAAGCTGGGCTGTGTGCACGAACCCCCCGTTCA
GCCCGACCGCTGCGCCTTATCCGGTAACTATCGTCTTGAGTCCAACCCGGTAAGACA
CGACTTATCGCCACTGGCAGCAGCCACTGGTAACAGGATTAGCAGAGCGAGGTATG
TAGGCGGTGCTACAGAGTTCTTGAAGTGGTGGCCTAACTACGGCTACACTAGAAGA
ACAGTATTTGGTATCTGCGCTCTGCTGAAGCCAGTTACCTTCGGAAAAAGAGTTGGT
AGCTCTTGATCCGGCAAACAAACCACCGCTGGTAGCGGTGGTTTTTTTGTTTGCAAG
CAGCAGATTACGCGCAGAAAAAAAGGATCTCAAGAAGATCCTTTGATCTTTTCTACG
GGGTCTGACGCTCAGTGGAACGAAAACTCACGTTAAGGGATTTTGGTCATGAGATTA
TCAAAAAGGATCTTCACCTAGATCCTTTTAAATTAAAAATGAAGTTTTAAATCAATC
TAAAGTATATATGAGTAAACTTGGTCTGACAGTTACCAATGCTTAATCAGTGAGGCA
CCTATCTCAGCGATCTGTCTATTTCGTTCATCCATAGTTGCCTGACTCCCCGTCGTGT
AGATAACTACGATACGGGAGGGCTTACCATCTGGCCCCAGTGCTGCAATGATACCG
CGAGACCCACGCTCACCGGCTCCAGATTTATCAGCAATAAACCAGCCAGCCGGAAG
GGCCGAGCGCAGAAGTGGTCCTGCAACTTTATCCGCCTCCATCCAGTCTATTAATTG
TTGCCGGGAAGCTAGAGTAAGTAGTTCGCCAGTTAATAGTTTGCGCAACGTTGTTGC
CATTGCTACAGGCATCGTGGTGTCACGCTCGTCGTTTGGTATGGCTTCATTCAGCTCC
GGTTCCCAACGATCAAGGCGAGTTACATGATCCCCCATGTTGTGCAAAAAAGCGGTT
AGCTCCTTCGGTCCTCCGATCGTTGTCAGAAGTAAGTTGGCCGCAGTGTTATCACTC

ATGGTTATGGCAGCACTGCATAATTCTCTTACTGTCATGCCATCCGTAAGATGCTTTT
CTGTGACTGGTGAGTACTCAACCAAGTCATTCTGAGAATAGTGTATGCGGCGACCGA
GTTGCTCTTGCCCGGCGTCAATACGGGATAATACCGCGCCACATAGCAGAACTTTAA
AAGTGCTCATCATTGGAAAACGTTCTTCGGGGCGAAAACTCTCAAGGATCTTACCGC
TGTTGAGATCCAGTTCGATGTAACCCACTCGTGCACCCAACTGATCTTCAGCATCTTT
TACTTTCACCAGCGTTTCTGGGTGAGCAAAAACAGGAAGGCAAAATGCCGCAAAAA
AGGGAATAAGGGCGACACGGAAATGTTGAATACTCATACTCTTCCTTTTTCAATATT
ATTGAAGCATTTATCAGGGTTATTGTCTCATGAGCGGATACATATTTGAATGTATTTA
GAAAAATAAACAAATAGGGGTTCCGCGCACATTTCCCCGAAAAGTGCCACCTAAAT
TGTAAGCGTTAATATTTTGTTAAAATTCGCGTTAAATTTTTGTTAAATCAGCTCATTT
TTTAACCAATAGGCCGAAATCGGCAAAATCCCTTATAAATCAAAAGAATAGACCGA
GATAGGGTTGAGTGTTGTTCCAGTTTGGAACAAGAGTCCACTATTAAAGAACGTGGA
CTCCAACGTCAAAGGGCGAAAAACCGTCTATCAGGGCGATGGCCCACTACGTGAAC
CATCACCCTAATCAAGTTTTTTGGGGTCGAGGTGCCGTAAAGCACTAAATCGGAACC
CTAAAGGGAGCCCCCGATTTAGAGCTTGACGGGGAAAGCCGGCGAACGTGGCGAGA
AAGGAAGGGAAGAAAGCGAAAGGAGCGGGCGCTAGGGCGCTGGCAAGTGTAGCGG
TCACGCTGCGCGTAACCACCACACCCGCCGCGCTTAATGCGCCGCTACAGGGCGCGT
CCCATTCGCCATTCAGGCTGCGCAACTGTTGGGAAGGGCGATCGGTGCGGGCCTCTT
CGCTATTACGCCAGCTGCGCGCTCGCTCGCTCACTGAGGCCGCCCGGGCAAAGCCCG
GGCGTCGGGCGACCTTTGGTCGCCCGGCCTCAGTGAGCGAGCGAGCGCGCAGAGAG
GGAGTGGCCAACTCCATCACTAGGGGTTCCTTGTAGTTAATGATTAACCCGCCATGC
TACTTATCTACGTAGCCATGCTCTAGGAAGAGTACCATTGACGTCAATAATGACGTA
TGTTCCCATAGTAACGCCAATAGGGACTTTCCATTGACGTCAATGGGTGGAGTATTT
ACGGTAAACTGCCCACTTGGCAGTACATCAAGTGTATCATATGCCAAGTACGCCCCC
TATTGACGTCAATGACGGTAAATGGCCCGCCTGGCATTATGCCCAGTACATGACCTT
ATGGGACTTTCCTACTTGGCAGTACATCTACGTATTAGTCATCGCTATTACCATGGTC
GAGGTGAGCCCCACGTTCTGCTTCACTCTCCCCATCTCCCCCCCCTCCCCACCCCCAA
TTTTGTATTTATTTATTTTTTAATTATTTTGTGCAGCGATGGGGGCGGGGGGGGGGG
GGGGGCGCGCGCCGGGCGGGGCGGGGCGGGGCGAGGGGCGGGGCGGGGCGAGGCG
GAGAGGTGCGGCGGCAGCCAATCAGAGCGGCGCGCTCCGAAAGTTTCCTTTTATGG

CGAGGCGGCGGCGGCGGCCCTATAAAAAGCGAAGCGCGCGGCGGGCGGGAGT
CGCTGCGCGCTGCCTTCGCCCCGTGCCCCGCTCCGCCGCCGCCTCGCGCCGCCCGCC
CCGGCTCTGACTGACCGCGTTACTCCCACAGGTGAGCGGGCGGGACGGCCCTTCTCC
TCCGGGCTGTAATTAGCGCTTGGTTTAATGACGGCTTGTTTCTTTTCTGTGGCTGCGT
GAAAGCCTTGAGGGGCTCCGGGAGGGCCCTTTGTGCGGGGGGAGCGGCTCGGGGCT
GTCCGCGGGGGGACGGCTGCCTTCGGGGGGGACGGGGCAGGGCGGGGTTCGGCTTC
TGGCGTGTGACCGGCGGCTCTAGAGCCTCTGCTAACCATGTTCATGCCTTCTTCTTTT
TCCTACAGCTCCTGGGCAACGTGCTGGTTATTGTGCTGTCTCATCATTTTGGCAAAGA
ATTGGATCCGCCACCATGGCATATCCGTATGATGTGCCGGATTATGCGGTGAGCAAG
GGCGAGGAGGATAACATGGCCATCATCAAGGAGTTCATGCGCTTCAAGGTGCACAT
GGAGGGCTCCGTGAACGGCCACGAGTTCGAGATCGAGGGCGAGGGCGAGGGCCGC
CCCTACGAGGGCACCCAGACCGCCAAGCTGAAGGTGACCAAGGGTGGCCCCCTGCC
CTTCGCCTGGGACATCCTGTCCCCTCAGTTCATGTACGGCTCCAAGGCCTACGTGAA
GCACCCCGCCGACATCCCCGACTACTTGAAGCTGTCCTTCCCCGAGGGCTTCAAGTG
GGAGCGCGTGATGAACTTCGAGGACGGCGGCGTGGTGACCGTGACCCAGGACTCCT
CCCTGCAGGACGGCGAGTTCATCTACAAGGTGAAGCTGCGCGGCACCAACTTCCCCT
CCGACGGCCCCGTAATGCAGAAGAAGACCATGGGCTGGGAGGCCTCCTCCGAGCGG
ATGTACCCCGAGGACGGCGCCCTGAAGGGCGAGATCAAGCAGAGGCTGAAGCTGA
AGGACGGCGGCCACTACGACGCTGAGGTCAAGACCACCTACAAGGCCAAGAAGCCC
GTGCAGCTGCCCGGCGCCTACAACGTCAACATCAAGTTGGACATCACCTCCCACAAC
GAGGACTACACCATCGTGGAACAGTACGAACGCGCCGAGGGCCGCCACTCCACCGG
CGGCATGGACGAGCTGTACAAGTCCGGACTCAGATCTGATCCAAAAAAGAAGAGAA
AGGTAGATCCAAAAAAGAAGAGAAAGGTAGATCCAAAAAAGAAGAGAAAGGTATA
AGAATTCGATATCAAGCTTATCGATAATCAACCTCTGGATTACAAAATTTGTGAAAG
ATTGACTGGTATTCTTAACTATGTTGCTCCTTTTACGCTATGTGGATACGCTGCTTTA
ATGCCTTTGTATCATGCTATTGCTTCCCGTATGGCTTTCATTTTCTCCTCCTTGTATAA
ATCCTGGTTGCTGTCTCTTTATGAGGAGTTGTGGCCCGTTGTCAGGCAACGTGGCGT
GGTGTGCACTGTGTTTGCTGACGCAACCCCCACTGGTTGGGGCATTGCCACCACCTG
TCAGCTCCTTTCCGGGACTTTCGCTTTCCCCCTCCCTATTGCCACGGCGGAACTCATC
GCCGCCTGCCTTGCCCGCTGCTGGACAGGGGCTCGGCTGTTGGGCACTGACAATTCC

GTGGTGTTGTCGGGGAAATCATCGTCCTTTCCTTGGCTGCTCGCCTGTGTTGCCACCT
GGATTCTGCGCGGGACGTCCTTCTGCTACGTCCCTTCGGCCCTCAATCCAGCGGACC
TTCCTTCCCGCGGCCTGCTGCCGGCTCTGCGGCCTCTTCCGCGTCTTCGCCTTCGCCC
TCAGACGAGTCGGATCTCCCTTTGGGCCGCCTCCCCGCATCGATACCGTCGACCCGG
GCGGCCGCTTCGAGCAGACATGATAAGATACATTGATGAGTTTGGACAAACCACAA
CTAGAATGCAGTGAAAAAAATGCTTTATTTGTGAAATTTGTGATGCTATTGCTTTATT
TGTAACCATTATAAGCTGCAATAAACAAGTTAACAACAACAATTGCATTCATTTTAT
GTTTCAGGTTCAGGGGGAGATGTGGGAGGTTTTTTAAAGCAAGTAAAACCTCTACAA
ATGTGGTAAAATCGATAAGGATCTTCCTAGAGCATGGCTACGTAGATAAGTAGCAT
GGCGGGTTAATCATTAACTACAAGGAACCCCTAGTGATGGAGTTGGCCACTCCCTCT
CTGCGCGCTCGCTCGCTCACTGAGGCCGGGCGACCAAAGGTCGCCCGACGCCCGGG
CTTTGCCCGGGCGGCCTCAGTGAGCGAGCGAGCGCGCAGCTGCATTAATGAATCGG
CCAACGCGCGGGGAGAGGCGGTTTGCGTATTGGGCGCT

### 4. Applications of AAV with Superior Gene Transduction Efficiency for Keratinocytes and Epidermal Tissues

The AAVDJK2 in the present invention, which exhibits superior gene transduction efficiency for keratinocytes and epidermal tissues, demonstrates higher gene transduction efficiency and superior tropism compared to AAV2, AAV6, and AAVDJ, which were previously known for their efficiency in keratinocytes. As such, it can be used as a gene transduction vector in gene therapy for epidermal tissues in vivo, as well as a research and development tool for gene therapy methods targeting epidermal tissues. Additionally, it can be utilized as a research tool for drug discovery and biological studies not aimed at the development of gene therapy methods. Because AAVDJK2 also exhibits high tropism for cultured keratinocytes that constitute epidermal tissues, it can serve as a gene transduction vector in regenerative medicine and gene therapy using cultured keratinocytes, as well as a research and development tool for gene therapy methods using cultured keratinocytes. Furthermore, it can be employed as a research tool for drug discovery and biological studies beyond the development of gene therapy methods using cultured keratinocytes.

### 5. Method for Producing AAV with Superior Gene Transduction Efficiency for Keratinocytes and Epidermal Tissues

The AAV in the present invention, which exhibits superior tropism for keratinocytes and epidermal tissues, includes capsids derived from the known AAVDJ capsid (SEQ ID NO: 3; amino acid sequence 1), with the NR region at the 589th and 590th amino acid positions replaced as follows:
- AAVDJK2 (SEQ ID NO: 14; amino acid sequence 3): NAAAPRGDLAPAAR (SEQ ID NO: 10)
- AAVDJK1 (SEQ ID NO: 15; amino acid sequence 4): NAAARGDTATLAAR (SEQ ID NO: 11)
- AAVDJK3 (SEQ ID NO: 16; amino acid sequence 5): NAAARGDQQSLAAR (SEQ ID NO: 12)
- AAVDJK8 (SEQ ID NO: 17; amino acid sequence 6): NAAAPRGDLRPAAR (SEQ ID NO: 13)
Each AAV is produced as an AAV capable of expressing a desired gene by general methods for AAV production, such as co-transfection of a Rep-Cap plasmid encoding the capsid sequence for each AAV, a vector plasmid carrying the target gene, and a helper plasmid, or by a baculovirus-based AAV production method. For reference, examples of nucleotide sequences are provided as follows: a nucleotide sequence encoding AAVDJK2 (SEQ ID NO: 18; capsid nucleotide sequence 2), a nucleotide sequence encoding AAVDJK1 (SEQ ID NO: 19; capsid nucleotide sequence 3), a nucleotide sequence encoding AAVDJK3 (SEQ ID NO: 20; capsid nucleotide sequence 4), a nucleotide sequence encoding AAVDJK8 (SEQ ID NO: 21; capsid nucleotide sequence 5), and a nucleotide sequence of the Rep-Cap plasmid encoding AAVDJK2 (SEQ ID NO: 22; plasmid nucleotide sequence 4).

The amino acid sequence of the AAVDJK2 capsid (SEQ ID NO: 14) is as follows. The substituted region, NAAAPRGDLAPAAR (SEQ ID NO: 10), is underlined.

MAADGYLPDWLEDTLSEGIRQWWKLKPGPPPPKPAERHKDDSRGLVLPGYKYLGPFNG
LDKGEPVNEADAAALEHDKAYDRQLDSGDNPYLKYNHADAEFQERLKEDTSFGGNLG
RAVFQAKKRLLEPLGLVEEAAKTAPGKKRPVEHSPVEPDSSSGTGKAGQQPARKRLNFG

QTGDADSVPDPQPIGEPPAAPSGVGSLTMAAGGGAPMADNNEGADGVGNSSGNWHCD
STWMGDRVITTSTRTWALPTYNNHLYKQISNSTSGGSSNDNAYFGYSTPWGYFDFNRFH
CHFSPRDWQRLINNNWGFRPKRLSFKLFNIQVKEVTQNEGTKTIANNLTSTIQVFTDSEY
QLPYVLGSAHQGCLPPFPADVFMIPQYGYLTLNNGSQAVGRSSFYCLEYFPSQMLRTGN
NFQFTYTFEDVPFHSSYAHSQSLDRLMNPLIDQYLYYLSRTQTTGGTTNTQTLGFSQGGP
NTMANQAKNWLPGPCYRQQRVSKTSADNNNSEYSWTGATKYHLNGRDSLVNPGPAMA
SHKDDEEKFFPQSGVLIFGKQGSEKTNVDIEKVMITDEEEIRTTNPVATEQYGSVSTNLQR
GNAAAPRGDLAPAARQAATADVNTQGVLPGMVWQDRDVYLQGPIWAKIPHTDGHFHP
SPLMGGFGLKHPPPQILIKNTPVPADPPTTFNQSKLNSFITQYSTGQVSVEIEWELQKENS
KRWNPEIQYTSNYYKSTSVDFAVNTEGVYSEPRPIGTRYLTRNL

The amino acid sequence of the AAVDJK1 capsid (SEQ ID NO: 15) is as follows. The substituted region, NAAARGDTATLAAR (SEQ ID NO: 11), is underlined.

MAADGYLPDWLEDTLSEGIRQWWKLKPGPPPPKPAERHKDDSRGLVLPGYKYLGPFNG
LDKGEPVNEADAAALEHDKAYDRQLDSGDNPYLKYNHADAEFQERLKEDTSFGGNLG
RAVFQAKKRLLEPLGLVEEAAKTAPGKKRPVEHSPVEPDSSSGTGKAGQQPARKRLNFG
QTGDADSVPDPQPIGEPPAAPSGVGSLTMAAGGGAPMADNNEGADGVGNSSGNWHCD
STWMGDRVITTSTRTWALPTYNNHLYKQISNSTSGGSSNDNAYFGYSTPWGYFDFNRFH
CHFSPRDWQRLINNNWGFRPKRLSFKLFNIQVKEVTQNEGTKTIANNLTSTIQVFTDSEY
QLPYVLGSAHQGCLPPFPADVFMIPQYGYLTLNNGSQAVGRSSFYCLEYFPSQMLRTGN
NFQFTYTFEDVPFHSSYAHSQSLDRLMNPLIDQYLYYLSRTQTTGGTTNTQTLGFSQGGP
NTMANQAKNWLPGPCYRQQRVSKTSADNNNSEYSWTGATKYHLNGRDSLVNPGPAMA
SHKDDEEKFFPQSGVLIFGKQGSEKTNVDIEKVMITDEEEIRTTNPVATEQYGSVSTNLQR
GNAAARGDTATLAARQAATADVNTQGVLPGMVWQDRDVYLQGPIWAKIPHTDGHFHP
SPLMGGFGLKHPPPQILIKNTPVPADPPTTFNQSKLNSFITQYSTGQVSVEIEWELQKENS
KRWNPEIQYTSNYYKSTSVDFAVNTEGVYSEPRPIGTRYLTRNL

The amino acid sequence of the AAVDJK3 capsid (SEQ ID NO: 16) is as follows. The substituted region, NAAARGDQQSLAAR (SEQ ID NO: 12), is underlined.

MAADGYLPDWLEDTLSEGIRQWWKLKPGPPPPKPAERHKDDSRGLVLPGYKYLGPFNG
LDKGEPVNEADAAALEHDKAYDRQLDSGDNPYLKYNHADAEFQERLKEDTSFGGNLG
RAVFQAKKRLLEPLGLVEEAAKTAPGKKRPVEHSPVEPDSSSGTGKAGQQPARKRLNFG
QTGDADSVPDPQPIGEPPAAPSGVGSLTMAAGGGAPMADNNEGADGVGNSSGNWHCD
STWMGDRVITTSTRTWALPTYNNHLYKQISNSTSGGSSNDNAYFGYSTPWGYFDFNRFH
CHFSPRDWQRLINNNWGFRPKRLSFKLFNIQVKEVTQNEGTKTIANNLTSTIQVFTDSEY
QLPYVLGSAHQGCLPPFPADVFMIPQYGYLTLNNGSQAVGRSSFYCLEYFPSQMLRTGN
NFQFTYTFEDVPFHSSYAHSQSLDRLMNPLIDQYLYYLSRTQTTGGTTNTQTLGFSQGGP
NTMANQAKNWLPGPCYRQQRVSKTSADNNNSEYSWTGATKYHLNGRDSLVNPGPAMA
SHKDDEEKFFPQSGVLIFGKQGSEKTNVDIEKVMITDEEEIRTTNPVATEQYGSVSTNLQR
GNAAARGDQQSLAARQAATADVNTQGVLPGMVWQDRDVYLQGPIWAKIPHTDGHFHP
SPLMGGFGLKHPPPQILIKNTPVPADPPTTFNQSKLNSFITQYSTGQVSVEIEWELQKENS
KRWNPEIQYTSNYYKSTSVDFAVNTEGVYSEPRPIGTRYLTRNL

The amino acid sequence of the AAVDJK8 capsid (SEQ ID NO: 17) is as follows. The substituted region, NAAAPRGDLRPAAR (SEQ ID NO: 13), is underlined.

MAADGYLPDWLEDTLSEGIRQWWKLKPGPPPPKPAERHKDDSRGLVLPGYKYLGPFNG
LDKGEPVNEADAAALEHDKAYDRQLDSGDNPYLKYNHADAEFQERLKEDTSFGGNLG
RAVFQAKKRLLEPLGLVEEAAKTAPGKKRPVEHSPVEPDSSSGTGKAGQQPARKRLNFG
QTGDADSVPDPQPIGEPPAAPSGVGSLTMAAGGGAPMADNNEGADGVGNSSGNWHCD
STWMGDRVITTSTRTWALPTYNNHLYKQISNSTSGGSSNDNAYFGYSTPWGYFDFNRFH
CHFSPRDWQRLINNNWGFRPKRLSFKLFNIQVKEVTQNEGTKTIANNLTSTIQVFTDSEY
QLPYVLGSAHQGCLPPFPADVFMIPQYGYLTLNNGSQAVGRSSFYCLEYFPSQMLRTGN
NFQFTYTFEDVPFHSSYAHSQSLDRLMNPLIDQYLYYLSRTQTTGGTTNTQTLGFSQGGP
NTMANQAKNWLPGPCYRQQRVSKTSADNNNSEYSWTGATKYHLNGRDSLVNPGPAMA
SHKDDEEKFFPQSGVLIFGKQGSEKTNVDIEKVMITDEEEIRTTNPVATEQYGSVSTNLQR
GNAAAPRGDLRPAARQAATADVNTQGVLPGMVWQDRDVYLQGPIWAKIPHTDGHFHP
SPLMGGFGLKHPPPQILIKNTPVPADPPTTFNQSKLNSFITQYSTGQVSVEIEWELQKENS
KRWNPEIQYTSNYYKSTSVDFAVNTEGVYSEPRPIGTRYLTRNL

An example of the nucleotide sequence encoding the AAVDJK2 capsid (SEQ ID NO: 18) is as follows. The modified region is underlined.

ATGGCTGCCGATGGTTATCTTCCAGATTGGCTCGAGGACACTCTCTCTGAAGGAATAA
GACAGTGGTGGAAGCTCAAACCTGGCCCACCACCACCAAAGCCCGCAGAGCGGCAT
AAGGACGACAGCAGGGGTCTTGTGCTTCCTGGGTACAAGTACCTCGGACCCTTCAAC
GGACTCGACAAGGGAGAGCCGGTCAACGAGGCAGACGCCGCGGCCCTCGAGCACG
ACAAAGCCTACGACCGGCAGCTCGACAGCGGAGACAACCCGTACCTCAAGTACAAC
CACGCCGACGCCGAGTTCCAGGAGCGGCTCAAAGAAGATACGTCTTTTGGGGGCAA
CCTCGGGCGAGCAGTCTTCCAGGCCAAAAAGAGGCTTCTTGAACCTCTTGGTCTGGT
TGAGGAAGCGGCTAAGACGGCTCCTGGAAAGAAGAGGCCTGTAGAGCACTCTCCTG
TGGAGCCAGACTCCTCCTCGGGAACCGGAAAGGCGGGCCAGCAGCCTGCAAGAAAA
AGATTGAATTTTGGTCAGACTGGAGACGCAGACTCAGTCCCAGACCCTCAACCAATC
GGAGAACCTCCCGCAGCCCCCTCAGGTGTGGGATCTCTTACAATGGCTGCAGGCGGT
GGCGCACCAATGGCAGACAATAACGAGGGCGCCGACGGAGTGGGTAATTCCTCGGG
AAATTGGCATTGCGATTCCACATGGATGGGCGACAGAGTCATCACCACCAGCACCCG
AACCTGGGCCCTGCCCACCTACAACAACCACCTCTACAAGCAAATCTCCAACAGCAC
ATCTGGAGGATCTTCAAATGACAACGCCTACTTCGGCTACAGCACCCCCTGGGGGTAT
TTTGACTTTAACAGATTCCACTGCCACTTTTCACCACGTGACTGGCAGCGACTCATCA
ACAACAACTGGGGATTCCGGCCCAAGAGACTCAGCTTCAAGCTCTTCAACATCCAGG
TCAAGGAGGTCACGCAGAATGAAGGCACCAAGACCATCGCCAATAACCTCACCAGC
ACCATCCAGGTGTTTACGGACTCGGAGTACCAGCTGCCGTACGTTCTCGGCTCTGCCC
ACCAGGGCTGCCTGCCTCCGTTCCCGGCGGACGTGTTCATGATTCCCCAGTACGGCTA
CCTAACACTCAACAACGGTAGTCAGGCCGTGGGACGCTCCTCCTTCTACTGCCTGGA
ATACTTTCCTTCGCAGATGCTGAGAACCGGCAACAACTTCCAGTTTACTTACACCTTC
GAGGACGTGCCTTTCCACAGCAGCTACGCCCACAGCCAGAGCTTGGACCGGCTGATG
AATCCTCTGATTGACCAGTACCTGTACTACTTGTCTCGGACTCAAACAACAGGAGGCA
CGACAAATACGCAGACTCTGGGCTTCAGCCAAGGTGGGCCTAATACAATGGCCAATC
AGGCAAAGAACTGGCTGCCAGGACCCTGTTACCGCCAGCAGCGAGTATCAAAGACA
TCTGCGGATAACAACAACAGTGAATACTCGTGGACTGGAGCTACCAAGTACCACCTC
AATGGCAGAGACTCTCTGGTGAATCCGGGCCCGGCCATGGCAAGCCACAAGGACGAT

GAAGAAAAGTTTTTTCCTCAGAGCGGGGTTCTCATCTTTGGGAAGCAAGGCTCAGAG
AAAACAAATGTGGACATTGAAAAGGTCATGATTACAGACGAAGAGGAAATCAGGAC
AACCAATCCCGTGGCTACGGAGCAGTATGGTTCTGTATCTACCAACCTCCAGAGAGGC
AACGCAGCAGCACCACGTGGTGATCTGGCACCAGCAGCAAGACAAGCAGCTACCGC
AGATGTCAACACACAAGGCGTTCTTCCAGGCATGGTCTGGCAGGACAGAGATGTGTA
CCTTCAGGGGCCCATCTGGGCAAAGATTCCACACACGGACGGACATTTTCACCCCTC
TCCCCTCATGGGTGGATTCGGACTTAAACACCCTCCGCCTCAGATCCTGATCAAGAAC
ACGCCTGTACCTGCGGATCCTCCGACCACCTTCAACCAGTCAAAGCTGAACTCTTTC
ATCACCCAGTATTCTACTGGCCAAGTCAGCGTGGAGATCGAGTGGGAGCTGCAGAAG
GAAAACAGCAAGCGCTGGAACCCCGAGATCCAGTACACCTCCAACTACTACAAATCT
ACAAGTGTGGACTTTGCTGTTAATACAGAAGGCGTGTACTCTGAACCCCGCCCCATTG
GCACCCGTTACCTCACCCGTAATCTGTAA

An example of the nucleotide sequence encoding the AAVDJK1 capsid (SEQ ID NO: 19) is as follows. The modified region is underlined.

ATGGCTGCCGATGGTTATCTTCCAGATTGGCTCGAGGACACTCTCTCTGAAGGAATAA
GACAGTGGTGGAAGCTCAAACCTGGCCCACCACCACCAAAGCCCGCAGAGCGGCAT
AAGGACGACAGCAGGGGTCTTGTGCTTCCTGGGTACAAGTACCTCGGACCCTTCAAC
GGACTCGACAAGGGAGAGCCGGTCAACGAGGCAGACGCCGCGGCCCTCGAGCACG
ACAAAGCCTACGACCGGCAGCTCGACAGCGGAGACAACCCGTACCTCAAGTACAAC
CACGCCGACGCCGAGTTCCAGGAGCGGCTCAAAGAAGATACGTCTTTTGGGGGCAA
CCTCGGGCGAGCAGTCTTCCAGGCCAAAAAGAGGCTTCTTGAACCTCTTGGTCTGGT
TGAGGAAGCGGCTAAGACGGCTCCTGGAAAGAAGAGGCCTGTAGAGCACTCTCCTG
TGGAGCCAGACTCCTCCTCGGGAACCGGAAAGGCGGGCCAGCAGCCTGCAAGAAAA
AGATTGAATTTTGGTCAGACTGGAGACGCAGACTCAGTCCCAGACCCTCAACCAATC
GGAGAACCTCCCGCAGCCCCCTCAGGTGTGGGATCTCTTACAATGGCTGCAGGCGGT
GGCGCACCAATGGCAGACAATAACGAGGGCGCCGACGGAGTGGGTAATTCCTCGGG
AAATTGGCATTGCGATTCCACATGGATGGGCGACAGAGTCATCACCACCAGCACCCG
AACCTGGGCCCTGCCCACCTACAACAACCACCTCTACAAGCAAATCTCCAACAGCAC
ATCTGGAGGATCTTCAAATGACAACGCCTACTTCGGCTACAGCACCCCCTGGGGGTAT

TTTGACTTTAACAGATTCCACTGCCACTTTTCACCACGTGACTGGCAGCGACTCATCA
ACAACAACTGGGGATTCCGGCCCAAGAGACTCAGCTTCAAGCTCTTCAACATCCAGG
TCAAGGAGGTCACGCAGAATGAAGGCACCAAGACCATCGCCAATAACCTCACCAGC
ACCATCCAGGTGTTTACGGACTCGGAGTACCAGCTGCCGTACGTTCTCGGCTCTGCCC
ACCAGGGCTGCCTGCCTCCGTTCCCGGCGGACGTGTTCATGATTCCCCAGTACGGCTA
CCTAACACTCAACAACGGTAGTCAGGCCGTGGGACGCTCCTCCTTCTACTGCCTGGA
ATACTTTCCTTCGCAGATGCTGAGAACCGGCAACAACTTCCAGTTTACTTACACCTTC
GAGGACGTGCCTTTCCACAGCAGCTACGCCCACAGCCAGAGCTTGGACCGGCTGATG
AATCCTCTGATTGACCAGTACCTGTACTACTTGTCTCGGACTCAAACAACAGGAGGCA
CGACAAATACGCAGACTCTGGGCTTCAGCCAAGGTGGGCCTAATACAATGGCCAATC
AGGCAAAGAACTGGCTGCCAGGACCCTGTTACCGCCAGCAGCGAGTATCAAAGACA
TCTGCGGATAACAACAACAGTGAATACTCGTGGACTGGAGCTACCAAGTACCACCTC
AATGGCAGAGACTCTCTGGTGAATCCGGGCCCGGCCATGGCAAGCCACAAGGACGAT
GAAGAAAAGTTTTTTCCTCAGAGCGGGGTTCTCATCTTTGGGAAGCAAGGCTCAGAG
AAAACAAATGTGGACATTGAAAAGGTCATGATTACAGACGAAGAGGAAATCAGGAC
AACCAATCCCGTGGCTACGGAGCAGTATGGTTCTGTATCTACCAACCTCCAGAGAGGC
AACGCAGCAGCACGTGGTGATACTGCAACTCTGGCAGCAAGACAAGCAGCTACCGC
AGATGTCAACACACAAGGCGTTCTTCCAGGCATGGTCTGGCAGGACAGAGATGTGTA
CCTTCAGGGGCCCATCTGGGCAAAGATTCCACACACGGACGGACATTTTCACCCCTC
TCCCCTCATGGGTGGATTCGGACTTAAACACCCTCCGCCTCAGATCCTGATCAAGAAC
ACGCCTGTACCTGCGGATCCTCCGACCACCTTCAACCAGTCAAAGCTGAACTCTTTC
ATCACCCAGTATTCTACTGGCCAAGTCAGCGTGGAGATCGAGTGGGAGCTGCAGAAG
GAAAACAGCAAGCGCTGGAACCCCGAGATCCAGTACACCTCCAACTACTACAAATCT
ACAAGTGTGGACTTTGCTGTTAATACAGAAGGCGTGTACTCTGAACCCCGCCCCATTG
GCACCCGTTACCTCACCCGTAATCTGTAA

An example of the nucleotide sequence encoding the AAVDJK3 capsid (SEQ ID NO: 20) is as follows. The modified region is underlined.

ATGGCTGCCGATGGTTATCTTCCAGATTGGCTCGAGGACACTCTCTCTGAAGGAATAA
GACAGTGGTGGAAGCTCAAACCTGGCCCACCACCACCAAAGCCCGCAGAGCGGCAT

AAGGACGACAGCAGGGGTCTTGTGCTTCCTGGGTACAAGTACCTCGGACCCTTCAAC
GGACTCGACAAGGGAGAGCCGGTCAACGAGGCAGACGCCGCGGCCCTCGAGCACG
ACAAAGCCTACGACCGGCAGCTCGACAGCGGAGACAACCCGTACCTCAAGTACAAC
CACGCCGACGCCGAGTTCCAGGAGCGGCTCAAAGAAGATACGTCTTTTGGGGGCAA
CCTCGGGCGAGCAGTCTTCCAGGCCAAAAAGAGGCTTCTTGAACCTCTTGGTCTGGT
TGAGGAAGCGGCTAAGACGGCTCCTGGAAAGAAGAGGCCTGTAGAGCACTCTCCTG
TGGAGCCAGACTCCTCCTCGGGAACCGGAAAGGCGGGCCAGCAGCCTGCAAGAAAA
AGATTGAATTTTGGTCAGACTGGAGACGCAGACTCAGTCCCAGACCCTCAACCAATC
GGAGAACCTCCCGCAGCCCCCTCAGGTGTGGGATCTCTTACAATGGCTGCAGGCGGT
GGCGCACCAATGGCAGACAATAACGAGGGCGCCGACGGAGTGGGTAATTCCTCGGG
AAATTGGCATTGCGATTCCACATGGATGGGCGACAGAGTCATCACCACCAGCACCCG
AACCTGGGCCCTGCCCACCTACAACAACCACCTCTACAAGCAAATCTCCAACAGCAC
ATCTGGAGGATCTTCAAATGACAACGCCTACTTCGGCTACAGCACCCCCTGGGGGTAT
TTTGACTTTAACAGATTCCACTGCCACTTTTCACCACGTGACTGGCAGCGACTCATCA
ACAACAACTGGGGATTCCGGCCCAAGAGACTCAGCTTCAAGCTCTTCAACATCCAGG
TCAAGGAGGTCACGCAGAATGAAGGCACCAAGACCATCGCCAATAACCTCACCAGC
ACCATCCAGGTGTTTACGGACTCGGAGTACCAGCTGCCGTACGTTCTCGGCTCTGCCC
ACCAGGGCTGCCTGCCTCCGTTCCCGGCGGACGTGTTCATGATTCCCCAGTACGGCTA
CCTAACACTCAACAACGGTAGTCAGGCCGTGGGACGCTCCTCCTTCTACTGCCTGGA
ATACTTTCCTTCGCAGATGCTGAGAACCGGCAACAACTTCCAGTTTACTTACACCTTC
GAGGACGTGCCTTTCCACAGCAGCTACGCCCACAGCCAGAGCTTGGACCGGCTGATG
AATCCTCTGATTGACCAGTACCTGTACTACTTGTCTCGGACTCAAACAACAGGAGGCA
CGACAAATACGCAGACTCTGGGCTTCAGCCAAGGTGGGCCTAATACAATGGCCAATC
AGGCAAAGAACTGGCTGCCAGGACCCTGTTACCGCCAGCAGCGAGTATCAAAGACA
TCTGCGGATAACAACAACAGTGAATACTCGTGGACTGGAGCTACCAAGTACCACCTC
AATGGCAGAGACTCTCTGGTGAATCCGGGCCCGGCCATGGCAAGCCACAAGGACGAT
GAAGAAAAGTTTTTTCCTCAGAGCGGGGTTCTCATCTTTGGGAAGCAAGGCTCAGAG
AAAACAAATGTGGACATTGAAAAGGTCATGATTACAGACGAAGAGGAAATCAGGAC
AACCAATCCCGTGGCTACGGAGCAGTATGGTTCTGTATCTACCAACCTCCAGAGAGGC
AACGCAGCAGCACGTGGTGATCAGCAGTCTCTGGCAGCAAGACAAGCAGCTACCGC

AGATGTCAACACACAAGGCGTTCTTCCAGGCATGGTCTGGCAGGACAGAGATGTGTA
CCTTCAGGGGCCCATCTGGGCAAAGATTCCACACACGGACGGACATTTTCACCCCTC
TCCCCTCATGGGTGGATTCGGACTTAAACACCCTCCGCCTCAGATCCTGATCAAGAAC
ACGCCTGTACCTGCGGATCCTCCGACCACCTTCAACCAGTCAAAGCTGAACTCTTTC
ATCACCCAGTATTCTACTGGCCAAGTCAGCGTGGAGATCGAGTGGGAGCTGCAGAAG
GAAAACAGCAAGCGCTGGAACCCCGAGATCCAGTACACCTCCAACTACTACAAATCT
ACAAGTGTGGACTTTGCTGTTAATACAGAAGGCGTGTACTCTGAACCCCGCCCCATTG
GCACCCGTTACCTCACCCGTAATCTGTAA

An example of the nucleotide sequence encoding the AAVDJK8 capsid (SEQ ID NO: 21) is as follows. The modified region is underlined.

ATGGCTGCCGATGGTTATCTTCCAGATTGGCTCGAGGACACTCTCTCTGAAGGAATAA
GACAGTGGTGGAAGCTCAAACCTGGCCCACCACCAAAGCCCGCAGAGCGGCAT
AAGGACGACAGCAGGGGTCTTGTGCTTCCTGGGTACAAGTACCTCGGACCCTTCAAC
GGACTCGACAAGGGAGAGCCGGTCAACGAGGCAGACGCCGCGGCCCTCGAGCACG
ACAAAGCCTACGACCGGCAGCTCGACAGCGGAGACAACCCGTACCTCAAGTACAAC
CACGCCGACGCCGAGTTCCAGGAGCGGCTCAAAGAAGATACGTCTTTTGGGGGCAA
CCTCGGGCGAGCAGTCTTCCAGGCCAAAAAGAGGCTTCTTGAACCTCTTGGTCTGGT
TGAGGAAGCGGCTAAGACGGCTCCTGGAAAGAAGAGGCCTGTAGAGCACTCTCCTG
TGGAGCCAGACTCCTCCTCGGGAACCGGAAAGGCGGGCCAGCAGCCTGCAAGAAAA
AGATTGAATTTTGGTCAGACTGGAGACGCAGACTCAGTCCCAGACCCTCAACCAATC
GGAGAACCTCCCGCAGCCCCCTCAGGTGTGGGATCTCTTACAATGGCTGCAGGCGGT
GGCGCACCAATGGCAGACAATAACGAGGGCGCCGACGGAGTGGGTAATTCCTCGGG
AAATTGGCATTGCGATTCCACATGGATGGGCGACAGAGTCATCACCACCAGCACCCG
AACCTGGGCCCTGCCCACCTACAACAACCACCTCTACAAGCAAATCTCCAACAGCAC
ATCTGGAGGATCTTCAAATGACAACGCCTACTTCGGCTACAGCACCCCCTGGGGGTAT
TTTGACTTTAACAGATTCCACTGCCACTTTTCACCACGTGACTGGCAGCGACTCATCA
ACAACAACTGGGGATTCCGGCCCAAGAGACTCAGCTTCAAGCTCTTCAACATCCAGG
TCAAGGAGGTCACGCAGAATGAAGGCACCAAGACCATCGCCAATAACCTCACCAGC
ACCATCCAGGTGTTTACGGACTCGGAGTACCAGCTGCCGTACGTTCTCGGCTCTGCCC

ACCAGGGCTGCCTGCCTCCGTTCCCGGCGGACGTGTTCATGATTCCCCAGTACGGCTA
CCTAACACTCAACAACGGTAGTCAGGCCGTGGGACGCTCCTCCTTCTACTGCCTGGA
ATACTTTCCTTCGCAGATGCTGAGAACCGGCAACAACTTCCAGTTTACTTACACCTTC
GAGGACGTGCCTTTCCACAGCAGCTACGCCCACAGCCAGAGCTTGGACCGGCTGATG
AATCCTCTGATTGACCAGTACCTGTACTACTTGTCTCGGACTCAAACAACAGGAGGCA
CGACAAATACGCAGACTCTGGGCTTCAGCCAAGGTGGGCCTAATACAATGGCCAATC
AGGCAAAGAACTGGCTGCCAGGACCCTGTTACCGCCAGCAGCGAGTATCAAAGACA
TCTGCGGATAACAACAACAGTGAATACTCGTGGACTGGAGCTACCAAGTACCACCTC
AATGGCAGAGACTCTCTGGTGAATCCGGGCCCGGCCATGGCAAGCCACAAGGACGAT
GAAGAAAAGTTTTTTCCTCAGAGCGGGGTTCTCATCTTTGGGAAGCAAGGCTCAGAG
AAAACAAATGTGGACATTGAAAAGGTCATGATTACAGACGAAGAGGAAATCAGGAC
AACCAATCCCGTGGCTACGGAGCAGTATGGTTCTGTATCTACCAACCTCCAGAGAGGC
AACGCAGCAGCACCACGTGGTGATCTGCGTCCAGCAGCAAGACAAGCAGCTACCGC
AGATGTCAACACACAAGGCGTTCTTCCAGGCATGGTCTGGCAGGACAGAGATGTGTA
CCTTCAGGGGCCCATCTGGGCAAAGATTCCACACACGGACGGACATTTTCACCCCTC
TCCCCTCATGGGTGGATTCGGACTTAAACACCCTCCGCCTCAGATCCTGATCAAGAAC
ACGCCTGTACCTGCGGATCCTCCGACCACCTTCAACCAGTCAAAGCTGAACTCTTTC
ATCACCCAGTATTCTACTGGCCAAGTCAGCGTGGAGATCGAGTGGGAGCTGCAGAAG
GAAAACAGCAAGCGCTGGAACCCCGAGATCCAGTACACCTCCAACTACTACAAATCT
ACAAGTGTGGACTTTGCTGTTAATACAGAAGGCGTGTACTCTGAACCCCGCCCCATTG
GCACCCGTTACCTCACCCGTAATCTGTAA

An example of the Rep-Cap plasmid nucleotide sequence encoding the AAVDJK2 capsid (SEQ ID NO: 22) is as follows. The underlined region corresponds to the AAVDJK2 capsid.

CCGCCATGCCGGGGTTTTACGAGATTGTGATTAAGGTCCCCAGCGACCTTGACGAGC
ATCTGCCCGGCATTTCTGACAGCTTTGTGAACTGGGTGGCCGAGAAGGAATGGGAGT
TGCCGCCAGATTCTGACATGGATCTGAATCTGATTGAGCAGGCACCCCTGACCGTGG
CCGAGAAGCTGCAGCGCGACTTTCTGACGGAATGGCGCCGTGTGAGTAAGGCCCCG
GAGGCCCTTTTCTTTGTGCAATTTGAGAAGGGAGAGAGCTACTTCCACATGCACGTG
CTCGTGGAAACCACCGGGGTGAAATCCATGGTTTTGGGACGTTTCCTGAGTCAGATT

CGCGAAAAACTGATTCAGAGAATTTACCGCGGGATCGAGCCGACTTTGCCAAACTG
GTTCGCGGTCACAAAGACCAGAAATGGCGCCGGAGGCGGGAACAAGGTGGTGGAT
GAGTGCTACATCCCCAATTACTTGCTCCCCAAAACCCAGCCTGAGCTCCAGTGGGCG
TGGACTAATATGGAACAGTATTTAAGCGCCTGTTTGAATCTCACGGAGCGTAAACGG
TTGGTGGCGCAGCATCTGACGCACGTGTCGCAGACGCAGGAGCAGAACAAAGAGAA
TCAGAATCCCAATTCTGATGCGCCGGTGATCAGATCAAAAACTTCAGCCAGGTACAT
GGAGCTGGTCGGGTGGCTCGTGGACAAGGGGATTACCTCGGAGAAGCAGTGGATCC
AGGAGGACCAGGCCTCATACATCTCCTTCAATGCGGCCTCCAACTCGCGGTCCCAAA
TCAAGGCTGCCTTGGACAATGCGGGAAAGATTATGAGCCTGACTAAAACCGCCCCC
GACTACCTGGTGGGCCAGCAGCCCGTGGAGGACATTTCCAGCAATCGGATTTATAA
AATTTTGGAACTAAACGGGTACGATCCCCAATATGCGGCTTCCGTCTTTCTGGGATG
GGCCACGAAAAAGTTCGGCAAGAGGAACACCATCTGGCTGTTTGGGCCTGCAACTA
CCGGGAAGACCAACATCGCGGAGGCCATAGCCCACACTGTGCCCTTCTACGGGTGC
GTAAACTGGACCAATGAGAACTTTCCCTTCAACGACTGTGTCGACAAGATGGTGATC
TGGTGGGAGGAGGGGAAGATGACCGCCAAGGTCGTGGAGTCGGCCAAAGCCATTCT
CGGAGGAAGCAAGGTGCGCGTGGACCAGAAATGCAAGTCCTCGGCCCAGATAGACC
CGACTCCCGTGATCGTCACCTCCAACACCAACATGTGCGCCGTGATTGACGGGAACT
CAACGACCTTCGAACACCAGCAGCCGTTGCAAGACCGGATGTTCAAATTTGAACTC
ACCCGCCGTCTGGATCATGACTTTGGGAAGGTCACCAAGCAGGAAGTCAAAGACTT
TTTCCGGTGGGCAAAGGATCACGTGGTTGAGGTGGAGCATGAATTCTACGTCAAAA
AGGGTGGAGCCAAGAAAAGACCCGCCCCCAGTGACGCAGATATAAGTGAGCCCAA
ACGGGTGCGCGAGTCAGTTGCGCAGCCATCGACGTCAGACGCGGAAGCTTCGATCA
ACTACGCAGACAGGTACCAAAACAAATGTTCTCGTCACGTGGGCATGAATCTGATG
CTGTTTCCCTGCAGACAATGCGAGAGAATGAATCAGAATTCAAATATCTGCTTCACT
CACGGACAGAAAGACTGTTTAGAGTGCTTTCCCGTGTCAGAATCTCAACCCGTTTCT
GTCGTCAAAAAGGCGTATCAGAAACTGTGCTACATTCATCATATCATGGGAAAGGT
GCCAGACGCTTGCACTGCCTGCGATCTGGTCAATGTGGATTTGGATGACTGCATCTT
TGAACAATAAATGATTTAAATCAGGTATGGCTGCCGATGGTTATCTTCCAGATTGGC
TCGAGGACACTCTCTCTGAAGGAATAAGACAGTGGTGGAAGCTCAAACCTGGCCCA
CCACCACCAAAGCCCGCAGAGCGGCATAAGGACGACAGCAGGGGTCTTGTGCTTCC

TGGGTACAAGTACCTCGGACCCTTCAACGGACTCGACAAGGGAGAGCCGGTCAACG
AGGCAGACGCCGCGGCCCTCGAGCACGACAAAGCCTACGACCGGCAGCTCGACAGC
GGAGACAACCCGTACCTCAAGTACAACCACGCCGACGCCGAGTTCCAGGAGCGGCT
CAAAGAAGATACGTCTTTTGGGGGCAACCTCGGGCGAGCAGTCTTCCAGGCCAAAA
AGAGGCTTCTTGAACCTCTTGGTCTGGTTGAGGAAGCGGCTAAGACGGCTCCTGGAA
AGAAGAGGCCTGTAGAGCACTCTCCTGTGGAGCCAGACTCCTCCTCGGGAACCGGA
AAGGCGGGCCAGCAGCCTGCAAGAAAAAGATTGAATTTTGGTCAGACTGGAGACGC
AGACTCAGTCCCAGACCCTCAACCAATCGGAGAACCTCCCGCAGCCCCCTCAGGTGT
GGGATCTCTTACAATGGCTGCAGGCGGTGGCGCACCAATGGCAGACAATAACGAGG
GCGCCGACGGAGTGGGTAATTCCTCGGGAAATTGGCATTGCGATTCCACATGGATG
GGCGACAGAGTCATCACCACCAGCACCCGAACCTGGGCCCTGCCCACCTACAACAA
CCACCTCTACAAGCAAATCTCCAACAGCACATCTGGAGGATCTTCAAATGACAACG
CCTACTTCGGCTACAGCACCCCCTGGGGGTATTTTGACTTTAACAGATTCCACTGCC
ACTTTTCACCACGTGACTGGCAGCGACTCATCAACAACAACTGGGGATTCCGGCCCA
AGAGACTCAGCTTCAAGCTCTTCAACATCCAGGTCAAGGAGGTCACGCAGAATGAA
GGCACCAAGACCATCGCCAATAACCTCACCAGCACCATCCAGGTGTTTACGGACTC
GGAGTACCAGCTGCCGTACGTTCTCGGCTCTGCCCACCAGGGCTGCCTGCCTCCGTT
CCCGGCGGACGTGTTCATGATTCCCCAGTACGGCTACCTAACACTCAACAACGGTAG
TCAGGCCGTGGGACGCTCCTCCTTCTACTGCCTGGAATACTTTCCTTCGCAGATGCTG
AGAACCGGCAACAACTTCCAGTTTACTTACACCTTCGAGGACGTGCCTTTCCACAGC
AGCTACGCCCACAGCCAGAGCTTGGACCGGCTGATGAATCCTCTGATTGACCAGTAC
CTGTACTACTTGTCTCGGACTCAAACAACAGGAGGCACGACAAATACGCAGACTCT
GGGCTTCAGCCAAGGTGGGCCTAATACAATGGCCAATCAGGCAAAGAACTGGCTGC
CAGGACCCTGTTACCGCCAGCAGCGAGTATCAAAGACATCTGCGGATAACAACAAC
AGTGAATACTCGTGGACTGGAGCTACCAAGTACCACCTCAATGGCAGAGACTCTCTG
GTGAATCCGGGCCCGGCCATGGCAAGCCACAAGGACGATGAAGAAAAGTTTTTTCC
TCAGAGCGGGGTTCTCATCTTTGGGAAGCAAGGCTCAGAGAAAACAAATGTGGACA
TTGAAAAGGTCATGATTACAGACGAAGAGGAAATCAGGACAACCAATCCCGTGGCT
ACGGAGCAGTATGGTTCTGTATCTACCAACCTCCAGAGAGGCAACGCAGCAGCACC
ACGTGGTGATCTGGCACCAGCAGCAAGACAAGCAGCTACCGCAGATGTCAACACAC

AAGGCGTTCTTCCAGGCATGGTCTGGCAGGACAGAGATGTGTACCTTCAGGGGCCC
ATCTGGGCAAAGATTCCACACACGGACGGACATTTTCACCCCTCTCCCCTCATGGGT
GGATTCGGACTTAAACACCCTCCGCCTCAGATCCTGATCAAGAACACGCCTGTACCT
GCGGATCCTCCGACCACCTTCAACCAGTCAAAGCTGAACTCTTTCATCACCCAGTAT
TCTACTGGCCAAGTCAGCGTGGAGATCGAGTGGGAGCTGCAGAAGGAAAACAGCAA
GCGCTGGAACCCCGAGATCCAGTACACCTCCAACTACTACAAATCTACAAGTGTGG
ACTTTGCTGTTAATACAGAAGGCGTGTACTCTGAACCCCGCCCCATTGGCACCCGTT
ACCTCACCCGTAATCTGTAATTGCCTGTTAATCAATAAACCGGTTGATTCGTTTCAGT
TGAACTTTGGTCTCTGCGAAGGGCGAATTCGTTTAAACCTGCAGGACTAGAGGTCCT
GTATTAGAGGTCACGTGAGTGTTTTGCGACATTTTGCGACACCATGTGGTCACGCTG
GGTATTTAAGCCCGAGTGAGCACGCAGGGTCTCCATTTTGAAGCGGGAGGTTTGAAC
GCGCAGCCGCCAAGCCGAATTCTGCAGATATCCATCACACTGGCGGCCGCTCGACT
AGAGCGGCCGCCACCGCGGTGGAGCTCCAGCTTTTGTTCCCTTTAGTGAGGGTTAAT
TGCGCGCTTGGCGTAATCATGGTCATAGCTGTTTCCTGTGTGAAATTGTTATCCGCTC
ACAATTCCACACAACATACGAGCCGGAAGCATAAAGTGTAAAGCCTGGGGTGCCTA
ATGAGTGAGCTAACTCACATTAATTGCGTTGCGCTCACTGCCCGCTTTCCAGTCGGG
AAACCTGTCGTGCCAGCTGCATTAATGAATCGGCCAACGCGCGGGGAGAGGCGGTT
TGCGTATTGGGCGCTCTTCCGCTTCCTCGCTCACTGACTCGCTGCGCTCGGTCGTTCG
GCTGCGGCGAGCGGTATCAGCTCACTCAAAGGCGGTAATACGGTTATCCACAGAAT
CAGGGGATAACGCAGGAAAGAACATGTGAGCAAAAGGCCAGCAAAAGGCCAGGAA
CCGTAAAAAGGCCGCGTTGCTGGCGTTTTTCCATAGGCTCCGCCCCCCTGACGAGCA
TCACAAAAATCGACGCTCAAGTCAGAGGTGGCGAAACCCGACAGGACTATAAAGAT
ACCAGGCGTTTCCCCCTGGAAGCTCCCTCGTGCGCTCTCCTGTTCCGACCCTGCCGCT
TACCGGATACCTGTCCGCCTTTCTCCCTTCGGGAAGCGTGGCGCTTTCTCATAGCTCA
CGCTGTAGGTATCTCAGTTCGGTGTAGGTCGTTCGCTCCAAGCTGGGCTGTGTGCAC
GAACCCCCCGTTCAGCCCGACCGCTGCGCCTTATCCGGTAACTATCGTCTTGAGTCC
AACCCGGTAAGACACGACTTATCGCCACTGGCAGCAGCCACTGGTAACAGGATTAG
CAGAGCGAGGTATGTAGGCGGTGCTACAGAGTTCTTGAAGTGGTGGCCTAACTACG
GCTACACTAGAAGAACAGTATTTGGTATCTGCGCTCTGCTGAAGCCAGTTACCTTCG
GAAAAAGAGTTGGTAGCTCTTGATCCGGCAAACAAACCACCGCTGGTAGCGGTGGT

TTTTTTGTTTGCAAGCAGCAGATTACGCGCAGAAAAAAAGGATCTCAAGAAGATCCT
TTGATCTTTTCTACGGGGTCTGACGCTCAGTGGAACGAAAACTCACGTTAAGGGATT
TTGGTCATGAGATTATCAAAAAGGATCTTCACCTAGATCCTTTTAAATTAAAAATGA
AGTTTTAAATCAATCTAAAGTATATATGAGTAAACTTGGTCTGACAGTTACCAATGC
TTAATCAGTGAGGCACCTATCTCAGCGATCTGTCTATTTCGTTCATCCATAGTTGCCT
GACTCCCCGTCGTGTAGATAACTACGATACGGGAGGGCTTACCATCTGGCCCCAGTG
CTGCAATGATACCGCGAGACCCACGCTCACCGGCTCCAGATTTATCAGCAATAAACC
AGCCAGCCGGAAGGGCCGAGCGCAGAAGTGGTCCTGCAACTTTATCCGCCTCCATC
CAGTCTATTAATTGTTGCCGGGAAGCTAGAGTAAGTAGTTCGCCAGTTAATAGTTTG
CGCAACGTTGTTGCCATTGCTACAGGCATCGTGGTGTCACGCTCGTCGTTTGGTATG
GCTTCATTCAGCTCCGGTTCCCAACGATCAAGGCGAGTTACATGATCCCCCATGTTG
TGCAAAAAAGCGGTTAGCTCCTTCGGTCCTCCGATCGTTGTCAGAAGTAAGTTGGCC
GCAGTGTTATCACTCATGGTTATGGCAGCACTGCATAATTCTCTTACTGTCATGCCAT
CCGTAAGATGCTTTTCTGTGACTGGTGAGTACTCAACCAAGTCATTCTGAGAATAGT
GTATGCGGCGACCGAGTTGCTCTTGCCCGGCGTCAATACGGGATAATACCGCGCCAC
ATAGCAGAACTTTAAAAGTGCTCATCATTGGAAAACGTTCTTCGGGGCGAAAACTCT
CAAGGATCTTACCGCTGTTGAGATCCAGTTCGATGTAACCCACTCGTGCACCCAACT
GATCTTCAGCATCTTTTACTTTCACCAGCGTTTCTGGGTGAGCAAAAACAGGAAGGC
AAAATGCCGCAAAAAAGGGAATAAGGGCGACACGGAAATGTTGAATACTCATACTC
TTCCTTTTTCAATATTATTGAAGCATTTATCAGGGTTATTGTCTCATGAGCGGATACA
TATTTGAATGTATTTAGAAAAATAAACAAATAGGGGTTCCGCGCACATTTCCCCGAA
AAGTGCCACCTAAATTGTAAGCGTTAATATTTTGTTAAAATTCGCGTTAAATTTTTGT
TAAATCAGCTCATTTTTTAACCAATAGGCCGAAATCGGCAAAATCCCTTATAAATCA
AAAGAATAGACCGAGATAGGGTTGAGTGTTGTTCCAGTTTGGAACAAGAGTCCACT
ATTAAAGAACGTGGACTCCAACGTCAAAGGGCGAAAAACCGTCTATCAGGGCGATG
GCCCACTACGTGAACCATCACCCTAATCAAGTTTTTTGGGGTCGAGGTGCCGTAAAG
CACTAAATCGGAACCCTAAAGGGAGCCCCCGATTTAGAGCTTGACGGGGAAAGCCG
GCGAACGTGGCGAGAAAGGAAGGGAAGAAAGCGAAAGGAGCGGGCGCTAGGGCGC
TGGCAAGTGTAGCGGTCACGCTGCGCGTAACCACCACACCCGCCGCGCTTAATGCG
CCGCTACAGGGCGCGTCCCATTCGCCATTCAGGCTGCGCAACTGTTGGGAAGGGCG

ATCGGTGCGGGCCTCTTCGCTATTACGCCAGCTGGCGAAAGGGGGATGTGCTGCAA
GGCGATTAAGTTGGGTAACGCCAGGGTTTTCCCAGTCACGACGTTGTAAAACGACG
GCCAGTGAGCGCGCGTAATACGACTCACTATAGGGCGAATTGGGTACCGGGCCCCC
CCTCGATCGAGGTCGACGGTATCGGGGGAGCTCGCAGGGTCTCCATTTTGAAGCGG
GAGGTTTGAACGCGCAG

### 6. Experimental Results 2

The present invention will be described in detail below based on examples and the like; however, the invention is not limited to these.

### [Example 2]

Figure 2 shows the results of a comparative study on the gene transduction efficiency of novel AAVDJ variants and known AAVs in mouse and human cultured keratinocytes. Capsid plasmids were constructed using the backbone plasmid of plasmid nucleotide sequence 4 (SEQ ID NO: 22), incorporating the capsid sequences of the novel AAVDJ variants AAVDJK1 (SEQ ID NO: 19; capsid nucleotide sequence 3), AAVDJK2 (SEQ ID NO: 18; capsid nucleotide sequence 2), AAVDJK3 (SEQ ID NO: 20; capsid nucleotide sequence 4), and AAVDJK8 (SEQ ID NO: 21; capsid nucleotide sequence 5). Additionally, capsid plasmids encoding known AAVs, including AAV2, AAV2K1, AAV2K2, AAV2K3, AAV2K8, AAV6, and AAVDJ, which are reported to exhibit superior gene transduction efficiency in keratinocytes, were also prepared (Sallach J, Di Pasquale G, Larcher F, Niehoff N, Rübsam M, Huber A, Chiorini J, Almarza D, Eming SA, Ulus H, Nishimura S, Hacker UT, Hallek M, Niessen CM, Büning H. Tropism-modified AAV vectors overcome barriers to successful cutaneous therapy. Mol Ther. 2014 May;22(5):929-39; Melo SP, Lisowski L, Bashkirova E, Zhen HH, Chu K, Keene DR, Marinkovich MP, Kay MA, Oro AE. Somatic correction of junctional epidermolysis bullosa by a highly recombinogenic AAV variant. Mol Ther. 2014 Apr;22(4):725-33; Bonafont J, Mencía A, Chacón-Solano E, Srifa W, Vaidyanathan S, Romano R, Garcia M, Hervás-Salcedo R, Ugalde L, Duarte B, Porteus MH, Del Rio M, Larcher F, Murillas R. Correction of recessive dystrophic epidermolysis bullosa by homology-directed repair-mediated genome editing. Mol Ther. 2021 Jun 2;29(6):2008-2018). AAVs were produced by transfection into 293AAV cells using the calcium phosphate method with the vector plasmid expressing GFPNLS under the control of a CAG promoter and the pAD5 plasmid as a helper plasmid. The AAVs were concentrated using ultracentrifugation according to standard methods, and vector solutions containing each AAV were obtained. Mouse keratinocytes were isolated and cultured under feeder conditions according to standard methods using primary cultured cells from the dorsal skin of 3-5-week-old C57BL/6J Jcl mice (see Non-Patent Document 2). Normal human epidermal keratinocytes (adult donor, pooled, Product code: C12006) (PromoCell, Heidelberg, Germany) were used under the same culture conditions as mouse keratinocytes. Both mouse and human keratinocytes were cultured in 24-well plates, and when they reached 50% confluence, the medium was replaced with medium containing AAV at 1.0×10⁹ GC/well. Four wells were treated under identical conditions for each AAV. Phase-contrast microscopy and fluorescence imaging were performed in four fields of view on days 2, 4, and 8 after AAV treatment. Using ImageJ software, the area of keratinocyte colonies on the images was calculated. The number of GFPNLS-positive cells within the colony area was counted from the images, and the number of GFPNLS-positive cells per unit area of the keratinocyte colony was determined. The results showed that the novel AAVDJ variants, AAVDJK1, AAVDJK2, AAVDJK3, and AAVDJK8, demonstrated higher gene transduction efficiency compared to known AAVs in both mouse and human keratinocytes, with AAVDJK2 exhibiting the highest efficiency among the tested variants.

### [Example 3]

Figure 3 shows the results of a comparative study on the gene transduction efficiency of known AAV2, AAV2K2, and AAVDJ, as well as the novel AAVDJK2, in mouse and human cultured mesenchymal cells. AAVs with each capsid expressing GFPNLS under the control of a CAG promoter were prepared using the same method as in Example 2. Mouse adipose-derived mesenchymal cells were obtained from the inguinal skin of 3-5-week-old C57BL/6J Jcl mice and cultured as primary cells according to standard methods (see Non-Patent Document 2). Normal human dermal fibroblasts (juvenile foreskin, Product Code: C12300) (PromoCell, Heidelberg, Germany), which are mesenchymal cells derived from the dermis, were used under the same culture conditions as the mouse adipose-derived mesenchymal cells. Both mouse and human mesenchymal cells were cultured in 24-well plates, and when they reached 50% confluence, the medium was replaced with medium containing AAV at 1.0×10⁹ GC/well. Four wells were treated under identical conditions for each AAV. Phase-contrast microscopy and fluorescence imaging were performed daily in four fields of view until day 8. The number of GFPNLS-positive cells per fluorescence image (area: 1.99 mm²) was counted. In mouse adipose-derived mesenchymal cells, the novel AAVDJK2 did not exhibit the high gene transduction efficiency observed with AAVDJ for mesenchymal cells. Similarly, in human fibroblasts, the novel AAVDJK2 did not exhibit the high gene transduction efficiency observed with AAV2 or AAVDJ for mesenchymal cells.

### [Reference Example 1]

Figure 4 shows the results of an injection test of fluorescent silica particles into the dorsal skin of mice. A solution was prepared by diluting fluorescent silica particles (sicastar^{®}-greenF, plain, 30 nm, 25 mg/ml, Filgen) with phosphate-buffered saline (PBS) at a 20-fold dilution. Using a 29G injection needle under a surgical microscope, 20 µl of the diluted solution was intradermally injected into the dorsal skin of mice at the shallowest depth possible. Tissues were collected at 0 hours, 1 hour, and 3 hours after injection, embedded in OCT compound, and frozen. Sections 10 µm thick were collected every 200 µm from the frozen tissue, washed with PBS, and stained with DAPI for nuclear staining. On tissue sections prepared immediately after injection, as well as 1 hour and 3 hours post-injection, most of the injected particles were distributed from the dermis to the subcutaneous tissue and were not observed in the epidermis. This indicates that, while intradermal injection is a conventional method for administering AAV targeting the epidermis, it is neither efficient nor specific.

### [Example 4]

Figure 5 shows the results of a comparative study on the gene transduction efficiency of AAVDJK2 and AAVDJ in the dorsal skin of mice. Using the concentrated AAV preparation method described in Example 2, an AAVDJK2 vector expressing GFPNLS under the control of a CAG promoter and an AAVDJ vector expressing mCherryNLS under the control of a CAG promoter were prepared. The solution was adjusted so that both AAVs were included at the same titer (10¹¹ GC (gene copies)/35 µl) and intradermally injected into the dorsal skin of mice. Two days after injection, the injection sites were observed using a fluorescence stereomicroscope, and tissues including the injection sites were collected. The tissues were fixed in 4% paraformaldehyde, embedded in OCT compound, and processed into frozen sections. Adjacent sections were stained with HE and with DAPI for nuclear staining, and the images were captured as image files using a fluorescence slide scanner (VS200, Olympus). Histological observations revealed that GFPNLS transduced by AAVDJK2 was strongly expressed in the epidermis and weakly expressed in the subcutaneous tissue, whereas mCherry transduced by AAVDJ was weakly expressed in the epidermis and strongly expressed in the subcutaneous tissue. It was concluded that AAVDJK2 exhibits higher gene transduction efficiency and specificity for epidermal tissues compared to AAVDJ.

### 7. Applications of Promoters with High Specificity for Keratinocytes and Epidermal Tissues

The K14SCP3 promoter, K16SCP3 promoter, and K16P5 short promoter, which exhibit high specificity for keratinocytes and epidermal tissues in the present invention, provide higher or comparable gene expression levels in keratinocytes and epidermal tissues compared to the widely known CAG promoter, which induces high gene expression regardless of tissue or cell type. In contrast, these promoters exhibit relatively low gene expression in non-keratinocyte or non-epidermal tissues and cells, such as mesenchymal cells near the epidermis. Therefore, they can be used as gene transduction vectors for gene therapy targeting cultured keratinocytes or for in vivo gene therapy targeting epidermal tissues. Additionally, they can be utilized as research and development tools for these gene therapy methods. Furthermore, they can serve as research tools for drug discovery and biological studies beyond the development of gene therapy methods using gene transduction. In gene therapy and its research and development targeting keratinocytes and epidermal tissues, it is often preferable to keep gene expression (non-specific gene expression) in non-keratinocyte or non-epidermal tissues low. Consequently, the promoters with high specificity for keratinocytes and epidermal tissues described in the present invention are expected to be effective in numerous situations.

The promoters in the present invention are not limited to the K14SCP3 promoter, K16SCP3 promoter, and K16P5 short promoter but may also include sequences with high homology to these nucleotide sequences. Such homology is generally 80% or higher, preferably 90% or higher, more preferably 95% or higher, and most preferably 99% or higher.

### 8. Method for Producing Gene Transduction Vectors with Promoters Exhibiting High Specificity for Keratinocytes and Epidermal Tissues

The K14SCP3 promoter, K16SCP3 promoter, and K16P5 short promoter, which exhibit high specificity for keratinocytes and epidermal tissues in the present invention, can be used as promoter sequences in AAV, lentiviral vectors, or naked plasmids to create gene transduction vectors that express genes with high specificity in keratinocytes and epidermal tissues. For example, in the case of an AAV expressing GFPNLS as a target gene under the control of the K14SCP3 promoter, the K14SCP3 promoter sequence (SEQ ID NO: 23; promoter nucleotide sequence 1) is incorporated into the vector plasmid (SEQ ID NO: 24; plasmid nucleotide sequence 5). AAVs capable of expressing the desired gene can be generated by standard AAV production methods, such as co-transfection of this plasmid, together with a Rep-Cap plasmid encoding the capsid and a helper plasmid, or by a baculovirus-based AAV production method. The same applies to the K16SCP3 promoter (SEQ ID NO: 25; promoter nucleotide sequence 2) and the K16P5 short promoter (SEQ ID NO: 26; promoter nucleotide sequence 3).

The K14SCP3 promoter sequence (SEQ ID NO: 23) is as follows.

CTCCGGAGCTTCTATTCCTGATCCCTGCATAAGAAGGAGACATGGTGGTGGTGG
TGGGTGGGGGTGGTGGGGCACAGAGGAAGCCGGTACTGGGCTCTGCACCCCATTCC
CGCTCCCAGATCCCTCTGGACACAGCATTTTTCTCCAGTGAGCACAGCCTCCCCTTG
CCCCACAGCCAACAGCAACATGCCTCCCAACAAAAGCATCTGTCCCTCAGCCAAAA
CCCCTGTTGCCTCTCTCTGGGGAAATTGTAGGGCTGGGCCAGGGTGGGGGGACCATT
CTCTGCAGGGAGATTAGGAGTGTCTGTCAGGGGCGGGTGGAGCGGGGTGGGGCCCT
GGCTTACTCACATCCTTGAGAGTCCTTTGCTGGCAGATTTGGGGAGCCCACAGCTCA
GATGTCTGTCTCAGCATTGTCTTCCAAGCTCCTAGGCCACAGTAGTGGGGGGCTCCC
TTCTCTGGCTTCTTCTTTGGTGACAGTCAAGGTGGGGTTGGGGGTGACAGAGGGTCC
TGCTTCTCTTCTAGGAGCAGTTGATCCCAGGAAGAGCATTGGAGCCTCCAGCAGGGG
CTGTTGGGGCCTGTCTGAGGAGATAGGATGCGTCAGGCAGCCCCAGACACGATCAC
ATTCCTCTCAACATGCCTGCCGGGGTCTGTGGAGCCTAGGGGCTGATGGGAGGGTGG

GGTGGGGGCCGGAAGGGTTTAGGTCTATATAAGCAGAGCTCGTTTAGTGAACCGTC
AGTCCGCCTGGAGACCTCGAGCCGAGTGGTCGTGCCTCCATAGAA

An example of the nucleotide sequence of the pAAV-K14SCP3-GFPNLS plasmid (SEQ ID NO: 24) is as follows. The underlined region corresponds to the K14SCP3 promoter.

CTTCCGCTTCCTCGCTCACTGACTCGCTGCGCTCGGTCGTTCGGCTGCGGCGAGCGG
TATCAGCTCACTCAAAGGCGGTAATACGGTTATCCACAGAATCAGGGGATAACGCA
GGAAAGAACATGTGAGCAAAAGGCCAGCAAAAGGCCAGGAACCGTAAAAAGGCCG
CGTTGCTGGCGTTTTTCCATAGGCTCCGCCCCCCTGACGAGCATCACAAAAATCGAC
GCTCAAGTCAGAGGTGGCGAAACCCGACAGGACTATAAAGATACCAGGCGTTTCCC
CCTGGAAGCTCCCTCGTGCGCTCTCCTGTTCCGACCCTGCCGCTTACCGGATACCTGT
CCGCCTTTCTCCCTTCGGGAAGCGTGGCGCTTTCTCATAGCTCACGCTGTAGGTATCT
CAGTTCGGTGTAGGTCGTTCGCTCCAAGCTGGGCTGTGTGCACGAACCCCCCGTTCA
GCCCGACCGCTGCGCCTTATCCGGTAACTATCGTCTTGAGTCCAACCCGGTAAGACA
CGACTTATCGCCACTGGCAGCAGCCACTGGTAACAGGATTAGCAGAGCGAGGTATG
TAGGCGGTGCTACAGAGTTCTTGAAGTGGTGGCCTAACTACGGCTACACTAGAAGA
ACAGTATTTGGTATCTGCGCTCTGCTGAAGCCAGTTACCTTCGGAAAAAGAGTTGGT
AGCTCTTGATCCGGCAAACAAACCACCGCTGGTAGCGGTGGTTTTTTTGTTTGCAAG
CAGCAGATTACGCGCAGAAAAAAAGGATCTCAAGAAGATCCTTTGATCTTTTCTACG
GGGTCTGACGCTCAGTGGAACGAAAACTCACGTTAAGGGATTTTGGTCATGAGATTA
TCAAAAAGGATCTTCACCTAGATCCTTTTAAATTAAAAATGAAGTTTTAAATCAATC
TAAAGTATATATGAGTAAACTTGGTCTGACAGTTACCAATGCTTAATCAGTGAGGCA
CCTATCTCAGCGATCTGTCTATTTCGTTCATCCATAGTTGCCTGACTCCCCGTCGTGT
AGATAACTACGATACGGGAGGGCTTACCATCTGGCCCCAGTGCTGCAATGATACCG
CGAGACCCACGCTCACCGGCTCCAGATTTATCAGCAATAAACCAGCCAGCCGGAAG
GGCCGAGCGCAGAAGTGGTCCTGCAACTTTATCCGCCTCCATCCAGTCTATTAATTG
TTGCCGGGAAGCTAGAGTAAGTAGTTCGCCAGTTAATAGTTTGCGCAACGTTGTTGC
CATTGCTACAGGCATCGTGGTGTCACGCTCGTCGTTTGGTATGGCTTCATTCAGCTCC
GGTTCCCAACGATCAAGGCGAGTTACATGATCCCCCATGTTGTGCAAAAAAGCGGTT
AGCTCCTTCGGTCCTCCGATCGTTGTCAGAAGTAAGTTGGCCGCAGTGTTATCACTC

ATGGTTATGGCAGCACTGCATAATTCTCTTACTGTCATGCCATCCGTAAGATGCTTTT
CTGTGACTGGTGAGTACTCAACCAAGTCATTCTGAGAATAGTGTATGCGGCGACCGA
GTTGCTCTTGCCCGGCGTCAATACGGGATAATACCGCGCCACATAGCAGAACTTTAA
AAGTGCTCATCATTGGAAAACGTTCTTCGGGGCGAAAACTCTCAAGGATCTTACCGC
TGTTGAGATCCAGTTCGATGTAACCCACTCGTGCACCCAACTGATCTTCAGCATCTTT
TACTTTCACCAGCGTTTCTGGGTGAGCAAAAACAGGAAGGCAAAATGCCGCAAAAA
AGGGAATAAGGGCGACACGGAAATGTTGAATACTCATACTCTTCCTTTTTCAATATT
ATTGAAGCATTTATCAGGGTTATTGTCTCATGAGCGGATACATATTTGAATGTATTTA
GAAAAATAAACAAATAGGGGTTCCGCGCACATTTCCCCGAAAAGTGCCACCTAAAT
TGTAAGCGTTAATATTTTGTTAAAATTCGCGTTAAATTTTTGTTAAATCAGCTCATTT
TTTAACCAATAGGCCGAAATCGGCAAAATCCCTTATAAATCAAAAGAATAGACCGA
GATAGGGTTGAGTGTTGTTCCAGTTTGGAACAAGAGTCCACTATTAAAGAACGTGGA
CTCCAACGTCAAAGGGCGAAAAACCGTCTATCAGGGCGATGGCCCACTACGTGAAC
CATCACCCTAATCAAGTTTTTTGGGGTCGAGGTGCCGTAAAGCACTAAATCGGAACC
CTAAAGGGAGCCCCCGATTTAGAGCTTGACGGGGAAAGCCGGCGAACGTGGCGAGA
AAGGAAGGGAAGAAAGCGAAAGGAGCGGGCGCTAGGGCGCTGGCAAGTGTAGCGG
TCACGCTGCGCGTAACCACCACACCCGCCGCGCTTAATGCGCCGCTACAGGGCGCGT
CCCATTCGCCATTCAGGCTGCGCAACTGTTGGGAAGGGCGATCGGTGCGGGCCTCTT
CGCTATTACGCCAGCTGCGCGCTCGCTCGCTCACTGAGGCCGCCCGGGCAAAGCCCG
GGCGTCGGGCGACCTTTGGTCGCCCGGCCTCAGTGAGCGAGCGAGCGCGCAGAGAG
GGAGTGGCCAACTCCATCACTAGGGGTTCCTTGTAGTTAATGATTAACCCGCCATGC
TACTTATCTACGTAGCCATGCTCTAGGAAGAGTACCATTGACGTCAATAATGACGTA
TGTTCCCATAGTAACGCCAATAGGGACTTTCCATTGACGTCAATGGGTGGAGTATTT
ACGGTAAACTGCCCACTTGGCAGTACATCAAGTGTATCATATGCTCCGGAGCTTCTA
TTCCTGATCCCTGCATAAGAAGGAGACATGGTGGTGGTGGTGGGTGGGGGTGG
TGGGGCACAGAGGAAGCCGGTACTGGGCTCTGCACCCCATTCCCGCTCCCAGATCCC
TCTGGACACAGCATTTTTCTCCAGTGAGCACAGCCTCCCCTTGCCCCACAGCCAACA
GCAACATGCCTCCCAACAAAAGCATCTGTCCCTCAGCCAAAACCCCTGTTGCCTCTC
TCTGGGGAAATTGTAGGGCTGGGCCAGGGTGGGGGGACCATTCTCTGCAGGGAGAT
TAGGAGTGTCTGTCAGGGGCGGGTGGAGCGGGGTGGGGCCCTGGCTTACTCACATC

CTTGAGAGTCCTTTGCTGGCAGATTTGGGGAGCCCACAGCTCAGATGTCTGTCTCAG
CATTGTCTTCCAAGCTCCTAGGCCACAGTAGTGGGGGGCTCCCTTCTCTGGCTTCTTC
TTTGGTGACAGTCAAGGTGGGGTTGGGGGTGACAGAGGGTCCTGCTTCTCTTCTAGG
AGCAGTTGATCCCAGGAAGAGCATTGGAGCCTCCAGCAGGGGCTGTTGGGGCCTGT
CTGAGGAGATAGGATGCGTCAGGCAGCCCCAGACACGATCACATTCCTCTCAACAT
GCCTGCCGGGGTCTGTGGAGCCTAGGGGCTGATGGGAGGGTGGGGTGGGGGCCGGA
AGGGTTTAGGTCTATATAAGCAGAGCTCGTTTAGTGAACCGTCAGTCCGCCTGGAGA
CCTCGAGCCGAGTGGTCGTGCCTCCATAGAAGGATCCGCCACCATGGTGAGCAAGG
GCGAGGAGCTGTTCACCGGGGTGGTGCCCATCCTGGTCGAGCTGGACGGCGACGTA
AACGGCCACAAGTTCAGCGTGTCCGGCGAGGGCGAGGGCGATGCCACCTACGGCAA
GCTGACCCTGAAGTTCATTTGCACCACCGGCAAGCTGCCCGTGCCCTGGCCCACCCT
CGTGACCACCCTGACCTACGGCGTGCAGTGCTTCAGCCGCTACCCCGACCACATGAA
GCAGCACGACTTCTTCAAGTCCGCCATGCCCGAAGGCTACGTCCAGGAGCGCACCA
TCTTCTTCAAGGACGACGGCAACTACAAGACCCGCGCCGAGGTGAAGTTCGAGGGC
GACACCCTGGTGAACCGCATCGAGCTGAAGGGCATCGACTTCAAGGAGGACGGCAA
CATCCTGGGGCACAAGCTGGAGTACAACTACAACAGCCACAACGTCTATATCATGG
CCGACAAGCAGAAGAACGGCATCAAGGTGAACTTCAAGATCCGCCACAACATCGAG
GACGGCAGCGTGCAGCTCGCCGACCACTACCAGCAGAACACCCCCATCGGCGACGG
CCCCGTGCTGCTGCCCGACAACCACTACCTGAGCACCCAGTCCGCCCTGAGCAAAG
ACCCCAACGAGAAGCGCGATCACATGGTCCTGCTGGAGTTCGTGACCGCCGCCGGG
ATCACTCTCGGCATGGACGAGCTGTACAAGTCCGGACTCAGATCTGATCCAAAAAA
GAAGAGAAAGGTAGATCCAAAAAAGAAGAGAAAGGTAGATCCAAAAAAGAAGAG
AAAGGTATAAAAGCTTCGAGGAATTCGAAGCTTCTAGGTACCGAATTCGATATCAA
GCTTATCGATAATCAACCTCTGGATTACAAAATTTGTGAAAGATTGACTGGTATTCT
TAACTATGTTGCTCCTTTTACGCTATGTGGATACGCTGCTTTAATGCCTTTGTATCAT
GCTATTGCTTCCCGTATGGCTTTCATTTTCTCCTCCTTGTATAAATCCTGGTTGCTGTC
TCTTTATGAGGAGTTGTGGCCCGTTGTCAGGCAACGTGGCGTGGTGTGCACTGTGTT
TGCTGACGCAACCCCCACTGGTTGGGGCATTGCCACCACCTGTCAGCTCCTTTCCGG
GACTTTCGCTTTCCCCCTCCCTATTGCCACGGCGGAACTCATCGCCGCCTGCCTTGCC
CGCTGCTGGACAGGGGCTCGGCTGTTGGGCACTGACAATTCCGTGGTGTTGTCGGGG

AAATCATCGTCCTTTCCTTGGCTGCTCGCCTGTGTTGCCACCTGGATTCTGCGCGGGA
CGTCCTTCTGCTACGTCCCTTCGGCCCTCAATCCAGCGGACCTTCCTTCCCGCGGCCT
GCTGCCGGCTCTGCGGCCTCTTCCGCGTCTTCGCCTTCGCCCTCAGACGAGTCGGAT
CTCCCTTTGGGCCGCCTCCCCGCATCGATACCGTCGACCCGGGCGGCCGCTTCGAGC
AGACATGATAAGATACATTGATGAGTTTGGACAAACCACAACTAGAATGCAGTGAA
AAAAATGCTTTATTTGTGAAATTTGTGATGCTATTGCTTTATTTGTAACCATTATAAG
CTGCAATAAACAAGTTAACAACAACAATTGCATTCATTTTATGTTTCAGGTTCAGGG
GGAGATGTGGGAGGTTTTTTAAAGCAAGTAAAACCTCTACAAATGTGGTAAAATCG
ATAAGGATCTTCCTAGAGCATGGCTACGTAGATAAGTAGCATGGCGGGTTAATCATT
AACTACAAGGAACCCCTAGTGATGGAGTTGGCCACTCCCTCTCTGCGCGCTCGCTCG
CTCACTGAGGCCGGGCGACCAAAGGTCGCCCGACGCCCGGGCTTTGCCCGGGCGGC
CTCAGTGAGCGAGCGAGCGCGCAGCTGCATTAATGAATCGGCCAACGCGCGGGGAG
AGGCGGTTTGCGTATTGGGCGCT

The K16SCP3 promoter sequence (SEQ ID NO: 25) is as follows.

CTCCGGAGCTTCTATTCCTGATCCCTGCAGAAGAAGGAGACGGTGGTGGTGGGT
GGGGGTGGCAGGGCACAGAGGAAGCCAGTACCGGGCCCTGCACCCCATTCCCACTC
CCAGATCCCTCTGGACACAGCATTTTTCTCCAGTGAGCACAGCCTTCCCTTGCCCCA
CAGCCAACAGCAACATGGCTCCCAACAAAAGCATCTGCCCCTCAGCCAAAACCCCT
GTTGCCTCTCTCTGGGGAAATTGTAGGACTGGGTCAGGGTGGGGGAACCATTCTCTG
CAGGGAGATTAGGAGTGTCTGTCAGGGGTGGGTGGAGCGGGGTGGGGCCCTGGCTT
ACTCACATCCTCGAGAGTCCTTTGCTGGCAGATTTGGGGAGCCCACAGCACAGGTGT
CTGTCTCAGTATTGTCTTCCAAGCTCCTAGGCCACAGTAGTGGGGGGCTCCCCTCTCT
GGCTTTTTCTTTGGTGACAGTCAAGGTTGGGGGTGGGGTGAGAGAGGGTCCTGCTTT
TCTTCTAGGAACAGTTGATCCCAGGAAGAGCAGTGGAGCCTCCAGCAGGGGCTGTT
GGGGCCTGTCTGAGGAGATAGGACGCGTCAGGCAGCCCCAGACACGACCACATTCC
TCCCAACATGCCTGCCGGGGTCTGTGGAGCCCAGGGGCTGAGGGGAGGGTGGGGTG
GGGGCCGGAAGGGTTTAGGTCTATATAAGCAGAGCTCGTTTAGTGAACCGTCAGTCC
GCCTGGAGACCTCGAGCCGAGTGGTCGTGCCTCCATAGAA

The K16P5 short promoter sequence (SEQ ID NO: 26) is as follows.

CTCCGGAGCTTCTATTCCTGATCCCTGCAGAAGAAGGAGACGGTGGTGGTGGGT
GGGGGTGGCAGGGCACAGAGGAAGCCAGTACCGGGCCCTGCACCCCATTCCCACTC
CCAGATCCCTCTGGACACAGCATTTTTCTCCAGTGAGCACAGCCTTCCCTTGCCCCA
CAGCCAACAGCAACATGGCTCCCAACAAAAGCATCTGCCCCTCAGCCAAAACCCCT
GTTGCCTCTCTCTGGGGAAATTGTAGGACTGGGTCAGGGTGGGGGAACCATTCTCTG
CAGGGAGATTAGGAGTGTCTGTCAGGGGTGGGTGGAGCGGGGTGGGGCCCTGGCTT
ACTCACATCCTCGAGAGTCCTTTGCTGGCAGATTTGGGGAGCCCACAGCACAGGTGT
CTGTCTCAGTATTGTCTTCCAAGCTCCTAGGCCACAGTAGTGGGGGGCTCCCCTCTCT
GGCTTTTTCTTTGGTGACAGTCAAGGTTGGGGGTGGGGTGAGAGAGGGTCCTGCTTT
TCTTCTAGGAACAGTTGATCCCAGGAAGAGCAGTGGAGCCTCCAGCAGGGGCTGTT
GGGGCCTGTCTGAGGAGATAGGACGCGTCAGGCAGCCCCAGACACGACCACATTCC
TCCCAACATGCCTGCCGGGGTCTGTGGAGCCCAGGGGCTGAGGGGAGGGTGGGGTG
GGGGCCGGAAGGGTTTGAGGGCTGGGACTCCCGGGGCCCCATGGGAAAGTGTAGCC
TGCAGGCCCATACCTCCCCCTGTGAATCACGCCTGGCGGGACAAGAAAGCCCAAAA
TACCCCAAACAATGAGTTTCCAGTAAAATATGACAGACATGATGAGGTGGAGGAGA
GGAGGGACCTGGCTGGGAGTTGGCGCTAGCCTGTGGGTGATGAAAGCCAAGGGGAA
TGGAAAGTGCCAGGCCCGCCTCCTACCCATGAGTATAAAGCACTCGCATCCCTTTCC
AATTTACCCGAGCACCTTCTCTTCACTCAGCCAACTGCTCGCTC

The K14 promoter sequence (SEQ ID NO: 27) is as follows.

CTCCGGAGCTTCTATTCCTGATCCCTGCATAAGAAGGAGACATGGTGGTGGTGG
TGGGTGGGGGTGGTGGGGCACAGAGGAAGCCGGTACTGGGCTCTGCACCCCATTCC
CGCTCCCAGATCCCTCTGGACACAGCATTTTTCTCCAGTGAGCACAGCCTCCCCTTG
CCCCACAGCCAACAGCAACATGCCTCCCAACAAAAGCATCTGTCCCTCAGCCAAAA
CCCCTGTTGCCTCTCTCTGGGGAAATTGTAGGGCTGGGCCAGGGTGGGGGGACCATT
CTCTGCAGGGAGATTAGGAGTGTCTGTCAGGGGCGGGTGGAGCGGGGTGGGGCCCT
GGCTTACTCACATCCTTGAGAGTCCTTTGCTGGCAGATTTGGGGAGCCCACAGCTCA
GATGTCTGTCTCAGCATTGTCTTCCAAGCTCCTAGGCCACAGTAGTGGGGGGCTCCC
TTCTCTGGCTTCTTCTTTGGTGACAGTCAAGGTGGGGTTGGGGGTGACAGAGGGTCC

TGCTTCTCTTCTAGGAGCAGTTGATCCCAGGAAGAGCATTGGAGCCTCCAGCAGGGG
CTGTTGGGGCCTGTCTGAGGAGATAGGATGCGTCAGGCAGCCCCAGACACGATCAC
ATTCCTCTCAACATGCCTGCCGGGGTCTGTGGAGCCTAGGGGCTGATGGGAGGGTGG
GGTGGGGGCCGGAAGGGTTTGCTTTGGGAGGTTGTCTGGGAGATTGCTGAAGTTTTG
ATATACACACCTCCAAAGCAGGACCAAGTGGACTCCTAGAAATGTCCCCTGACCCTT
GGGGCTTCAGGAGTCAGGGACCCTCGTGTCCACCTCAGCCTTGCCCTTGGCACAGCC
CAGCTCCACTCCAGCCTCTACTCCTCCCCAGAACATCTCCTGGGCCAGTTCCACAAG
GGGCTCAAACGAGGGCGCCTGAGCTGCCCACACTAGGGATGTTCTGGGGGTCTGAG
AAGATATCTGGGGCTGGAAGAATAAAAGGCCCCCCTAGGCCTGTTCCTGGATGCAG
CTCCAGCCACTTTGGGGCTAAGCCTGGGCTATAACAATGCCAACGAGGCTTCTTGCC
ATACTCGGTTTACAAAACCCTTTCACATACATTGTCGCATTGGATTCTCAGAGCTGA
CTGCACTAAGCAGAATAGATGGTATGACTCCCACTTTGCAGATGAGAACACTGAGG
CTCAGAGAAGTGCCAAGCCCTGGGTCACAGAGGCGTAAATGGCAGAGCCAGGACCC
ACCTGACTCCAGGCTGTTTCCTGGCCTCCATGAGGCCACCTGCCCTATGGTGTGGTG
GATGTGAGATCCTCACCATAGGGAGGAGATTAGGGTCTGTGCTCAGGGATGGGGAG
AGCTGCCTGGATTTCTCTTTGATGGGGATGTTGGGGTGGGAATCACGATACACCTGA
CTAGCTGGGTGTATTTCAGGGATGGGACAGACTTCTCAGCACAGCATGGGAGGTCA
GGCCTGGGAGGGCCCCCCAGACCTCCTTGTCTCTAATAGAGGGTCATGGTGAGGGA
GGCCTGTCTGTGCCCAAGGTGACCTTGCCATGCCGGTGCTTTCCAGCCGGGTATCCA
TCCCCTGCAGCAGCAGGCTTCCTCTACGTGGATGTTAAAGGCCCATTCAGTTCATGG
AGAGCTAGCAGGTAACTAGGTTTAAGGTGCAGAGGCCCTGCTCTCTGTCACCCTGGC
TAAGCCCAGTGCGCGGGTTCCTGAGGGCTGGGACTCCCAGGGTCCGATGGGAAAGT
GTAGCCTGCAGGCCCACACCTCCCCCTGTGAATCACGCCTGGCGGGACAAGAAAGC
CCAAAACACTCCAAACAATGAGTTTCCAGTAAAATATGACAGACATGATGAGGCGG
ATGAGAGGAGGGACCTGGCTGGGAGTTGGCGCTAGCCTGTGGGTGATGAAAGCCAA
GGGGAATGGAAAGTGCCAGACCCGCCCCCTACCCACGAGTATAAAGCACTCGCATC
CCTTTCCAATTTACCCGAGCACC

The K14 short promoter sequence (SEQ ID NO: 28) is as follows.

CTCCGGAGCTTCTATTCCTGATCCCTGCATAAGAAGGAGACATGGTGGTGGTGG
TGGGTGGGGGTGGTGGGGCACAGAGGAAGCCGGTACTGGGCTCTGCACCCCATTCC
CGCTCCCAGATCCCTCTGGACACAGCATTTTTCTCCAGTGAGCACAGCCTCCCCTTG
CCCCACAGCCAACAGCAACATGCCTCCCAACAAAAGCATCTGTCCCTCAGCCAAAA
CCCCTGTTGCCTCTCTCTGGGGAAATTGTAGGGCTGGGCCAGGGTGGGGGGACCATT
CTCTGCAGGGAGATTAGGAGTGTCTGTCAGGGGCGGGTGGAGCGGGGTGGGGCCCT
GGCTTACTCACATCCTTGAGAGTCCTTTGCTGGCAGATTTGGGGAGCCCACAGCTCA
GATGTCTGTCTCAGCATTGTCTTCCAAGCTCCTAGGCCACAGTAGTGGGGGGCTCCC
TTCTCTGGCTTCTTCTTTGGTGACAGTCAAGGTGGGGTTGGGGGTGACAGAGGGTCC
TGCTTCTCTTCTAGGAGCAGTTGATCCCAGGAAGAGCATTGGAGCCTCCAGCAGGGG
CTGTTGGGGCCTGTCTGAGGAGATAGGATGCGTCAGGCAGCCCCAGACACGATCAC
ATTCCTCTCAACATGCCTGCCGGGGTCTGTGGAGCCTAGGGGCTGATGGGAGGGTGG
GGTGGGGGCCGGAAGGGTTTGAGGGCTGGGACTCCCAGGGTCCGATGGGAAAGTGT
AGCCTGCAGGCCCACACCTCCCCCTGTGAATCACGCCTGGCGGGACAAGAAAGCCC
AAAACACTCCAAACAATGAGTTTCCAGTAAAATATGACAGACATGATGAGGCGGAT
GAGAGGAGGGACCTGGCTGGGAGTTGGCGCTAGCCTGTGGGTGATGAAAGCCAAGG
GGAATGGAAAGTGCCAGACCCGCCCCCTACCCACGAGTATAAAGCACTCGCATCCC
TTTCCAATTTACCCGAGCACCTTCTCTTCACTCAGCCAACTGCTCGCTCG

The K16P5 promoter sequence (SEQ ID NO: 29) is as follows.

CTCCGGAGCTTCTATTCCTGATCCCTGCAGAAGAAGGAGACGGTGGTGGTGGGT
GGGGGTGGCAGGGCACAGAGGAAGCCAGTACCGGGCCCTGCACCCCATTCCCACTC
CCAGATCCCTCTGGACACAGCATTTTTCTCCAGTGAGCACAGCCTTCCCTTGCCCCA
CAGCCAACAGCAACATGGCTCCCAACAAAAGCATCTGCCCCTCAGCCAAAACCCCT
GTTGCCTCTCTCTGGGGAAATTGTAGGACTGGGTCAGGGTGGGGGAACCATTCTCTG
CAGGGAGATTAGGAGTGTCTGTCAGGGGTGGGTGGAGCGGGGTGGGGCCCTGGCTT
ACTCACATCCTCGAGAGTCCTTTGCTGGCAGATTTGGGGAGCCCACAGCACAGGTGT
CTGTCTCAGTATTGTCTTCCAAGCTCCTAGGCCACAGTAGTGGGGGGCTCCCCTCTCT
GGCTTTTTCTTTGGTGACAGTCAAGGTTGGGGGTGGGGTGAGAGAGGGTCCTGCTTT
TCTTCTAGGAACAGTTGATCCCAGGAAGAGCAGTGGAGCCTCCAGCAGGGGCTGTT

GGGGCCTGTCTGAGGAGATAGGACGCGTCAGGCAGCCCCAGACACGACCACATTCC
TCCCAACATGCCTGCCGGGGTCTGTGGAGCCCAGGGGCTGAGGGGAGGGTGGGGTG
GGGGCCGGAAGGGTTTGCTTTGGGAGGTTGTCTGGGAGATTGCTGAAGTTTTGATAT
ACACACCTCCAAAGCAGGACCAAGTGGACTCCTAGAAATGTCCCCTGACCCTTGGG
GCTTCAGGAGTCAGGGACCCTTGTGTCCACTTCAGCCTTGCCCTTGGCACAGCCTGG
CACCACTCCAGCTTCTACTCCTCCCCAGAACATCTCCTGGGCCAGTTCCACAAGGGG
CTCAAACGAGGGCACCTGAGCCAGACTTCTGCCTACACTAGGGATGTTCTGGGGGTC
TGAGAGGATATCTGGGGCTGGAAGAAAAAAAGGCCCCCCTAGGCCTGTTCCTGGAT
GCAGCTCCATCCACTTTGGGGCTAAGCCTGGGCTACAACAATGCCAACCAGGCTTCT
TGCCATACTCGGTTTACAAAAGCCTTTCACATACGCTGTCGCATTGGCTTCTCACAGC
TGACTGCAGTAGGCAGAGTAGATGGTATGACTCCCACTTTGCAGATGAGAACACTG
AGGCTCAGAGAAGCGCCAAGCCCTGGGTCACGGGGGCGTAAATGGCAGAGCCAGG
ACCCACCTGACTCCAGGCTGTTTCCTGGCCTCCATGAGGCCACCCGCCCTATGGTGT
GGTGGATGTCAGATCCTCACCGTAGGGAGGAAATTAGGGTCTGTGCCCGGGGCTGG
GGAGAGCTGCCTAGATTTCTCTTTGATGGGGGTGTTGGGGTGGGAATCACCATACAC
CTGACTGGCTGAGTGTATTTCAGGGATGGGACAGGCTTCTCAGCACAGCATGGCAG
GTCAGGCCTGGGAGGGCCCCCCAGACCTCCTTGTCTCTAACAGCGGGTCATGGTGAG
GGAGGCCTCTCTGTGCCCAAGGTGACCTTGCCATGCCGGTGCTCTCCAGCTGGGTAT
CTGTCCCCTGCAGTGGTGGGCTTCCTCTAAGTGGATGGTAAAGGCCCATCCAGTTCA
TGGAGAGCTAGCAGGTCACCAGGTTTAAGGTGCAGAGGCCCTGCTCTCTGTCACCCT
GGCTGAGCCCAGTGTGCGGGTTCCTGAGGGCTGGGACTCCCGGGGCCCCATGGGAA
AGTGTAGCCTGCAGGCCCATACCTCCCCCTGTGAATCACGCCTGGCGGGACAAGAA
AGCCCAAAATACCCCAAACAATGAGTTTCCAGTAAAATATGACAGACATGATGAGG
TGGAGGAGAGGAGGGACCTGGCTGGGAGTTGGCGCTAGCCTGTGGGTGATGAAAGC
CAAGGGGAATGGAAAGTGCCAGGCCCGCCTCCTACCCATGAGTATAAAGCACTCGC
ATCCCTTTCCAATTTACCCGAGCACC

### 9. Experimental Results 3

The present invention will be described in detail below based on examples and the like; however, the invention is not limited to these.

### [Example 5]

Figure 6 shows the results of a comparative study on gene expression in mouse and human cultured keratinocytes and mesenchymal cells using AAV with an AAVDJK2 capsid as the vector. These AAVs expressed GFPNLS under the control of the newly developed K14SCP3 promoter (SEQ ID NO: 23), K16SCP3 promoter (SEQ ID NO: 24), and K16P5 short promoter (SEQ ID NO: 25), as well as the known CAG promoter, K14 promoter (SEQ ID NO: 27), K14 short promoter (SEQ ID NO: 28), and K16P5 promoter (SEQ ID NO: 29). The concentrated AAV preparation method described in Example 2 was used to prepare AAVDJK2 vectors expressing GFPNLS under each promoter. Mouse cultured keratinocytes were prepared using the method described in Example 2, and mouse adipose-derived mesenchymal cells were prepared using the method described in Example 3. Mouse keratinocytes were cultured in 24-well plates, and when they reached 50% confluence, the medium was replaced with medium containing AAV at 1.0×10⁹ GC/well. Four wells were treated under identical conditions for each AAV. The medium was replaced on days 1 and 3. On days 2 and 4, phase-contrast microscopy and fluorescence imaging were performed in four fields of view. Using ImageJ software, the area of keratinocyte colonies in the images was calculated. The number of GFPNLS-positive cells within each colony area was counted from the images, and the number of GFPNLS-positive cells per unit area of the keratinocyte colony was determined. The results showed that in mouse cultured keratinocytes, the K14SCP3 promoter outperformed the CAG promoter, K14 promoter, and K14 short promoter. Additionally, the K16SCP3 promoter and K16P5 short promoter outperformed the K14 promoter, K14 short promoter, and K16P5 promoter in terms of gene transduction efficiency.

Mouse keratinocytes were cultured in 24-well plates, and when they reached 50% confluence, the medium was replaced with medium containing AAV at 1.0×10⁹ GC/well. Four wells were treated under identical conditions for each AAV. The medium was replaced on days 1 and 3, and on day 4, the keratinocytes were trypsinized in a manner similar to passaging to prepare a suspension. The suspension was passed through a 35 µm filter, spun down, and resuspended in phosphate-buffered saline (PBS) containing 5% FBS and 2 mM EDTA to prepare a single-cell suspension. Flow cytometry analysis was performed using a FACSMelody Cell Sorter (BD, USA) and FlowJo software (BD, USA). Plots were generated based on the percentage of GFP-positive cells and the fluorescence intensity of GFP. The results showed that the K14SCP3 promoter outperformed the CAG promoter, K14 promoter, and K14 short promoter. Additionally, the K16SCP3 promoter and K16P5 short promoter outperformed the K14 promoter, K14 short promoter, and K16P5 promoter in terms of gene transduction efficiency.

Mouse adipose-derived mesenchymal cells were cultured in 24-well plates, and when they reached 50% confluence, the medium was replaced with medium containing AAV at 1.0×10⁹ GC/well. Four wells were treated under identical conditions for each AAV. The medium was replaced on days 1 and 3. Phase-contrast microscopy and fluorescence imaging were performed in four fields of view on days 2 and 4. The number of GFPNLS-positive cells per fluorescence image (area: 1.99 mm²) was counted. The results showed that the gene transduction efficiency of keratin-specific promoters, including the novel K14SCP3 promoter, K16SCP3 promoter, and K16P5 short promoter, was significantly lower than that of the CAG promoter.

Mouse adipose-derived mesenchymal cells were cultured in 24-well plates, and when they reached 50% confluence, the medium was replaced with medium containing AAV at 1.0×10⁹ GC/well. Four wells were treated under identical conditions for each AAV. The medium was replaced on days 1 and 3, and on day 4, the mouse adipose-derived mesenchymal cells were trypsinized in a manner similar to passaging to prepare a suspension. The suspension was passed through a 35 µm filter, spun down, and resuspended in phosphate-buffered saline (PBS) containing 5% FBS and 2 mM EDTA to prepare a single-cell suspension. Flow cytometry analysis was performed using a FACSMelody Cell Sorter (BD, USA) and FlowJo software (BD, USA). Plots were generated based on the percentage of GFP-positive cells and the fluorescence intensity of GFP. The results showed that the gene transduction efficiency of keratin-specific promoters, including the novel K14SCP3 promoter, K16SCP3 promoter, and K16P5 short promoter, was significantly lower than that of the CAG promoter in mouse adipose-derived mesenchymal cells. Overall, the novel promoters demonstrated higher gene transduction efficiency and specificity for keratinocytes.

### [Example 6]

Figure 7 shows the results of a gene transduction experiment in which AAVDJK2 expressing GFPNLS under the control of the K14SCP3 promoter was intradermally injected into the dorsal skin of mice. Using the concentrated AAV preparation method described in Example 2, an AAVDJK2 vector expressing GFPNLS under the control of the K14SCP3 promoter was prepared. After subcutaneous injection of 10 µl of steroid (Kenacort A, 40 mg/ml) into the abdominal region, the dorsal skin of the mouse was shaved using a razor. Intradermal injection of AAVDJK2-K14SCP3-GFPNLS (10¹¹ GC/35 µl) was then performed. Two days later, the surrounding skin tissue, including the injection site, was collected and fixed overnight in 4% paraformaldehyde-phosphate buffer (pH 7.4). The tissue was subsequently replaced with 30% sucrose/phosphate-buffered saline (PBS), embedded in OCT compound, and frozen. Cryofilm (Cryofilm type 2C(9), 2.5 cm, C-FP094, SECTION-LAB, Kawamoto Sheet) was used to collect 10 µm thick sections every 200 µm from the frozen tissue. The sections were washed with PBS and stained with DAPI for nuclear staining. The images were captured as image files using a slide scanner (VS200, Olympus). High GFPNLS expression was observed specifically in the epidermal tissue, while GFPNLS-positive cells in the dermis and subcutaneous tissue were observed at a low frequency, excluding the epidermal tissue of hair follicles. These findings demonstrate that AAVDJK2, with the K14SCP3 promoter, functions as a gene transduction vector with high efficiency and specificity for epidermal tissues.

### 10. Applications of Methods for Inducing Skin Appendages In Vivo

Skin appendages, including hair follicles, sebaceous glands, and sweat glands, play roles such as protection from mechanical damage, insulation, moisturizing, and temperature regulation. Skin appendages are formed during the fetal stage through interactions between epithelial and mesenchymal tissues during organogenesis (see Non-Patent Document 4). Representative conditions caused by the loss, damage, or dysfunction of skin appendages include alopecia and sebaceous deficiency. These conditions can lead to thermoregulatory disorders and itching due to skin dryness, as well as cosmetic impairments. Such conditions are often associated with physiological changes that occur during the aging process. Clinically, it is challenging to regenerate lost skin appendages, typically requiring the transplantation of existing appendages through procedures such as skin grafting or flap surgery. In contrast, if the regeneration and creation of skin appendages could be induced in vivo, it would offer a therapeutic approach to address various conditions, including pathological states characterized by the loss of skin and skin appendages, such as skin ulcers, alopecia, sebaceous deficiency, and age-related changes accompanied by a decline in skin appendage function. This approach could promote the regeneration, creation, and functional enhancement of skin appendages, thereby restoring skin appendage functions. Furthermore, it could serve as a research tool for drug discovery and biological studies aimed at the regeneration, creation, and functional enhancement of skin appendages.

### 11. Method for Inducing Skin Appendages In Vivo

The method for inducing skin appendages in vivo described in the present invention is characterized by a process that confers skin appendage-inducing ability to somatic cells that lack this ability through gene transduction, protein administration, or compound administration. Hereinafter, genes transduced during gene transduction will be referred to as "transduced genes." The term "transduced genes" encompasses not only genes encoding proteins but also noncoding RNAs such as microRNAs.

Genes that confer skin appendage-inducing ability include at least the following protein-coding genes: DNP63A, GRHL2, TFAP2A, cMYC, LEF1, SOX2, HOXC4, HOXC9, HOXC13, JARID2, HEY1, HEY2, FOXO1, FOXD1, EGR3, MEF2C, LHX2, PRRX1, PRRX2, CREB3, ETV1, TBX6, MSX2, PRDM1, and SHH.

Proteins that confer skin appendage-inducing ability include at least the proteins encoded by the following genes: DNP63A, GRHL2, TFAP2A, cMYC, LEF1, SOX2, HOXC4, HOXC9, HOXC13, JARID2, HEY1, HEY2, FOXO1, FOXD1, EGR3, MEF2C, LHX2, PRRX1, PRRX2, CREB3, ETV1, TBX6, MSX2, PRDM1, and SHH.

Any of the genes used in the present invention are publicly known (Table 1). In this specification, "NCBI" stands for the National Center for Biotechnology Information, and the Accession Nos. listed in Table 1 refer to those registered in the database provided by NCBI.

**[Table 1]**

| **Gene** | **Human** | **Mouse** | **Gene** | **Human** | **Mouse** |
|---|---|---|---|---|---|
| | **Reference sequences** | **Reference sequences** | | **Reference sequences** | **Reference sequences** |
| **DNP63A** | NM_001114980.2 | NM_011641.2 | **MEF2C** | NM_001131005.2 | NM_001170537.1 |
| **GRHL2** | NM_001330593.2 | NM_026496.4 | | NM_001193347.1 | NM_001347564.1 |
| | NM_024915.4 | | | NM_001193348.1 | NM_001347566.1 |
| **TFAP2A** | NM_001032280.3 | NM_001122948.2 | | NM_001193349.3 | NM_001347567.1 |
| | NM_001042425.3 | NM_001301674.2 | | NM_001193350.2 | NM_001347568.1 |
| | NM_001372066.1 | NM_011547.5 | | NM_001308002.3 | NM_001347569.1 |
| **cMYC** | NM_001354870.1 | NM_001177352.1 | | NM_001363581.2 | NM_001347571.1 |
| | NM_002467.6 | NM_001177353.1 | | NM_001364329.2 | NM_001347572.1 |
| | | NM_001177354.1 | | NM_001364330.2 | NM_001347573.1 |
| | | NM_010849.4 | | NM_001364331.2 | NM_001347574.1 |
| **LEF1** | NM_001130713.3 | NM_001276402.1 | | NM_001364332.2 | NM_001347575.1 |
| | NM_001130714.3 | NM_001276403.1 | | NM_001364333.2 | NM_001347576.1 |
| | NM_001166119.2 | NM_001379059.1 | | NM_001364334.2 | NM_001347577.1 |
| | NM_016269.5 | NM_001379060.1 | | NM_001364335.2 | NM_001347578.1 |
| | | NM_001379061.1 | | NM_001364336.2 | NM_001347579.1 |
| | | NM_001379062.1 | | NM_001364337.2 | NM_001347580.1 |
| | | NM_010703.4 | | NM_001364338.2 | NM_001347581.1 |
| **SOX2** | NM_003106.4 | NM_011443.4 | | NM_001364339.2 | NM_025282.3 |
| **HOXA9** | NM_152739.4 | NM_001277238.1 | | NM_001364340.2 | |
| | | NM_010456.3 | | NM_001364341.2 | |
| **HOXC4** | NM_014620.6 | NM_013553.2 | | | |
| | NM_153633.3 | | | NM_001364342.2 | |
| **HOXC9** | NM_006897.3 | NM_008272.3 | | NM_001364343.2 | |
| **HOXC13** | NM_017410.3 | NM_010464.2 | | NM_001364344.2 | |
| **JARID2** | NM_001267040.1 | NM_001205043.1 | | NM_001364345.2 | |
| | NM_004973.4 | NM_001205044.1 | | NM_001364346.2 | |
| | | NM_001360281.1 | | NM_001364347.2 | |
| | | NM_021878.3 | | NM_001364348.2 | |
| | | | | NM_001364349.2 | |
| **HEY1** | NM_001040708.2 | NM_010423.2 | | NM_001364350.2 | |
| | NM_001282851.2 | | | NM_001364352.2 | |
| | NM_012258.4 | | | NM_001364353.2 | |
| **HEY2** | NM_012259.3 | NM_013904.1 | | NM_001364354.2 | |
| **FOXO1** | NM_002015.4 | NM_019739.3 | | NM_001364355.2 | |
| **FOXD1** | NM_004472.3 | NM_008242.2 | | NM_001364356.2 | |
| | | | | NM_001364357.2 | |
| **EGR3** | NM_001199880.2 | NM_001289925.2 | | NM_002397.5 | |
| | NM_001199881.2 | NM_001289927.1 | | | |
| | NM_004430.3 | NM_018781.4 | | | |
| | | | **CREB3** | NM_006368.5 | NM_013497.4 |

| **Gene** | **Human** | **Mouse** | | | |
|---|---|---|---|---|---|
| | **Reference sequences** | **Reference sequences** | | | |
| **LHX2** | NM_004789.4 | NM_001290646.1 | | | |
| | | NM_010710.4 | | | |
| **PRRX1** | NM_006902.5 | NM_001025570.1 | | | |
| | NM_022716.4 | NM_011127.2 | | | |
| | | NM_175686.3 | | | |
| **ETV1** | NM_001163147.2 | NM_001163154.1 | | | |
| | NM_001163148.2 | NM_001347379.1 | | | |
| | NM_001163149.2 | NM_007960.5 | | | |
| | NM_001163150.2 | | | | |
| | NM_001163151.2 | | | | |
| | NM_001163152.2 | | | | |
| | NM_001370555.1 | | | | |
| | NM_001370556.1 | | | | |
| | NM_004956.5 | | | | |
| **cMYC** | NM_001354870.1 | NM_001177352.1 | | | |
| | NM_002467.6 | NM_001177353.1 | | | |
| | | NM_001177354.1 | | | |
| | | NM_010849.4 | | | |
| **TBX6** | NM_004608.4 | NM_011538.2 | | | |
| **MSX2** | NM_001363626.2 | NM_013601.2 | | | |
| | NM_002449.5 | | | | |
| **PRDM1** | NM_001198.4 | NM_007548.4 | | | |
| | NM_182907.3 | | | | |

Many of these genes are commonly found in mammals, including humans, and any mammalian-derived genes can be used. However, it is preferable to select the genes appropriately based on the origin of the somatic cells into which they are transduced. For example, in cases where the target is humans, it is preferable that the transduced genes and proteins are human- derived. Additionally, the transduced genes and proteins may include mutant genes that encode mutant gene products with equivalent functions to the wild-type gene products. Such mutant genes may involve substitutions, deletions, and/or insertions of a small number of amino acids (e.g., 1 to 10, preferably 1 to 6, more preferably 1 to 4, even more preferably 1 to 3, and most preferably 1 or 2) in the amino acid sequence. Furthermore, codon changes or optimization to encode amino acids equivalent to those encoded by the original genes may also be used.

In the present invention, the transduced genes can be prepared according to conventional methods based on publicly available sequence information. For example, RNA can be extracted from mammalian cells, and the cDNA of the target gene can be prepared through cloning in accordance with standard methods. Additionally, artificial genes can be synthesized. During artificial gene synthesis, codon optimization can be performed to better fit the origin species of the somatic cells into which the genes are transduced.

In the present invention, the "somatic cells" induced to acquire skin appendage-inducing ability are not particularly limited in type and include those derived from any tissue or site. Specifically, somatic cells expected to serve as the origin for induction in the present invention include those derived from tissues such as skin, subcutaneous fat, muscle, fascia, and blood cells. More specifically, examples include dermal fibroblasts, stromal cells derived from subcutaneous adipose tissue (subcutaneous adipocytes), adipocytes, muscle cells, fascial cells, tissue monocytes, and tissue macrophages.

The transduction of the above-mentioned transduced genes into somatic cells can be performed using methods commonly employed for transfection into animal cells. Specifically, methods for transducing the genes into somatic cells include vector-based methods and mRNA-based methods, which may involve appropriate delivery techniques such as lipid nanoparticles. Among these methods, vector-based methods are preferred due to their superior transduction efficiency. When transducing the above-mentioned genes into somatic cells using vectors, virus vectors, non-viral vectors (including plasmid DNA vectors), and artificial viruses can be employed. Virus vectors such as adeno-associated viruses, retroviruses, and lentiviruses are particularly suitable from the perspective of transduction efficiency. mRNA-based methods are particularly suitable from a safety perspective. When multiple transduced genes are used, they may be incorporated into separate vectors or mRNAs, or two or more transduced genes may be incorporated into a single vector or mRNA.

The administration of the above-mentioned proteins to somatic cells can be performed either by directly administering the proteins themselves or by using protein transfection methods employing cell-penetrating peptides, depending on the properties of the proteins.

In the present invention, somatic cells in vivo can be induced to acquire skin appendage-inducing ability through gene transduction, protein administration, or compound administration, enabling the regeneration and creation of skin appendages. The transduced genes and proteins may include those that are relatively highly expressed in epithelial cells with skin appendage-inducing ability or those that are relatively highly expressed in mesenchymal cells with skin appendage-inducing ability. Additionally, both types of genes and proteins may be transduced or administered simultaneously.

The method for inducing skin appendages in vivo described in the present invention is characterized by a process that confers skin appendage-inducing ability to somatic cells that lack this ability through gene transduction, protein administration, or compound administration. The present invention can be applied not only to pathological conditions characterized by the loss of skin and skin appendages, such as skin ulcers, but also to various conditions involving a decline in skin appendage function, including alopecia, sebaceous deficiency, and age-related changes associated with these conditions. It can improve states where skin appendage function is reduced in terms of both quantity and quality. However, in experimental animals such as wild-type mice that do not have genetic abnormalities, it is difficult to accurately assess the degree of improvement from therapeutic interventions because it is challenging to create conditions such as hair sparsity or sebaceous deficiency, which represent a quantitative decline in skin appendage function.

Therefore, in testing the effectiveness of the present invention in conferring skin appendage-inducing ability to somatic cells that lack this ability through gene transduction, protein administration, or compound administration, an experimental model was adopted in which skin tissue with skin appendages was newly induced on a skin ulcer isolated from surrounding skin by a chamber. As confirmed in Non-Patent Document 2, skin ulcers isolated from surrounding skin do not physiologically regenerate even the skin or epidermal tissue. This ensures that the skin appendages associated with the induced skin are clearly attributable to the effects of the present invention.

### 12. Experimental Results 4

The present invention will be described in detail below based on examples and the like; however, the invention is not limited to these.

### [Example 7]

Figure 8 shows the results of an experiment in which skin with skin appendages was induced on an ulcerated area isolated from the surrounding skin using AAV-based gene transduction. A skin ulcer was created on the dorsal side of 4-5-week-old C57BL/6J Jcl mice, and a chamber was sutured to the deep fascia and secured to the surrounding skin to create an ulcerated area isolated from the surrounding skin. As confirmed in Non-Patent Document 2, ulcerated areas isolated by the chamber do not physiologically regenerate skin or epidermal structures, and the ulcer does not heal. The AAV vectors used for gene transduction were prepared using the concentrated AAV preparation method described in Example 2. After chamber attachment, gene transduction was performed by seeding AAV vectors into the chamber. The capsids, transduced genes, and titers of each vector were as follows:
- AAVDJ1-DNP63A: 1×10¹² GC (Gene copies)
- AAVDJ1-GRHL2: 1×10¹¹ GC
- AAVDJ1-TFAP2A: 5×10¹¹ GC
- AAVDJ1-cMYC: 5×10¹¹ GC
- AAVDJ1-LEF1: 2×10¹¹ GC

On day 9 after chamber attachment and initial AAV administration, additional gene transduction was performed by seeding the following AAV vectors into the chamber:
- AAVDJ-FOXD1: 2.5×10¹¹ GC
- AAVDJ-PRDM1 : 2.5×10¹¹ GC
- AAVDJ1-LEF1: 1×10¹¹ GC
- AAVDJ1-SHH: 5×10¹⁰ GC
From day 21 after chamber attachment and initial AAV administration, epidermal tissue induction in the ulcerated area was observed. By day 28, the formation of epidermal tissue in part of the ulcerated area was clearly visible, and hair emergence was confirmed in some areas. After capturing images of the ulcerated area using a stereomicroscope (Axio Zoom .V16, Zeiss), tissues including the ulcerated area were collected, fixed in 4% paraformaldehyde, embedded in OCT compound, and processed into frozen sections. The sections were immunostained with:
- Cytokeratin 14 antibody (ab181595, Abcam)
- A secondary antibody
- DAPI Fluoromount-G (Southern Biotech) for nuclear staining and mounting
Fluorescent images were acquired using a fluorescence slide scanner (VS200, Olympus) and a confocal microscope (Zeiss LSM900). After imaging, the mounting medium was gradually washed away, and the same sections were stained with hematoxylin and eosin (HE), remounted, and imaged again using a slide scanner. Histological observations confirmed the presence of skin appendages, such as hair follicle structures and sebaceous gland structures, in newly induced epidermal regions independent of the surrounding tissue. Since skin with skin appendages was observed in an ulcerated area that physiologically does not regenerate even epidermal structures, these results demonstrate that skin with skin appendages can be induced through gene transduction using AAV.

### [Example 8]

Figure 9 shows the results of an experiment in which skin with skin appendages was induced on an ulcerated area isolated from the surrounding skin using AAV-based gene transduction. The experiment was conducted using the same method as in Example 7, except for differences in the timing of chamber attachment and gene transduction, as well as the vectors used for gene transduction. On the day after chamber attachment, gene transduction was performed by seeding AAV vectors into the chamber. The capsids, transduced genes, and titers of each vector were as follows:
- AAVDJ-DNP63A: 1×10¹² GC (Gene copies)
- AAVDJ-GRHL2: 1×10¹¹ GC
- AAVDJ-TFAP2A: 5×10¹¹ GC
- AAVDJ-cMYC: 5×10¹¹ GC
- AAVDJ-LEF1: 2×10¹¹ GC

On day 9 after the initial AAV administration, additional gene transduction was performed by seeding the following AAV vectors into the chamber:
- AAVDJ-FOXD1: 2.5×10¹¹ GC
- AAVDJ-PRDM1 : 2.5×10¹¹ GC
- AAVDJK2-LEF1: 2.5×10¹¹ GC
- AAVDJ1-SHH: 1×10¹¹ GC
From day 21 after chamber attachment and the initial AAV administration, epidermal tissue induction in the ulcerated area was observed. By day 28, the formation of epidermal tissue in part of the ulcerated area was clearly observed, and by day 30, black hair emergence was confirmed in some areas. These findings demonstrate that gene transduction using AAV can induce skin tissue, independent of the surrounding area, with the emergence of black hair.

As understood from Examples 7 and 8, the in vivo induction of skin with skin appendages was achieved by transducing the five genes DNP63A, GRHL2, TFAP2A, c-MYC, and LEF1, followed by FOXD1, PRDM1, LEF1, and SHH into a skin ulcer isolated from the surrounding skin. As confirmed in Non-Patent Document 2, ulcerated areas isolated from the surrounding skin do not physiologically regenerate even skin or epidermal structures. Therefore, it is evident that the skin appendages associated with the induced skin were a result of the effects of the present invention.

According to Patent Document 1 (International Publication No. WO 2022/244502), it has been demonstrated that cells with skin appendage-inducing ability, particularly epithelial cells, can be induced by transducing DNP63A, GRHL2, TFAP2A, c-MYC, and LEF1, or by transducing DNP63A, GRHL2, TFAP2A, and LEF1 into primary cultured mesenchymal cells derived from adult mouse adipose tissue. Furthermore, it has been shown that cells with skin appendage-inducing ability, particularly mesenchymal cells, can be induced by transducing one or two groups of genes listed in Table 2 into primary cultured mesenchymal cells derived from adult mouse adipose tissue. Additionally, based on findings that mixed transplantation of induced epithelial cells with skin appendage-inducing ability and induced mesenchymal cells with skin appendage-inducing ability into a silicone chamber implanted on the dorsal side of immunodeficient animals leads to the reconstitution and regeneration of skin tissue with skin appendages, as well as the results of Examples 7 and 8, it is suggested that in vivo induction of skin with skin appendages can be achieved by transducing either the five genes (DNP63A, GRHL2, TFAP2A, c-MYC, and LEF1) or the four genes (DNP63A, GRHL2, TFAP2A, and LEF1), followed by transducing genes that induce mesenchymal cells with hair follicle-inducing ability **as listed in Table 2.** By performing gene transduction on a skin ulcer isolated from the surrounding skin, it is suggested that skin with skin appendages can be induced in vivo.

**[Table 2]**

| First induced mesenchymal cells | | | | | Second induced mesenchymal cells | | |
|---|---|---|---|---|---|---|---|
| SHH | LEF1 | | | | ETV1 | FOXD1 | PRDM1 |
| SHH | | | | | ETV1 | FOXD1 | PRDM1 |
| SHH | LEF1 | | | | ETV1 | FOXD1 | |
| SHH | LEF1 | | | | | FOXD1 | PRDM1 |
| SHH | LEF1 | | | | ETV1 | | PRDM1 |
| SHH | | ETV1 | FOXD1 | PRDM1 | | | |
| SHH | LEF1 | ETV1 | FOXD1 | PRDM1 | | | |
| SHH | LEF1 | | | | | | |
| SHH | LEF1 | | | | | | PRDM1 |
| SHH | | | | | | FOXD1 | PRDM1 |
| SHH | | | | | | | PRDM1 |
| SHH | | | | | | FOXD1 | |
| SHH | | | | | | | |
| | | ETV1 | FOXD1 | PRDM1 | | | |

In Examples 7 and 8, skin and skin appendages were induced in a skin ulcer isolated from the surrounding skin, where physiological regeneration of even the surrounding skin and epidermal tissue does not occur. This approach was taken to clearly demonstrate that the induced skin appendages were a result of the effects of the present invention. To induce cells with skin appendage-inducing ability, particularly epithelial cells, DNP63A, GRHL2, TFAP2A, c-MYC, and LEF1 were transduced. However, when applying the present invention to conditions associated with alopecia, sebaceous deficiency, and age-related changes that accompany these conditions, the treatment target area contains existing skin and epidermal tissues. Therefore, it is suggested that the transduction of all five genes (DNP63A, GRHL2, TFAP2A, c-MYC, and LEF1) or four genes (DNP63A, GRHL2, TFAP2A, and LEF1) may not be necessary. As confirmed in Non-Patent Document 2, DNP63A, GRHL2, TFAP2A, and c-MYC function to induce epidermal cells from mesenchymal cells. Thus, when applying the present invention to alopecia, sebaceous deficiency, and age-related changes that accompany these conditions, where skin and epidermal tissues are already present in the treatment target area, it is suggested that LEF1 alone or LEF1 in combination with a subset of DNP63A, GRHL2, TFAP2A, and c-MYC may be sufficient to induce epithelial cells with skin appendage-inducing ability from the pre-existing skin and epidermal tissues in the treatment area.

That is, when applying the present invention to conditions involving a decline in skin appendage function, such as alopecia, sebaceous deficiency, and age-related changes that accompany these conditions, where skin and epidermal tissues are present in the treatment area, it is suggested that transduction of LEF1 alone, or LEF1 in combination with a subset of DNP63A, GRHL2, TFAP2A, and c-MYC, as well as one or two groups of genes listed in Table 2, may improve conditions characterized by a quantitative and functional decline in skin appendage function.

### 13. Applications of Methods for Enhancing In Vivo Gene Transduction Efficiency

The method for enhancing in vivo gene transduction efficiency in the present invention can be used not only as a therapeutic approach for disease treatment involving gene transduction but also as a research and development tool for gene therapy methods. Furthermore, it can be utilized as a research tool for drug discovery and biological studies beyond the development of gene therapy methods using gene transduction. Various methods have been employed for in vivo gene transduction, including viral vectors such as AAV, retroviral vectors, lentiviral vectors, adenoviral vectors, and Sendai viral vectors; naked DNA such as plasmid DNA and mini-circle DNA; and, more recently, mRNA, including those carried by lipid nanoparticles (LNPs). However, achieving high-efficiency gene transduction has often been challenging. Since the method for enhancing in vivo gene transduction efficiency in the present invention achieves a higher gene transduction efficiency than conventional methods, it is applicable not only to the skin, skin ulcers, limb tissues, and muscle tissues described in the examples but also to the central nervous system, ocular nerves, the brain, the eye, the retina, and other sensory organs, as well as intraperitoneal and intrathoracic organs. This method can be applied as a gene therapy approach for all organs, tissues, and cells throughout the body, as well as a research and development tool for gene therapy methods and a research tool for drug discovery and biological studies beyond the development of gene therapy methods using gene transduction.

### 14. Method for Enhancing In Vivo Gene Transduction Efficiency

The method for enhancing in vivo gene transduction efficiency in the present invention provides a technique to achieve high gene transduction efficiency and increased gene expression by administering steroids before, simultaneously with, or shortly after in vivo gene transduction, either systemically or locally. Systemic administration is performed using methods commonly employed for systemic steroid administration, such as intravenous injection, intradermal injection, subcutaneous injection, intramuscular injection, and oral administration. Local administration is performed using methods commonly employed for local steroid administration, such as intradermal injection, subcutaneous injection, intramuscular injection, intra-articular injection, topical application to the skin, intraluminal spraying, eye drops, ear drops, and nasal drops. Additionally, direct administration to target areas for gene transduction is also included, such as administration to the brain parenchyma in cases of intracranial or brain parenchymal gene transduction, including injection, local application during surgery, or endoscopic-assisted administration, even though such methods are not conventionally used in steroid therapy. Steroid formulations that can be administered include adrenocortical hormones and all formulations with steroid hormone activity, including methylprednisolone, methylprednisolone sodium succinate, methylprednisolone acetate, hydrocortisone, hydrocortisone sodium phosphate, hydrocortisone sodium succinate, hydrocortisone butyrate, hydrocortisone butyrate propionate, prednisolone, prednisolone acetate, prednisolone sodium phosphate, prednisolone sodium succinate, prednisolone valerate acetate, clobetasone butyrate, fluorometholone, triamcinolone, triamcinolone acetonide, alclometasone dipropionate, dexamethasone, dexamethasone sodium phosphate, dexamethasone metasulfobenzoate sodium, dexamethasone palmitate, dexamethasone propionate, dexamethasone valerate, dexamethasone cipate, betamethasone, betamethasone sodium phosphate, betamethasone valerate, betamethasone dipropionate, betamethasone butyrate propionate, fluocinolone acetonide, diflucortolone valerate, fludroxycortide, fluocinonide, budesonide, diflorasone acetate, amcinonide, mometasone furoate, mometasone furoate monohydrate, beclomethasone dipropionate, fluticasone propionate, fluticasone furoate, difluprednate, clobetasol propionate, cortisone acetate, ciclesonide, deprodone propionate, fludrocortisone acetate, and halobetasol propionate. The method of administering steroid formulations (including dosage, administration frequency, timing, and duration) should be optimized based on the specific formulation used, the target organ, tissue, or cells for gene transduction, and the intended effect of gene transduction. The present invention encompasses all administration methods that enhance gene transduction efficiency and gene expression by combining steroids with in vivo gene transduction.

### 15. Experimental Results 5

The present invention will be described in detail below based on examples and the like; however, the invention is not limited to these.

### [Example 9]

Figure 10 presents a schematic of an experiment evaluating the effect of steroids on the gene transduction efficiency of AAV in skin ulcers. Figure 11 shows stereomicroscopic observations of the ulcerated area under visible light and fluorescence on the seventh day after AAV administration. Figure 12 depicts histological observations on the seventh day after AAV administration in the same experiment. To investigate the effect of steroid administration on gene transduction efficiency during AAV-mediated gene transduction into skin ulcers, a model animal was prepared by creating a skin ulcer on the dorsal side of a mouse and attaching a silicone chamber. The silicone chamber was fabricated and attached following the method described in Du Z, Shen Q, Mito D, Kato M, Okazaki M, Kurita M. Optimized 3D-printed template design for production of silicone skin chambers. J Dermatol Sci. 2022 Jan;105(1):55-57. At the time of silicone chamber attachment, the experimental groups were categorized as follows: a control group without steroid administration, a remote administration group in which 10 µl of Kenacort A (40 mg/ml) was subcutaneously injected into the abdominal region, and a local administration group in which 10 µl of Kenacort was applied into the chamber. The AAV vector used in this experiment was an AAVDJ viral vector expressing GFP under the control of the CAG promoter, which was ordered and purchased from VectorBuilder. On the day following chamber attachment and steroid administration, 100 µl of AAVDJ-CAG-GFP virus containing a titer of 10¹¹ GC was applied inside the chamber of each animal. On the seventh day after AAV administration, fluorescence stereomicroscopy (Axio Zoom .V16, Zeiss) was used to observe the ulcerated area. Stronger GFP expression was observed in the remote administration group compared to both the control group (no steroid administration) and the local administration group. Subsequently, tissues from the ulcerated area were collected, fixed overnight in 4% paraformaldehyde-phosphate buffered solution (pH 7.4), then replaced with 30% sucrose/phosphate-buffered saline (PBS), embedded in OCT compound, and frozen. Using Kawamoto's film method (SECTION-LAB, Cryofilm type 2C(9), 2.5 cm C-FP094), 10 µm thick frozen sections were collected at 200 µm intervals. The sections were washed with PBS and stained with DAPI for nuclear staining. Adjacent sections were prepared and stained with hematoxylin-eosin (HE). The images were scanned and digitized using a slide scanner (VS200, Olympus). Histological analysis revealed a higher number of GFP-positive cells in the remote administration group. To quantitatively compare the histological findings across the groups, the number of GFP-positive cells in enlarged images of the central ulcer region was counted. Based on adjacent HE-stained sections, the tissue was classified into three regions: the superficial layer (above the fascia of the subcutaneous adipose tissue), the fat layer (subcutaneous adipose tissue), and the muscle layer (muscles such as the trapezius). The number of GFP-positive cells in each region was counted. For each animal and tissue sample, five sections were analyzed, and the average value was calculated. The differences in the mean values among the three animals in each group were statistically analyzed using a t-test. The total number of GFP-positive cells in the remote administration group was significantly higher than that in the control group without steroid administration. In particular, in the deeper tissues (fat layer + muscle layer), the remote administration group exhibited a significantly higher number of GFP-positive cells compared to both the control group and the local administration group.

### [Example 10]

Figure 13 presents the results of an experiment evaluating the effect of different concentrations of steroids on the gene transduction efficiency of AAV in skin ulcers. The experimental groups included a control group without steroid administration, a remote administration group in which 1 µl or 10 µl of Kenacort A (40 mg/ml) was subcutaneously injected into the abdominal region, and a local administration group in which 1 µl or 10 µl of Kenacort was applied inside the chamber. Except for these modifications, the experiment was conducted following the same procedures as in Example 9, up to the histological observations. Histological analysis revealed that in the remote administration group, a higher number of GFP-positive cells were observed with the 10 µl dose, whereas in the local administration group, a higher number of GFP-positive cells were observed with the 1 µl dose. Particularly in inflamed areas such as skin ulcers, where steroids exert anti-inflammatory effects, it is anticipated that the concentration of steroids may influence the number of cells present. These findings suggest that to optimize gene transduction efficiency, it is necessary to determine the appropriate dosage based on the method of administration, such as systemic or local.

### [Example 11]

Figure 14 presents the results of an experiment evaluating the effect of steroid administration on the gene transduction efficiency of AAV in the dorsal skin of mice. The experimental groups included a control group without steroid administration and a remote administration group in which 10 µl of Kenacort A (40 mg/ml) was subcutaneously injected into the abdominal region. As the AAV vector, an AAVDJK2 capsid-based AAV expressing GFPNLS under the control of the K14SCP3 promoter was used. One day after steroid administration in the remote administration group, the dorsal skin of each mouse was shaved, and 35 µl of AAVDJK2-K14SCP3-GFPNLS containing a titer of 10¹¹ GC was intradermally injected. On the second day after AAV injection, the injection site was observed using fluorescence stereomicroscopy (Axio Zoom .V16, Zeiss), and tissues, including the surrounding skin, were collected. The tissues were fixed overnight in 4% paraformaldehyde-phosphate buffered solution (pH 7.4), then replaced with 30% sucrose/phosphate-buffered saline (PBS), embedded in OCT compound, and frozen. Using Kawamoto's film method (SECTION-LAB, Cryofilm type 2C(9), 2.5 cm C-FP094), 10 µm thick frozen sections were collected at 200 µm intervals. The sections were washed with PBS and stained with DAPI for nuclear staining. Adjacent sections were prepared and stained with hematoxylin-eosin (HE). The images were scanned and digitized using a slide scanner (VS200, Olympus). Histological analysis revealed that in the systemic steroid administration group, a high efficiency and continuous distribution of GFPNLS-positive cells were observed, primarily in the epidermal tissue. In contrast, in the control group without steroid administration, GFPNLS-positive cells were observed at low frequency and scattered throughout the tissue. These findings indicate that steroid administration enhances the gene transduction efficiency of AAV in the skin.

### [Example 12]

Figure 15 presents the results of an experiment evaluating the effect of different concentrations of steroids on the gene transduction efficiency of AAV in the skin. The experimental groups included remote administration groups in which 10 µl, 0.1 µl, or 0.01 µl of Kenacort A (40 mg/ml) was subcutaneously injected into the abdominal region. Except for these modifications, the experiment was conducted following the same procedures as in Example 11. Histological analysis revealed that in animals that received remote administration of 10 µl or 0.1 µl of Kenacort A, GFPNLS-positive cells were predominantly observed in the epidermal tissue. In contrast, in animals that received 0.01 µl of Kenacort A, histological findings were largely similar to those of animals without steroid administration. These results indicate that as the amount of steroid decreases (0.01 µl), the enhancing effect of steroids on gene transduction efficiency is reduced.

### [Example 13]

Figure 16 illustrates the surgical procedure for repositioning the left upper limb to the dorsal side prior to amputation in mice. The purpose of this procedure is to prevent self-inflicted trauma to the stump and to maintain local stability as much as possible. The sequential steps of the surgical technique are shown from top to bottom in the left, middle, and right columns. After inducing inhalation anesthesia with isoflurane, a traction suture is placed on the left hand to position the upper limb laterally, securing the surgical limb position. A vertical skin incision is made on the anterior axillary region, and the muscle mass on the ventral side of the shoulder joint is dissected using bipolar electrocautery to expose the joint capsule. The joint capsule is entered from the ventral side, and the dissection is extended in the superior-inferior direction to expose the scapular stump. The scapula is grasped and, while applying traction, the surrounding muscles are dissected to excise the scapula. After severing major muscles such as the teres major, which connects the humerus to the chest wall, the dorsal skin incision is extended to the base of the upper limb. The mouse is then repositioned into a right lateral decubitus position, and the muscles connecting the humerus to the chest wall are severed, completing the separation of the upper limb. Throughout the entire dissection process, the brachial plexus and major arteries and veins supplying the upper limb are preserved. The released upper limb is repositioned dorsally, and while securing part of the ventral muscles, including the deltoid, to the chest wall, skin suturing is performed. During suturing, excess skin is trimmed as necessary. The wound is typically closed with 4-6 sutures. Through this procedure, the left upper limb is successfully repositioned to the dorsal side while preserving its primary nerves and blood vessels.

In this example, an upper limb amputation model was created in mice. However, an upper limb amputation model can also be established in non-human mammals by repositioning the upper limb and performing amputation at the upper arm, forearm, hand, or finger level, or by repositioning the upper limb and amputating at the forearm level. It is preferable that the upper limb amputation model be created while preserving the major nerves, arteries, and veins of the upper limb. The repositioning of the upper limb may be performed at the shoulder joint or may involve the removal of the scapula. By applying a test substance to such an upper limb amputation model and evaluating its effects on the amputation site, a screening method for pharmaceutical substances can be conducted. The pharmaceutical substance may be a gene delivery vector intended for gene transduction or a material used for the regeneration of amputated tissues. Examples of non-human mammals that can be used for this model include rabbits, dogs, cats, guinea pigs, hamsters, rats, and mice.

### [Example 14]

Figure 17 presents the results of an experiment evaluating the effect of steroid administration on the gene transduction efficiency of AAV and retrovirus at the amputation site of the mouse forearm. In this experiment, the left upper limb was repositioned dorsally using the surgical technique described in Example 13, followed by amputation at the forearm level. After tissue collection from the forearm, the samples were fixed overnight in 4% paraformaldehyde-phosphate buffered solution (pH 7.4), embedded in OCT compound, and frozen. Using Kawamoto's film method (SECTION-LAB, Cryofilm type 2C(9), 2.5 cm C-FP094), 10 µm thick frozen sections were collected at 200 µm intervals. The sections were either stained with hematoxylin-eosin (HE) or, for adjacent sections, washed with phosphate-buffered saline (PBS) and stained with DAPI for nuclear staining. The images were scanned and digitized using a slide scanner (VS200, Olympus). Histological analysis of the forearm amputation tissue revealed that the central region contained bone, surrounded by muscle, with skin and subcutaneous fat covering the outer layer. For AAV-mediated gene transduction, an AAVDJ capsid-based AAV expressing GFPNLS under the control of the CAG promoter was prepared using the concentrated AAV production method described in Example 2. The experimental groups included a control group without steroid administration, a remote administration group in which 10 µl of Kenacort A (40 mg/ml) was subcutaneously injected into the abdominal region, and a local administration group in which 5 µl of Kenacort A (40 mg/ml) was injected into the amputation stump. One day after repositioning the left upper limb dorsally, the forearm was amputated. Steroids were administered according to the designated conditions one hour after amputation. The following day, each animal received 9 µl of AAVDJ-CAG-GFPNLS containing a titer of 10¹¹ GC, administered in three separate 3 µl injections at 2-hour intervals into the amputation site. One week after AAV administration, tissues were collected for analysis. Fluorescence imaging of the tissue revealed that both local and systemic steroid administration increased the number of GFPNLS-positive cells in the amputated tissue. The use of steroids significantly enhanced gene transduction efficiency across a wide range of tissues and cells constituting the amputated limb stump, including the subcutaneous tissue, fascia, muscle, and periosteum when AAV was used for gene delivery. For retroviral gene transduction, the PMXs retroviral backbone plasmid coding for GFPNLS, along with packaging plasmids (pCMV-gagpol-PA, pCMV-VSVg), was transfected into 293FT cells (Thermo Fisher Scientific) using Lipofectamine 2000 (Thermo Fisher Scientific). The culture medium was replaced, and the collected supernatant containing the retrovirus was used as the retroviral solution, following standard procedures (refer to Non-Patent Document 2). In this experiment, 2 ml of retroviral solution was concentrated using polyethylene glycol (PEG) precipitation to obtain 100 µl of a concentrated viral solution per animal. One day after repositioning the left upper limb dorsally, the forearm was amputated. One hour after amputation, steroids were administered along with the first injection of the viral solution. In the remote administration group, 10 µl of Kenacort A (40 mg/ml) was subcutaneously injected into the abdominal region. In the local administration group, 5 µl of Kenacort A (40 mg/ml) was mixed with the viral solution before injection. The retroviral solution was injected ten times per animal over two days at 3-hour intervals. One week after the final viral injection, tissues were collected. Fluorescence imaging of the tissue revealed that both local and systemic steroid administration increased the number of GFPNLS-positive cells in the amputated tissue. The use of steroids significantly enhanced gene transduction efficiency across the various tissues and cells constituting the amputated limb stump when retroviral gene delivery was performed.

### [Example 15]

Figure 18 presents the results of an experiment evaluating the effect of steroid administration on the gene transduction efficiency of mRNA in the anterior thigh muscle of mice. The mRNA used in this experiment was eGFP mRNA, which was custom-manufactured by Arcalis Co., Ltd. Following the method described in the product manual, Lipofectamine 2000 transfection reagent was used to prepare the transfection complex. The complex was prepared using 1 µg of mRNA, 1.2 µl of Lipofectamine, and 100 µl of Opti-MEM medium, of which 50 µl per muscle was used as the mRNA transfection reagent. Nude mice (BALB/cAJcl-nu/nu) were used in this study. The experimental groups included a control group without steroid administration and a remote administration group, in which 10 µl of Kenacort A (40 mg/ml) was subcutaneously injected into the abdominal region one day before mRNA transfection. The mRNA transfection reagent was prepared immediately before injection and injected 5-15 minutes after preparation into the anterior thigh muscle. Twelve hours after injection, the animals were euthanized, and the muscles were excised. Fluorescence stereomicroscopy (Axio Zoom .V16, Zeiss) was used to observe the muscle surface, revealing strong green fluorescence signals in specific areas of the systemic steroid administration group. The tissues were then fixed overnight in 4% paraformaldehyde-phosphate buffered solution (pH 7.4), embedded in OCT compound, and frozen. Using Kawamoto's film method (SECTION-LAB, Cryofilm type 2C(9), 2.5 cm C-FP094), frozen tissue sections were collected perpendicular to the muscle fiber direction at 10 µm thickness, washed with phosphate-buffered saline (PBS), and stained with DAPI for nuclear staining. Adjacent sections were stained with hematoxylin-eosin (HE). The prepared tissue sections were digitized using a slide scanner (VS200, Olympus). In the systemic steroid administration group, strong GFP signals were observed in parts of the muscle, with signals spreading in a centrifugal pattern. In contrast, in the control group without steroid administration, only weak GFP signals were observed. These results indicate that steroid administration enhances gene transduction efficiency even for mRNA-based in vivo gene delivery into tissues.

### [Example 16]

Figure 19 presents the results of an experiment evaluating the effect of steroid administration on the gene transduction efficiency of AAV in in vitro cultured cells. 293AAV cells (purchased from Cell Biolabs) were used as the cultured cells. The AAV vector used was an AAVDJ viral vector expressing GFP under the control of the CAG promoter, which was ordered and purchased from VectorBuilder. 293AAV cells were cultured in DMEM medium supplemented with 10% fetal bovine serum (FBS) following standard protocols. 1.5 × 10⁵ cells were seeded per well in a 24-well plate. On day 1, the medium was replaced with medium containing Kenacort A at concentrations of 1 µg/ml, 10 µg/ml, 100 µg/ml, or 1 mg/ml. On day 2, half of the medium was replaced with medium containing AAV at a concentration of 10¹⁰ GC/well. On day 3, the medium was completely replaced with fresh medium containing neither steroids nor AAV. On day 6, fluorescence microscopy (IX73, Olympus) was used for phase-contrast and fluorescence imaging. GFP-positive cells were observed under green fluorescence imaging, while non-specific fluorescence expression was detected under red fluorescence imaging. Cells cultured in the presence of 1 µg/ml and 10 µg/ml steroids exhibited a higher number of GFP-positive cells compared to those without steroids. In contrast, at 100 µg/ml and 1 mg/ml steroid concentrations, the number of cells and GFP-positive cells decreased due to the effects of steroids on cell viability. These findings indicate that AAV-mediated gene transduction efficiency in in vitro cultured cells can be enhanced in the presence of an appropriate concentration of steroids.

### [Example 17]

Figure 20 presents the results of an experiment evaluating the effect of steroid administration on the dynamics of subcutaneously injected fluorescent silica particles. The experimental groups included a control group without steroid administration and a remote administration group, in which 10 µl of Kenacort A (40 mg/ml) was subcutaneously injected into the abdominal region. On the day following steroid administration, the dorsal region of the mice was widely shaved, and 30 µl of red fluorescent silica particles (sicastar^{®}-redF, plain, 30 nm, 25 mg/ml), which have a similar size to AAV, was injected subcutaneously. Immediately after injection, as well as at 1, 3, 6, 9, 12, 18, and 24 hours post-injection, the injection site was observed using fluorescence stereomicroscopy (Axio Zoom .V16, Zeiss) to analyze the effect of steroid administration on the local retention of fluorescent silica particles. Time-lapse fluorescence microscopy images were taken using a fixed exposure time (30 msec), and the red fluorescence signals in the images were compared. In the control group without steroids, a rapid decrease in fluorescence intensity was observed early on. In contrast, in the systemic steroid administration group, strong fluorescence signals persisted for a longer duration. Additionally, when images taken with a longer exposure time at 24 hours post-injection were compared, weaker fluorescence signals were found to be more widely distributed in the control group, whereas in the systemic steroid administration group, fluorescence signals remained stronger and more localized. For a more detailed evaluation of the diffusion of fluorescent silica particles within the tissue, skin tissue was collected 24 hours post-injection and immediately embedded in OCT compound and frozen. Using Kawamoto's film method (SECTION-LAB, Cryofilm type 2C(9), 2.5 cm C-FP094), 10 µm thick frozen sections were collected, washed with phosphate-buffered saline (PBS), and stained with DAPI for nuclear staining. Adjacent sections were stained with hematoxylin-eosin (HE). The tissue sections were digitized using a slide scanner (VS200, Olympus). Histological analysis revealed that in the systemic steroid administration group, strong fluorescence signals were observed in a more localized area, whereas in the control group, weaker fluorescence signals were spread over a wider area. These findings indicate that in steroid-administered animals, the diffusion of silica particles into surrounding tissues was suppressed, resulting in stronger local retention compared to animals without steroid administration.

The present invention enables high gene transduction efficiency and high gene expression by administering systemic or local steroids prior to, simultaneously with, or shortly after in vivo gene delivery. In gene therapy using viral vectors, steroids have been used to suppress pre-existing immune responses to viral vectors and immune reactions triggered by repeated treatments, thereby enhancing gene transduction efficiency (see Yanda MK, Tomar V, Cebotaru CV, Guggino WB, Cebotaru L. Short-Term Steroid Treatment of Rhesus Macaque Increases Transduction. Hum Gene Ther. 2022 Feb;33(3-4):131-147). However, as demonstrated in Examples 9, 10, 11, 12, 14, and 15, the effects of the present invention were observed at the initial gene transduction, indicating that immune suppression in repeated treatments is not the primary mechanism. Additionally, under laboratory animal housing conditions, it is unlikely that AAV would non-specifically infect the animals, leading to pre-existing immune responses to AAV. Therefore, the possibility that the present invention functions solely through the suppression of pre-existing immune responses, as previously reported, is low. Furthermore, in Example 14, which used retroviral vectors, and in Example 15, which involved mRNA-based gene delivery, there was no potential for immune responses to be triggered by exogenous retroviruses or exogenous mRNA administration. These findings indicate that steroid administration in the present invention differs significantly from conventional steroid usage methods, highlighting its high level of novelty.

As demonstrated in Example 16, the enhancement of gene transduction efficiency and the achievement of high gene expression due to steroid action were also observed in cultured cells. This suggests that the effect is not solely attributed to systemic immune modulation, but rather to a direct cellular-level effect of steroids. It is possible that steroid administration suppresses the cGAS-STING pathway, which is an intracellular innate immune response induced by exogenous gene transduction.

As demonstrated in Example 17, steroid administration increased the local retention of subcutaneously injected fluorescent silica particles and reduced clearance from the injection site. Since steroids are known to affect fluid circulation in peripheral tissues, as evidenced by their ability to reduce inflammatory edema, it is suggested that steroid administration enhances gene transduction efficiency by increasing the local retention time of gene delivery vectors administered peripherally, reducing clearance from the local site, increasing the exposure of gene delivery vectors to target cells, and extending the duration of gene vector in action.

Steroids are biologically active substances with a wide range of effects at both the cellular and organismal levels. Including their characteristic anti-inflammatory and immunosuppressive actions at the organismal level, which may enhance in vivo gene transduction efficiency by suppressing biological responses that would otherwise attenuate the effects of gene delivery vectors, the present findings suggest that the mechanisms by which systemic or local steroid administration-prior to, simultaneously with, or shortly after gene transduction-enhances gene transduction efficiency and gene expression are multifaceted.

### 16. Applications of Methods for Regenerating Tissues at Amputated Limb Sites

In mammals, wound healing occurs at the stump of an amputated limb. Specifically, the bone stump, muscle stump, and skin/subcutaneous tissue undergo primary wound healing or secondary wound healing, leading to scar formation rather than the regeneration of functional tissue. In contrast, the present invention enables the functional regeneration of tissue at mammalian limb amputation sites through interventions such as gene transduction, protein administration, compound administration, or cell therapy. As a result, it provides a medical (therapeutic) application, as well as biological and tissue regeneration technologies, and serves as a model that contributes to future technological advancements. Mammals applicable to this method include humans, primates, rats, and mice, with mice being particularly preferred as a research and development model.

In the present invention, "tissue regeneration at amputated limb sites in mammals" refers to the regeneration of tissues that cannot be restored through primary wound healing or secondary wound healing, which physiologically occur at the bone stump, muscle stump, and skin/subcutaneous tissue after limb amputation. It refers to the neogenesis, proliferative growth, and regeneration of various tissues included in peripheral limb tissues, including but not limited to the formation and proliferative growth of adipose tissue beyond physiological levels, the formation and proliferative growth of bone tissue, the formation and proliferative growth of cartilage tissue, and the formation and proliferative growth of muscle tissue, as well as the regeneration of limb-like structures.

In the present invention, "a model for developing methods to induce tissue regeneration at amputated limb sites in mammals" refers to a research and development model that can be used for medical (therapeutic) research and development involving gene therapy, protein or compound administration, as well as a methodology for drug discovery and biological research.

### 17. Method for Regenerating Tissues at Amputated Limb Sites

The present invention relates to a method for inducing tissue regeneration at amputated limb sites in mammals and a model for developing such methods. It comprises an animal model (including experimental surgical techniques for model creation and amputation methods), transduced genes, administered proteins, administered compounds, bioactive substances used as auxiliaries, and methods for gene transduction and substance administration.

In the present invention, a mouse forearm amputation model was developed as an animal model for investigating the effects of gene transduction, protein administration, and compound administration on tissue regeneration at amputated limb stumps in mammals. Using the surgical procedure described in Example 13, upper limb amputation was performed in mice, allowing for the avoidance of self-inflicted trauma to the amputation site and the maximization of local stability.

Figure 21 illustrates a schematic of the stump treatment method for forearm amputation in nude mice. The forearm is amputated proximal to a skin structure on the surface of the forearm that exhibits a mild protrusion and erythema. The amputation site may be treated by a simple transverse amputation, transverse amputation followed by excision of the surrounding skin at the stump, or transverse amputation while preserving a longer portion of the nerve. After amputation, the wound surface may be managed as an open wound, manually compressed using the surrounding skin, or closed with sutures. If excessive bleeding occurs from the stump, a silicone cap can be applied to achieve hemostasis.

The surgical procedure described in Example 13 and the stump treatment method illustrated as a schematic in Figure 21 provide a research and development platform for developing methods for regenerating amputated limb sites in mammals.

The method of inducing tissue regeneration at amputated limb sites in mammals in the present invention is characterized by the transduction or application of genes and proteins that are relatively highly expressed in cells possessing tissue regenerative capacity at the distal portions of amputated limbs (e.g., cells present in mammalian fetal limb buds and lateral plate mesoderm, as well as cells from amphibians with intrinsically higher regenerative capacity, such as the Mexican axolotl *(Ambystoma mexicanum),* the Japanese fire-bellied newt *(Cynops pyrrhogaster),* and the African clawed frog *(Xenopus laevis)),* or that function as master regulator genes during the differentiation and developmental processes of these cells. Additionally, the method involves the application of cytokines and hormones that are highly active in environments where tissue regeneration occurs (e.g., mammalian fetal limb buds and lateral plate mesoderm, as well as the regenerating tissues of amputated limbs in amphibians with intrinsically higher regenerative capacity, such as the Mexican axolotl *(Ambystoma mexicanum),* the Japanese fire-bellied newt *(Cynops pyrrhogaster),* and the African clawed frog *(Xenopus laevis)),* and compounds with equivalent or similar functions. These genes, proteins, cytokines, hormones, and compounds are transduced or applied to somatic cells at the amputation site that lack intrinsic regenerative capacity and do not undergo physiological tissue regeneration (including blood-derived cells that migrate locally in response to amputation). Hereinafter, transduced genes and transduced proteins are referred to as 'transduced genes' and 'transduced proteins,' respectively. Proteins applied to exert an effect are referred to as 'applied proteins,' and compounds applied to exert an effect are referred to as 'applied compounds.'

Transduced genes, transduced proteins, applied proteins, and applied compounds may be used in parallel with multiple types. They may also be combined with genes and proteins that are not relatively highly expressed in cells possessing tissue regenerative capacity, or that do not function as master regulator genes during the differentiation and developmental processes of these cells, as well as cytokines and hormones that are not highly active in environments where tissue regeneration occurs, and compounds that do not possess equivalent or similar functions to these factors.

The genes and proteins that are relatively highly expressed in cells possessing tissue regenerative capacity or function as master regulator genes during the differentiation and developmental processes of these cells, as well as the cytokines and hormones that are highly active in environments where tissue regeneration occurs in the present invention, include at least the genes listed in Table 3 and the proteins encoded by these genes.

**[Table 3]**

| | | | | |
|---|---|---|---|---|
| BMP2 | HMGB3 | Sp8 | NR2C2 | WNT3A |
| BMP4 | HMGN1 | SP9 | TBX15 | WNT5A |
| BMP5 | HMGN2 | STMN1 | BCL11A | ZIC3 |
| BMP7 | HOXA10 | TRIM69 | SMARCA1 | POU5F1 |
| BMP9 | HOXA9 | XB5727893 | SMARCA4 | SOX2 |
| FGF2 | HSPA8 | ZNF326 | CIRBP | KLF4 |
| FGF4 | LEF1 | TBX5 | LIN28A | cMYC |
| FGF8 | MARCKSL1 | TCF7L2 | HOXB9 | PBX1 |
| FGF10 | MEIS1 | NFIB | SALL4 | |
| FGF20 | MSX1 | SHOX2 | FOXD1 | |
| SHH | MSX2 | ZNF449 | PRDM1 | |
| RSPO2 | MYCN | EGR1 | EGR3 | |
| BRCA1 | nAG | HDAC1 | WNT7A | |
| DNMT1 | PRDX1 | POLE4 | IL6 | |
| E2F8 | PRDX2 | ATF1 | FOXA1 | |
| ETV2 | Prod1 | PPARD | FOXA2 | |
| GTF2F1 | PRRX1 | TIMELESS | MBD4 | |
| HAND2 | SAP30 | TFDP1 | NR2F2 | |
| HDAC2 | SMC3 | TFDP2 | PA2G4 | |
| HMGB2 | SNRPF | KAZALDI1 | SPDEF | |

Many of these genes are commonly found across mammals, including humans, and any mammalian-derived version may be used. However, it is preferable to select an appropriate version based on the origin of the somatic cells to be transduced. For example, if the target is human application, the transduced genes and proteins should preferably be of human origin. Additionally, the transduced genes and proteins may not only be wild-type but also mutant genes encoding mutant gene products that have equivalent functions to the wild-type gene product. These mutant genes may contain a few (e.g., 1-10, preferably 1-6, more preferably 1-4, even more preferably 1-3, and most preferably 1 or 2) amino acid substitutions, deletions, and/or insertions in their amino acid sequence. Codon-modified or codon-optimized sequences that encode the same amino acids as those encoded by each gene may also be used.

In the present invention, the transduced genes can be prepared by conventional methods based on publicly available sequence information. For example, RNA can be extracted from mammalian-derived cells, and cDNA of the target gene can be prepared by cloning according to standard methods. Additionally, the genes can be synthesized as artificial genes. During artificial gene synthesis, codon optimization can be performed according to the species of origin of the somatic cells to be transduced.

### 18. Experimental Results 6

The present invention will be described in detail below based on examples and the like; however, the invention is not limited to these.

### [Example 18]

Figure 22 presents the results of an experiment in which the left upper limb of a nude mouse was repositioned dorsally, followed by a transverse amputation at the forearm, leaving the cross-section as an open wound. Four- to five-week-old BALB/cAJcl-nu/nu mice (obtained from Central Institute for Experimental Animals) were used. On the fifth day after dorsal repositioning of the left upper limb using the surgical technique described in Example 13, a transverse amputation was performed proximal to a skin structure on the forearm surface that exhibited a mild protrusion and erythema, and the amputation site was left as an open wound. Observations were conducted weekly under a surgical microscope (MM100-YOH, Mitaka Kohki Co., Ltd.), and photographs were taken until the 70th day post-amputation. After collection of forearm tissue, the samples were fixed overnight in 4% paraformaldehyde-phosphate buffered solution (pH 7.4), embedded in OCT compound, and frozen. Using Kawamoto's film method (SECTION-LAB, Cryofilm type 2C(9), 2.5 cm C-FP094), 10 µm thick frozen sections were collected at 200 µm intervals, washed with phosphate-buffered saline (PBS), and stained with hematoxylin-eosin (HE). The prepared slides were scanned and digitized using a slide scanner (VS200, Olympus). Histological analysis showed that the bone stump was sealed with cortical bone, and soft tissues healed through contraction and closure of the surrounding skin. No evident tissue regeneration was observed at the amputated limb site.

### [Example 19]

Figure 23 presents the results of an experiment in which the left upper limb of a nude mouse was repositioned dorsally, followed by a transverse amputation at the forearm, leaving the cross-section as an open wound, and performing gene transduction. Gene transduction was conducted using retroviruses to transduce POLE4, NFIB, PPARD, FGF10, FGF20, and FGF2, AAVDJ1 to transduce PRRX1 and HDAC2, and AAVDJ to transduce LEF1 and SHH. The retroviruses were prepared following standard methods using PMXs retroviral backbone plasmids encoding each gene and packaging plasmids (pCMV-gagpol-PA, pCMV-VSVg). These plasmids were transfected into 293FT cells (Thermo Fisher Scientific) using Lipofectamine 2000 (Thermo Fisher Scientific). After medium replacement, the cell culture supernatant was collected and used as the retroviral solution. For each animal, 2 ml of retroviral solution was used for POLE4, NFIB, and PPARD, and 1 ml of retroviral solution was used for FGF10, FGF20, and FGF2. These solutions were concentrated using polyethylene glycol (PEG) precipitation to obtain a total of 500 µl of concentrated virus solution. For AAV, AAV vectors expressing each gene under the CAG promoter were prepared and used following the AAV concentration method described in Example 2, with each vector having the designated capsid. Four- to five-week-old BALB/cAJcl-nu/nu mice (obtained from the Central Institute for Experimental Animals) underwent dorsal repositioning of the left upper limb using the surgical technique described in Example 13. On the fifth day after repositioning, the amputation level was set proximal to a skin structure on the forearm surface that exhibited a mild protrusion and erythema. At the planned amputation site, the first retroviral injection was administered before performing a transverse amputation, leaving the cross-section as an open wound. The retrovirus was locally injected ten times between the initial injection just before amputation and the fifth day post-amputation. AAVDJ-SHH was locally injected once immediately after amputation at a dose of 2.5 × 10¹¹ GC/8 µl. For AAVDJ-LEF1, AAVDJ1-PRRX1, and AAVDJ1-HDAC2, 18 µl of viral solution containing 1.0 × 10¹² GC of each vector was applied. On the day of amputation, 2 µl was topically applied to the wound surface twice. This was followed by two injections on the next day and three injections on the second day, administered as localized injections. Photographs were taken weekly after amputation under a surgical microscope (MM100-YOH, Mitaka Kohki Co., Ltd.). On the 36th day post-amputation, photographs were taken before collecting forearm tissue. The samples were fixed overnight in 4% paraformaldehyde-phosphate buffered solution (pH 7.4), embedded in OCT compound, and frozen. Using Kawamoto's film method (SECTION-LAB, Cryofilm type 2C(9), 2.5 cm C-FP094), 10 µm thick frozen sections were collected at 200 µm intervals, stained with hematoxylin-eosin (HE), and scanned into image files using a slide scanner (VS200, Olympus). Histological analysis revealed the formation of a soft tissue mass resembling a blastema, similar to those observed at the amputated limb stumps of amphibians with intrinsically high tissue regeneration capacity, such as the Mexican axolotl *(Ambystoma mexicanum),* the Japanese fire-bellied newt *(Cynops pyrrhogaster),* and the African clawed frog *(Xenopus laevis).* Additionally, axial elongation of bone was observed extending into the tissue.

### [Example 20]

Figure 24 presents the results of an experiment in which the left upper limb of a nude mouse was repositioned dorsally, followed by a transverse amputation at the forearm, leaving the cross-section as an open wound, and performing gene transduction. Gene transduction was conducted using retroviruses to transduce PRDX2, POLE4, NFIB, PPARD, FGF10, FGF20, FGF2, BMP5, and SHH, AAVDJ1 to transduce PRRX1 and HDAC2, and AAVDJ to transduce LEF1. The retroviruses were prepared following standard methods using PMXs retroviral backbone plasmids encoding each gene and packaging plasmids (pCMV-gagpol-PA, pCMV-VSVg). These plasmids were transfected into 293FT cells (Thermo Fisher Scientific) using Lipofectamine 2000 (Thermo Fisher Scientific). After medium replacement, the cell culture supernatant was collected and used as the retroviral solution. For each animal, 1 ml of retroviral solution was used for PRDX2, POLE4, NFIB, PPARD, FGF10, FGF20, FGF2, and BMP5. These solutions were concentrated using polyethylene glycol (PEG) precipitation to obtain a total of 400 µl of concentrated virus solution. For AAV, AAV vectors expressing each gene under the CAG promoter were prepared and used following the AAV concentration method described in Example 2, with each vector having the designated capsid. Four- to five-week-old BALB/cAJcl-nu/nu mice (obtained from the Central Institute for Experimental Animals) underwent dorsal repositioning of the left upper limb using the surgical technique described in Example 13. On the second day after repositioning, the amputation level was set proximal to a skin structure on the forearm surface that exhibited a mild protrusion and erythema. At the planned amputation site, 100 µl of retroviral solution was initially injected. This was followed by the injection of 5 µl solutions containing 1.0 × 10¹¹ GC of AAVDJ-LEF1, AAVDJ1-PRRX1, and AAVDJ1-HDAC2, respectively. A transverse amputation was then performed, leaving the cross-section as an open wound, and a silicone cap was applied for hemostasis. On the following day, 5 µl solutions containing 1.0 × 10¹¹ GC of AAVDJ-LEF1, AAVDJ1-PRRX1, and AAVDJ1-HDAC2 were injected twice. From the second to the fifth day, retroviral solution was injected once daily into the distal stump. Photographs were taken weekly after amputation under a surgical microscope (MM100-YOH, Mitaka Kohki Co., Ltd.). On the 40th day post-amputation, photographs were taken before collecting forearm tissue. The samples were fixed overnight in 4% paraformaldehyde-phosphate buffered solution (pH 7.4), embedded in OCT compound, and frozen. Using Kawamoto's film method (SECTION-LAB, Cryofilm type 2C(9), 2.5 cm C-FP094), 10 µm thick frozen sections were collected at 200 µm intervals, stained with hematoxylin-eosin (HE), and scanned into image files using a slide scanner (VS200, Olympus). Histological analysis revealed the presence of a blastema-like soft tissue mass at the distal portion of the bone stump, along with newly formed bone tissue containing abundant trabecular structures.

### [Example 21]

Figure 25 presents the results of an experiment in which the left upper limb of a nude mouse was repositioned dorsally, followed by a transverse amputation at the forearm, leaving the cross-section as an open wound, and performing gene transduction. Gene transduction was conducted using retroviruses to transduce FGF10, FGF20, FGF2, NFIB, PPARD, and POLE4 into the central region of the stump, SHH into the lateral side of the stump, and AAVDJK2 to transduce FGF8 into the stump. The retroviruses were prepared following standard methods using PMXs retroviral backbone plasmids encoding each gene and packaging plasmids (pCMV-gagpol-PA, pCMV-VSVg). These plasmids were transfected into 293FT cells (Thermo Fisher Scientific) using Lipofectamine 2000 (Thermo Fisher Scientific). After medium replacement, the cell culture supernatant was collected and used as the retroviral solution. For each animal, 4 ml of retroviral solution was used for FGF 10, FGF20, FGF2, and SHH, while 12 ml of retroviral solution was used for NFIB, PPARD, and POLE4. These solutions were concentrated using polyethylene glycol (PEG) precipitation to obtain the concentrated viral solution. For AAV, AAV vectors expressing FGF8 under the K14 promoter were prepared and used following the AAV concentration method described in Example 2, with the AAVDJK2 capsid. Four- to five-week-old BALB/cAJcl-nu/nu mice (obtained from the Central Institute for Experimental Animals) underwent dorsal repositioning of the left upper limb using the surgical technique described in Example 13. On the second day after repositioning, the amputation level was set proximal to a skin structure on the forearm surface that exhibited a mild protrusion and erythema. At the planned amputation site, a retroviral solution expressing FGF10, FGF20, FGF2, NFIB, PPARD, and POLE4 was injected, followed by a transverse amputation, leaving the cross-section as an open wound. After amputation, an additional injection of the retroviral solution expressing FGF10, FGF20, FGF2, NFIB, PPARD, and POLE4 was administered. Subsequently, a retroviral solution expressing SHH was injected into the anterolateral forearm region. The retroviral solution expressing FGF10, FGF20, FGF2, NFIB, PPARD, and POLE4 was injected at 20 µl in total before and after amputation, while the retroviral solution expressing SHH was injected at 2 µl. Afterward, 4 µl of the retroviral solution expressing FGF10, FGF20, FGF2, NFIB, PPARD, and POLE4 was locally injected 18 times from before amputation until the fifth day post-amputation. The retroviral solution expressing SHH was locally injected 11 times at 2 µl each into the anterolateral region from after amputation until the third day post-amputation. The AAVDJK2 vector expressing FGF8 under the K14 promoter, containing 1.0 × 10¹¹ GC per 2 µl solution, was locally injected seven times from the fifth to the twenty-third day post-amputation. Photographs were taken weekly after amputation under a surgical microscope (MM100-YOH, Mitaka Kohki Co., Ltd.). After three weeks post-amputation, the distal stump underwent flattening in shape, and when observed from the ventral side, branched white structures became visible. On the 28th day post-amputation, photographs were taken before collecting forearm tissue. The samples were fixed overnight in 4% paraformaldehyde-phosphate buffered solution (pH 7.4), embedded in OCT compound, and frozen. Using Kawamoto's film method (SECTION-LAB, Cryofilm type 2C(9), 2.5 cm C-FP094), 10 µm thick frozen sections were collected at 200 µm intervals, stained with hematoxylin-eosin (HE), and scanned into image files using a slide scanner (VS200, Olympus). Histological analysis revealed that within the soft tissue mass, bone marrow cells were clustered at a location corresponding to the white structures.

### [Example 22]

Figure 26 presents the results of an experiment in which the left upper limb of a nude mouse was repositioned dorsally, followed by a transverse amputation at the forearm, with the cross-section manually compressed using the surrounding skin, and performing gene transduction. Gene transduction was conducted using retroviruses to transduce FGF10, FGF20, FGF2, and OCT4 into the stump, AAVDJ to transduce SHH into the lateral side of the stump, and AAVDJ to transduce FGF8 from the medial side of the stump to the distal region. The retroviruses were prepared following standard methods using PMXs retroviral backbone plasmids encoding each gene and packaging plasmids (pCMV-gagpol-PA, pCMV-VSVg). These plasmids were transfected into 293FT cells (Thermo Fisher Scientific) using Lipofectamine 2000 (Thermo Fisher Scientific). After medium replacement, the cell culture supernatant was collected and used as the retroviral solution. For each animal, 4 ml of retroviral solution was used for FGF10, FGF20, and FGF2, and 24 ml of retroviral solution was used for OCT4 for the first 20 local injections. These solutions were concentrated using polyethylene glycol (PEG) precipitation to obtain the concentrated viral solution. For the subsequent 16 local injections, 24 ml of retroviral solution expressing OCT4 was used as the base and concentrated using polyethylene glycol (PEG) precipitation to obtain the concentrated viral solution. For AAV, AAV vectors expressing SHH and FGF8 under the CAG promoter, with an AAVDJ capsid, were ordered and purchased from VectorBuilder. Four- to five-week-old BALB/cAJcl-nu/nu mice (obtained from the Central Institute for Experimental Animals) underwent dorsal repositioning of the left upper limb using the surgical technique described in Example 13. On the following day, the amputation level was set proximal to a skin structure on the forearm surface that exhibited a mild protrusion and erythema. AAVDJ-SHH (1.0 × 10¹¹ GC/2 µl) was locally injected into the lateral forearm region, and AAVDJ-FGF8 (1.0 × 10¹¹ GC/2 µl) was locally injected into the medial forearm region. On the next day, 20 µl of concentrated retroviral solution expressing FGF10, FGF20, FGF2, and OCT4 was mixed with 5 µl of Kenacort A (40 mg/ml) as a steroid and injected subcutaneously, intramuscularly, and into the periosteal tissue. The forearm was then amputated at the planned amputation level, and the anterior and posterior skin of the forearm was manually compressed to close the wound surface. Until the fourth day post-amputation, 10 µl of the same retroviral solution was locally injected 20 times. From the fifth to the tenth day post-amputation, 10 µl of concentrated retroviral solution expressing OCT4 was locally injected 16 times. On the sixth, thirteenth, and twentieth days post-amputation, AAVDJ-SHH (1.0 × 10¹¹ GC/2 µl) was locally injected into the lateral side of the amputation stump, while AAVDJ-FGF8 (1.0 × 10¹¹ GC/2 µl) was locally injected into the medial side of the amputation stump. Photographs were taken weekly after amputation under a surgical microscope (MM100-YOH, Mitaka Kohki Co., Ltd.). Around ten weeks post-amputation, a protruding structure appeared at the distal stump, which subsequently continued to elongate. On the 105th day post-amputation, photographs were taken before collecting forearm tissue. The samples were fixed overnight in 4% paraformaldehyde-phosphate buffered solution (pH 7.4), embedded in OCT compound, and frozen. Using Kawamoto's film method (SECTION-LAB, Cryofilm type 2C(9), 2.5 cm C-FP094), 10 µm thick frozen sections were collected at 200 µm intervals, stained with hematoxylin-eosin (HE), and scanned into image files using a slide scanner (VS200, Olympus). Histological analysis revealed axially aligned cellular structures within the elongated tissue.

### [Example 23]

Figure 27 presents the results of an experiment in which the left upper limb of a nude mouse was repositioned dorsally, followed by a transverse amputation at the forearm, with the cross-section manually compressed using the surrounding skin, and performing gene transduction. Gene transduction was conducted using retroviruses to transduce FGF10, FGF20, FGF2, OCT4, and SOX2 into the stump, AAVDJ to transduce SHH into the lateral side of the stump, and AAVDJ to transduce FGF8 from the medial side of the stump to the distal region. The retroviruses were prepared following standard methods using PMXs retroviral backbone plasmids encoding each gene and packaging plasmids (pCMV-gagpol-PA, pCMV-VSVg). These plasmids were transfected into 293FT cells (Thermo Fisher Scientific) using Lipofectamine 2000 (Thermo Fisher Scientific). After medium replacement, the cell culture supernatant was collected and used as the retroviral solution. For each animal, 4 ml of retroviral solution was used for FGF10, FGF20, and FGF2, and 12 ml of retroviral solution was used for OCT4 and SOX2 for the first 20 local injections. These solutions were concentrated using polyethylene glycol (PEG) precipitation to obtain the concentrated viral solution. For the subsequent 16 local injections, 12 ml of retroviral solution expressing OCT4 and SOX2 was concentrated using polyethylene glycol (PEG) precipitation to obtain the concentrated viral solution. For AAV, AAV vectors expressing SHH and FGF8 under the CAG promoter, with an AAVDJ capsid, were ordered and purchased from VectorBuilder. Four- to five-week-old BALB/cAJcl-nu/nu mice (obtained from the Central Institute for Experimental Animals) underwent dorsal repositioning of the left upper limb using the surgical technique described in Example 13. On the following day, the amputation level was set proximal to a skin structure on the forearm surface that exhibited a mild protrusion and erythema. AAVDJ-SHH (1.0 × 10¹¹ GC/2 µl) was locally injected into the lateral forearm region, and AAVDJ-FGF8 (1.0 × 10¹¹ GC/2 µl) was locally injected into the medial forearm region. On the next day, 20 µl of concentrated retroviral solution expressing FGF10, FGF20, FGF2, OCT4, and SOX2 was mixed with 5 µl of Kenacort A (40 mg/ml) as a steroid and injected subcutaneously, intramuscularly, and into the periosteal tissue. The forearm was then amputated at the planned amputation level, and the anterior and posterior skin of the forearm was manually compressed to close the wound surface. Until the fourth day post-amputation, 10 µl of the same retroviral solution was locally injected 20 times. From the fifth to the tenth day post-amputation, 10 µl of concentrated retroviral solution expressing OCT4 and SOX2 was locally injected 16 times. On the sixth, thirteenth, and twentieth days post-amputation, AAVDJ-SHH (1.0 × 10¹¹ GC/2 µl) was locally injected into the lateral side of the amputation stump, while AAVDJ-FGF8 (1.0 × 10¹¹ GC/2 µl) was locally injected into the medial side of the amputation stump. Photographs were taken weekly after amputation under a surgical microscope (MM100-YOH, Mitaka Kohki Co., Ltd.). Around eight weeks post-amputation, a protruding structure appeared at the distal stump, which subsequently continued to elongate in a branched form. On the 70th day post-amputation, photographs were taken before collecting forearm tissue. The samples were fixed overnight in 4% paraformaldehyde-phosphate buffered solution (pH 7.4), embedded in OCT compound, and frozen. Using Kawamoto's film method (SECTION-LAB, Cryofilm type 2C(9), 2.5 cm C-FP094), 10 µm thick frozen sections were collected at 200 µm intervals, stained with hematoxylin-eosin (HE), and scanned into image files using a slide scanner (VS200, Olympus). Histological analysis revealed axially aligned cellular structures within the elongated branched region.

### [Example 24]

Figure 28 presents the results of an experiment in which the left upper limb of a nude mouse was repositioned dorsally, followed by a transverse amputation at the forearm, with the cross-section manually compressed using the surrounding skin, and performing gene transduction. Gene transduction was conducted using retroviruses to transduce FGF10, FGF20, FGF2, OCT4, SOX2, and TBX6 into the stump, AAVDJ to transduce SHH into the lateral side of the stump, and AAVDJ to transduce FGF8 from the medial side of the stump to the distal region. The retroviruses were prepared following standard methods using PMXs retroviral backbone plasmids encoding each gene and packaging plasmids (pCMV-gagpol-PA, pCMV-VSVg). These plasmids were transfected into 293FT cells (Thermo Fisher Scientific) using Lipofectamine 2000 (Thermo Fisher Scientific). After medium replacement, the cell culture supernatant was collected and used as the retroviral solution. For each animal, 4 ml of retroviral solution was used for FGF10, FGF20, and FGF2, and 8 ml of retroviral solution was used for OCT4, SOX2, and TBX6 for the first 20 local injections. These solutions were concentrated using polyethylene glycol (PEG) precipitation to obtain the concentrated viral solution. For the subsequent 16 local injections, 8 ml of retroviral solution expressing OCT4, SOX2, and TBX6 was concentrated using polyethylene glycol (PEG) precipitation to obtain the concentrated viral solution. For AAV, AAV vectors expressing SHH and FGF8 under the CAG promoter, with an AAVDJ capsid, were ordered and purchased from VectorBuilder. Four- to five-week-old BALB/cAJcl-nu/nu mice (obtained from the Central Institute for Experimental Animals) underwent dorsal repositioning of the left upper limb using the surgical technique described in Example 13. On the following day, the amputation level was set proximal to a skin structure on the forearm surface that exhibited a mild protrusion and erythema. AAVDJ-SHH (1.0 × 10¹¹ GC/2 µl) was locally injected into the lateral forearm region, and AAVDJ-FGF8 (1.0 × 10¹¹ GC/2 µl) was locally injected into the medial forearm region. On the next day, 20 µl of concentrated retroviral solution expressing FGF10, FGF20, FGF2, OCT4, SOX2, and TBX6 was mixed with 5 µl of Kenacort A (40 mg/ml) as a steroid and injected subcutaneously, intramuscularly, and into the periosteal tissue. The forearm was then amputated at the planned amputation level, and the anterior and posterior skin of the forearm was manually compressed to close the wound surface. Until the fourth day post-amputation, 10 µl of the same retroviral solution was locally injected 20 times. From the fifth to the tenth day post-amputation, 10 µl of concentrated retroviral solution expressing OCT4, SOX2, and TBX6 was locally injected 16 times. On the sixth, thirteenth, and twentieth days post-amputation, AAVDJ-SHH (1.0 × 10¹¹ GC/2 µl) was locally injected into the lateral side of the amputation stump, while AAVDJ-FGF8 (1.0 × 10¹¹ GC/2 µl) was locally injected into the medial side of the amputation stump. Photographs were taken weekly after amputation under a surgical microscope (MM100-YOH, Mitaka Kohki Co., Ltd.). Around eight weeks post-amputation, a protruding structure appeared at the distal stump, which subsequently continued to elongate in a branched form. On the 98th day post-amputation, photographs were taken before collecting forearm tissue. The samples were fixed overnight in 4% paraformaldehyde-phosphate buffered solution (pH 7.4), embedded in OCT compound, and frozen. Using Kawamoto's film method (SECTION-LAB, Cryofilm type 2C(9), 2.5 cm C-FP094), 10 µm thick frozen sections were collected at 200 µm intervals, stained with hematoxylin-eosin (HE), and scanned into image files using a slide scanner (VS200, Olympus). Histological analysis revealed a cartilaginous tissue structure within the elongated branched region, which exhibited an axially aligned arrangement resembling the terminal phalanges of fingers, along with a trabecular bone structure accompanied by new bone formation.

### [Example 25]

Figure 29 presents the results of an experiment in which the left upper limb of a nude mouse was repositioned dorsally, followed by a transverse amputation at the forearm, with the cross-section manually compressed using the surrounding skin, and performing gene transduction. The figure shows the external progression of the stump, along with representative histological findings and magnified images. Gene transduction was conducted using retroviruses to transduce FGF10, FGF20, FGF2, OCT4, SOX2, TBX6, and SHH into the stump, and AAVDJ to transduce FGF8 into the stump tip. The retroviruses were prepared following standard methods using PMXs retroviral backbone plasmids encoding each gene and packaging plasmids (pCMV-gagpol-PA, pCMV-VSVg). These plasmids were transfected into 293FT cells (Thermo Fisher Scientific) using Lipofectamine 2000 (Thermo Fisher Scientific). After medium replacement, the cell culture supernatant was collected and used as the retroviral solution. For each animal, 4 ml of retroviral solution was used for FGF10, FGF20, and FGF2, and 8 ml of retroviral solution was used for OCT4, SOX2, and TBX6 for the first set of local injections (20 times in total). These solutions were concentrated using polyethylene glycol (PEG) precipitation to obtain the concentrated viral solution. For SHH, 6 ml of retroviral solution was used, which was prepared separately from the other gene-expressing retroviruses. For AAV, AAV vectors expressing FGF8 under the CAG promoter, with an AAVDJ capsid, were ordered and purchased from VectorBuilder. Four- to five-week-old BALB/cAJcl-nu/nu mice (obtained from the Central Institute for Experimental Animals) underwent dorsal repositioning of the left upper limb using the surgical technique described in Example 13. After repositioning, the amputation level was set proximal to a skin structure on the forearm surface that exhibited a mild protrusion and erythema. AAVDJ-SHH (2.0 × 10¹¹ GC/6 µl) was locally injected subcutaneously at the planned amputation line. On the following day, 20 µl of concentrated retroviral solution expressing FGF10, FGF20, FGF2, OCT4, SOX2, and TBX6 was injected subcutaneously, intramuscularly, and into periosteal tissue at the planned amputation level. The forearm was then amputated, and the anterior and posterior skin of the forearm was manually compressed to close the wound surface. Immediately after amputation, 3 µl of retroviral solution expressing SHH was injected subcutaneously near the lateral side of the stump. Two hours post-amputation, 8 µl of retroviral solution expressing FGF10, FGF20, FGF2, OCT4, SOX2, and TBX6, mixed with 2 µl of Kenacort A (40 mg/ml) as a steroid, was locally injected. From the first to the fifth day post-amputation, 10 µl of retroviral solution expressing FGF10, FGF20, FGF2, OCT4, SOX2, and TBX6 was locally injected 16 times. From the first to the third day post-amputation, 3 µl of retroviral solution expressing SHH was locally injected three times subcutaneously near the lateral side of the stump. From the sixth to the eighth day post-amputation, 8 µl of concentrated retroviral solution expressing OCT4, SOX2, and TBX6 was locally injected seven times. On the sixth, thirteenth, and twentieth days post-amputation, AAVDJ-FGF8 (1.0 × 10¹¹ GC/2.5 µl) was locally injected into the stump tip. Photographs were taken weekly after amputation under a surgical microscope (MM100-YOH, Mitaka Kohki Co., Ltd.) (No records were available for the 21st day post-amputation). On the 56th day post-amputation, photographs were taken before collecting forearm tissue. The samples were fixed overnight in 4% paraformaldehyde-phosphate buffered solution (pH 7.4), embedded in OCT compound, and frozen. Using Kawamoto's film method (SECTION-LAB, Cryofilm type 2C(9), 2.5 cm C-FP094), 10 µm thick frozen sections were collected at 200 µm intervals, stained with hematoxylin-eosin (HE), and scanned into image files using a slide scanner (VS200, Olympus). Histological analysis revealed a pouch-like structure at the distal bone stump, with newly formed cartilage tissue and muscle bundles that were independent of the surrounding muscle tissue.

### [Example 26]

Figure 30 presents the results of an experiment in which the left upper limb of a nude mouse was repositioned dorsally, followed by gene transduction at the planned amputation site through FGF2 injection and viral vector administration, then a transverse amputation at the forearm, leaving the cross-section as an open wound, and continuing gene transduction post-amputation. Gene transduction was conducted using retroviruses to transduce HMGB3, DMNT1, HDAC2, PRDX2, and FGF10 into the stump, and AAVDJ to transduce SHH into the lateral side of the stump. The retroviruses were prepared following standard methods using PMXs retroviral backbone plasmids encoding each gene and packaging plasmids (pCMV-gagpol-PA, pCMV-VSVg). These plasmids were transfected into 293FT cells (Thermo Fisher Scientific) using Lipofectamine 2000 (Thermo Fisher Scientific). After medium replacement, the cell culture supernatant was collected and used as the retroviral solution. For each animal, 6 ml of retroviral solution was concentrated using polyethylene glycol (PEG) precipitation to obtain the concentrated viral solution. For AAV, AAV vectors expressing SHH under the CAG promoter, with an AAVDJ capsid, were prepared and used following the AAV concentration method described in Example 2. As the FGF2 solution, Fiblast Spray (Kaken Pharmaceutical Co., Ltd.) dissolved in PBS (500 µg/ml) was used. Four- to five-week-old BALB/cAJcl-nu/nu mice (obtained from the Central Institute for Experimental Animals) underwent dorsal repositioning of the left upper limb using the surgical technique described in Example 13. On the seventh day after repositioning, the amputation level was set proximal to a skin structure on the forearm surface that exhibited a mild protrusion and erythema. 30 µl of concentrated retroviral solution expressing HMGB3, DMNT1, HDAC2, PRDX2, and FGF10 was locally injected into the planned amputation site. Six hours later, 25 µl of FGF2 solution was locally injected. On the following day and the second day as well, in the same manner, retroviral solution and FGF2 solution were administered again at scheduled intervals to the planned amputation site. On the third day, after a final injection of 30 µl of concentrated retroviral solution, a transverse amputation was performed, leaving the cross-section as an open wound. Immediately after amputation, AAVDJ-SHH (5.0 × 10¹¹ GC) was locally injected into the lateral side of the stump. On the first and second days post-amputation, 2 µl of concentrated retroviral solution was applied to the amputation surface. Photographs were taken weekly after amputation under a surgical microscope (MM100-YOH, Mitaka Kohki Co., Ltd.). Around the fifth week post-amputation, the distal stump began to protrude and elongate over time. On the 63rd day post-amputation, photographs were taken before collecting forearm tissue. The samples were fixed overnight in 4% paraformaldehyde-phosphate buffered solution (pH 7.4), embedded in OCT compound, and frozen. Using Kawamoto's film method (SECTION-LAB, Cryofilm type 2C(9), 2.5 cm C-FP094), 10 µm thick frozen sections were collected at 200 µm intervals, stained with hematoxylin-eosin (HE), and scanned into image files using a slide scanner (VS200, Olympus). Histological analysis revealed cartilage tissue accompanied by endochondral ossification-like structures resembling intra-articular cartilage at the bone stump.

### [Example 27]

Figure 31 presents the results of an experiment in which the left upper limb of a nude mouse was repositioned dorsally, followed by gene transduction at the planned amputation site through FGF2 injection and viral vector administration, then a transverse amputation at the forearm, leaving the cross-section as an open wound, and continuing gene transduction post-amputation. Gene transduction was conducted using retroviruses to transduce FGF10 into the stump, and AAVDJ to transduce SHH into the lateral side of the stump. The retroviruses were prepared following standard methods using PMXs retroviral backbone plasmids encoding each gene and packaging plasmids (pCMV-gagpol-PA, pCMV-VSVg). These plasmids were transfected into 293FT cells (Thermo Fisher Scientific) using Lipofectamine 2000 (Thermo Fisher Scientific). After medium replacement, the cell culture supernatant was collected and used as the retroviral solution. For each animal, 6 ml of retroviral solution was concentrated using polyethylene glycol (PEG) precipitation to obtain 80 µl of concentrated viral solution. For AAV, AAV vectors expressing SHH under the CAG promoter, with an AAVDJ1 capsid, were prepared and used following the AAV concentration method described in Example 2. As the FGF2 solution, Fiblast Spray (Kaken Pharmaceutical Co., Ltd.) dissolved in PBS (500 µg/ml) was used. Four- to five-week-old BALB/cAJcl-nu/nu mice (obtained from the Central Institute for Experimental Animals) underwent dorsal repositioning of the left upper limb using the surgical technique described in Example 13. On the tenth day after repositioning, the amputation level was set proximal to a skin structure on the forearm surface that exhibited a mild protrusion and erythema. From four to two days before amputation, 20 µl of concentrated retroviral solution was injected, followed by a local injection of 20 µl of FGF2 solution 3 to 5 hours later. At the time of amputation, the nerve stump was left longer, and the surrounding skin of the stump was excised. Then, 20 µl of concentrated retroviral solution was injected. On the seventh day post-amputation, AAVDJ1-SHH (1.0 × 10¹¹ GC) was locally injected into the lateral side of the stump. Photographs were taken weekly after amputation under a surgical microscope (MM100-YOH, Mitaka Kohki Co., Ltd.). Around two weeks post-amputation, the distal stump began to protrude and elongate over time. On the 63rd day post-amputation, photographs were taken before collecting forearm tissue. The samples were fixed overnight in 4% paraformaldehyde-phosphate buffered solution (pH 7.4), embedded in OCT compound, and frozen. Using Kawamoto's film method (SECTION-LAB, Cryofilm type 2C(9), 2.5 cm C-FP094), 10 µm thick frozen sections were collected at 200 µm intervals, stained with hematoxylin-eosin (HE), and scanned into image files using a slide scanner (VS200, Olympus). Histological analysis revealed that cartilage tissue was continuously induced from the cutaneous muscle layer near the stump.

### [Example 28]

Figure 32 presents the results of an experiment in which the left upper limb of a nude mouse was repositioned dorsally, followed by gene transduction at the planned amputation site through FGF2 injection and viral vector administration, then a transverse amputation at the forearm, leaving the cross-section as an open wound, and continuing gene transduction post-amputation. Gene transduction was conducted using retroviruses to transduce HMGB3, DMNT1, HDAC2, and FGF10 into the stump, and AAVDJ to transduce SHH into the lateral side of the stump. The retroviruses were prepared following standard methods using PMXs retroviral backbone plasmids encoding each gene and packaging plasmids (pCMV-gagpol-PA, pCMV-VSVg). These plasmids were transfected into 293FT cells (Thermo Fisher Scientific) using Lipofectamine 2000 (Thermo Fisher Scientific). After medium replacement, the cell culture supernatant was collected and used as the retroviral solution. For each animal, 6 ml of retroviral solution was concentrated using polyethylene glycol (PEG) precipitation to obtain 80 µl of concentrated viral solution. For AAV, AAV vectors expressing SHH under the CAG promoter, with an AAVDJ1 capsid, were prepared and used following the AAV concentration method described in Example 2. As the FGF2 solution, Fiblast Spray (Kaken Pharmaceutical Co., Ltd.) dissolved in PBS (500 µg/ml) was used. Four- to five-week-old BALB/cAJcl-nu/nu mice (obtained from the Central Institute for Experimental Animals) underwent dorsal repositioning of the left upper limb using the surgical technique described in Example 13. On the tenth day after repositioning, the amputation level was set proximal to a skin structure on the forearm surface that exhibited a mild protrusion and erythema. From four to two days before amputation, 20 µl of concentrated retroviral solution was injected, followed by a local injection of 20 µl of FGF2 solution 3 to 5 hours later. At the time of amputation, the nerve stump was left longer, and the surrounding skin of the stump was excised. Then, 20 µl of concentrated retroviral solution was injected. On the seventh day post-amputation, AAVDJ1-SHH (1.0 × 10¹¹ GC) was locally injected into the lateral side of the stump. Photographs were taken weekly after amputation under a surgical microscope (MM100-YOH, Mitaka Kohki Co., Ltd.). Around two weeks post-amputation, the distal stump began to protrude and elongate over time. On the 63rd day post-amputation, photographs were taken before collecting forearm tissue. The samples were fixed overnight in 4% paraformaldehyde-phosphate buffered solution (pH 7.4), embedded in OCT compound, and frozen. Using Kawamoto's film method (SECTION-LAB, Cryofilm type 2C(9), 2.5 cm C-FP094), 10 µm thick frozen sections were collected at 200 µm intervals, stained with hematoxylin-eosin (HE), and scanned into image files using a slide scanner (VS200, Olympus). Histological analysis revealed that cartilage tissue was continuously induced from the cutaneous muscle layer near the stump, and cartilage-like tissue covered the bone stump.

### [Example 29]

Figure 33 presents the results of an experiment in which the left upper limb of a nude mouse was repositioned dorsally, followed by gene transduction at the planned amputation site through FGF2 injection and viral vector administration, then a transverse amputation at the forearm, leaving the cross-section as an open wound, and continuing gene transduction post-amputation. Gene transduction was conducted using retroviruses to transduce MSX1, MSX2, LIN28A, MEIS 1, and FGF10 into the stump, AAVDJ1 to transduce SHH into the lateral side of the stump, and AAVDJK2 to transduce LEF1 and FGF8 into the distal stump. The retroviruses were prepared following standard methods using PMXs retroviral backbone plasmids encoding each gene and packaging plasmids (pCMV-gagpol-PA, pCMV-VSVg). These plasmids were transfected into 293FT cells (Thermo Fisher Scientific) using Lipofectamine 2000 (Thermo Fisher Scientific). After medium replacement, the cell culture supernatant was collected and used as the retroviral solution. For each animal, 8 ml of retroviral solution was used for MSX1, MSX2, and LIN28A, and 4 ml of retroviral solution was used for MEIS1 and FGF10. These solutions were concentrated using polyethylene glycol (PEG) precipitation to obtain the concentrated viral solution, which was prepared twice. For AAV, AAV vectors expressing SHH under the CAG promoter, with an AAVDJ1 capsid, and AAV vectors expressing LEF1 or FGF8 under the K14 promoter, with an AAVDJK2 capsid, were prepared and used following the AAV concentration method described in Example 2. As the FGF2 solution, Fiblast Spray (Kaken Pharmaceutical Co., Ltd.) dissolved in PBS (500 µg/ml) was used. Four- to five-week-old BALB/cAJcl-nu/nu mice (obtained from the Central Institute for Experimental Animals) underwent dorsal repositioning of the left upper limb using the surgical technique described in Example 13. On the fifth day after repositioning (eight days before amputation), the amputation level was set proximal to a skin structure on the forearm surface that exhibited a mild protrusion and erythema. From eight to one days before amputation, 20 µl of concentrated retroviral solution was injected, followed by a local injection of 20 µl of FGF2 solution 3 to 5 hours later. At the time of amputation, the nerve stump was left longer, and the surrounding skin of the stump was excised. Then, 20 µl of concentrated retroviral solution was injected. On the eleventh day post-amputation, AAVDJ1-SHH (5 × 10¹¹ GC/4 µl) was transduced into the lateral side of the stump. On the fourteenth day post-amputation, AAVDJK2-LEF1 and AAVDJK2-FGF8 (5 × 10¹¹ GC each, total 4 µl) were transduced into the distal stump. Photographs were taken weekly after amputation under a surgical microscope (MM100-YOH, Mitaka Kohki Co., Ltd.). Around two weeks post-amputation, the distal stump began to protrude and elongate over time. On the 56th day post-amputation, photographs were taken before collecting forearm tissue. The samples were fixed overnight in 4% paraformaldehyde-phosphate buffered solution (pH 7.4), embedded in OCT compound, and frozen. Using Kawamoto's film method (SECTION-LAB, Cryofilm type 2C(9), 2.5 cm C-FP094), 10 µm thick frozen sections were collected at 200 µm intervals, stained with hematoxylin-eosin (HE), and scanned into image files using a slide scanner (VS200, Olympus). Histological analysis confirmed axial elongation of the bone stump and the induction of cartilage tissue accompanied by endochondral ossification-like structures resembling intra-articular cartilage at the bone stump.

### [Example 30]

Figure 34 presents the results of an experiment in which the left upper limb of a nude mouse was repositioned dorsally, followed by gene transduction at the planned amputation site through FGF2 injection and viral vector administration, then a transverse amputation at the forearm, leaving the cross-section as an open wound, and continuing gene transduction post-amputation. Gene transduction was conducted using retroviruses to transduce MSX1, MSX2, LIN28A, WNT7A, and FGF10 into the stump, AAVDJ1 to transduce SHH into the lateral side of the stump, and AAVDJK2 to transduce LEF1 into the subcutaneous tissue circumferentially. The retroviruses were prepared following standard methods using PMXs retroviral backbone plasmids encoding each gene and packaging plasmids (pCMV-gagpol-PA, pCMV-VSVg). These plasmids were transfected into 293FT cells (Thermo Fisher Scientific) using Lipofectamine 2000 (Thermo Fisher Scientific). After medium replacement, the cell culture supernatant was collected and used as the retroviral solution. For each animal, 6 ml of retroviral solution was used for MSX1, MSX2, and LIN28A, and 8 ml of retroviral solution was used for WNT7A and FGF10. These solutions were concentrated using polyethylene glycol (PEG) precipitation to obtain the concentrated viral solution. For AAV, AAV vectors expressing SHH under the CAG promoter, with an AAVDJ1 capsid, and AAV vectors expressing LEF1 under the K16P5 promoter, with an AAVDJK2 capsid, were prepared and used following the AAV concentration method described in Example 2. As the FGF2 solution, Fiblast Spray (Kaken Pharmaceutical Co., Ltd.) dissolved in PBS (500 µg/ml) was used. Four- to five-week-old BALB/cAJcl-nu/nu mice (obtained from the Central Institute for Experimental Animals) underwent dorsal repositioning of the left upper limb using the surgical technique described in Example 13. On the fourth day after repositioning, the amputation level was set proximal to a skin structure on the forearm surface that exhibited a mild protrusion and erythema. From five to two days before amputation, 20 µl of concentrated retroviral solution was injected, followed by a local injection of 20 µl of FGF2 solution 3 to 5 hours later. On the day before amputation, 3.3 × 10¹⁰ GC/4 µl of AAVDJ1 virus solution expressing SHH under the CAG promoter was injected into the lateral side, followed by 1.6 × 10¹¹ GC/10 µl of AAVDJK2 virus solution expressing LEF1 under the K16P5 promoter, which was circumferentially injected into the subcutaneous tissue. Twelve hours later, 20 µl of FGF2 solution was locally injected. Before amputation, 20 µl of retroviral solution was injected into the planned amputation site, followed by a transverse amputation. The surrounding skin of the stump was excised. On the day after amputation, 4 µl of retroviral solution was injected into the distal stump. Photographs were taken weekly after amputation under a surgical microscope (MM100-YOH, Mitaka Kohki Co., Ltd.). Around two weeks post-amputation, the distal stump began to protrude progressively over time. On the 56th day post-amputation, photographs were taken before collecting forearm tissue. The samples were fixed overnight in 4% paraformaldehyde-phosphate buffered solution (pH 7.4), embedded in OCT compound, and frozen. Using Kawamoto's film method (SECTION-LAB, Cryofilm type 2C(9), 2.5 cm C-FP094), 10 µm thick frozen sections were collected at 200 µm intervals, stained with hematoxylin-eosin (HE), and scanned into image files using a slide scanner (VS200, Olympus). Histological analysis confirmed that muscle tissue had thickened and proliferated in the subcutaneous area surrounding the amputation site.

### [Example 31]

Figure 35 presents the results of an experiment in which the left upper limb of a nude mouse was repositioned dorsally, followed by gene transduction at the planned amputation site through FGF2 injection and viral vector administration, then a transverse amputation at the forearm, leaving the cross-section as an open wound, and continuing gene transduction post-amputation. Gene transduction was conducted using retroviruses to transduce LEF1, HDAC2, PRDX2, WNT7A, and FGF10 into the stump, AAVDJ1 to transduce SHH into the lateral side of the stump, and AAVDJK2 to transduce LEF1 into the subcutaneous tissue circumferentially. The retroviruses were prepared following standard methods using PMXs retroviral backbone plasmids encoding each gene and packaging plasmids (pCMV-gagpol-PA, pCMV-VSVg). These plasmids were transfected into 293FT cells (Thermo Fisher Scientific) using Lipofectamine 2000 (Thermo Fisher Scientific). After medium replacement, the cell culture supernatant was collected and used as the retroviral solution. For each animal, 6 ml of retroviral solution was used for LEF1, HDAC2, and PRDX2, and 8 ml of retroviral solution was used for WNT7A and FGF10. These solutions were concentrated using polyethylene glycol (PEG) precipitation to obtain the concentrated viral solution. For AAV, AAV vectors expressing SHH under the CAG promoter, with an AAVDJ1 capsid, and AAV vectors expressing LEF1 under the K16P5 promoter, with an AAVDJK2 capsid, were prepared and used following the AAV concentration method described in Example 2. As the FGF2 solution, Fiblast Spray (Kaken Pharmaceutical Co., Ltd.) dissolved in PBS (500 µg/ml) was used. Four- to five-week-old BALB/cAJcl-nu/nu mice (obtained from the Central Institute for Experimental Animals) underwent dorsal repositioning of the left upper limb using the surgical technique described in Example 13. On the fourth day after repositioning, the amputation level was set proximal to a skin structure on the forearm surface that exhibited a mild protrusion and erythema. From five to two days before amputation, 20 µl of concentrated retroviral solution was injected, followed by a local injection of 20 µl of FGF2 solution 3 to 5 hours later. On the day before amputation, 3.3 × 10¹⁰ GC/4 µl of AAVDJ1-SHH virus solution was injected into the lateral side, followed by 1.6 × 10¹¹ GC/10 µl of AAVDJK2-LEF1 virus solution, which was injected circumferentially into the subcutaneous tissue. Twelve hours later, 20 µl of FGF2 solution was locally injected. Before amputation, 20 µl of retroviral solution was injected into the planned amputation site, followed by a transverse amputation. The surrounding skin of the stump was excised, and 4 µl of retroviral solution was injected into the distal stump on the following day. Photographs were taken weekly after amputation under a surgical microscope (MM100-YOH, Mitaka Kohki Co., Ltd.). Around two weeks post-amputation, the distal stump began to protrude progressively over time. On the 56th day post-amputation, photographs were taken before collecting forearm tissue. The samples were fixed overnight in 4% paraformaldehyde-phosphate buffered solution (pH 7.4), embedded in OCT compound, and frozen. Using Kawamoto's film method (SECTION-LAB, Cryofilm type 2C(9), 2.5 cm C-FP094), 10 µm thick frozen sections were collected at 200 µm intervals, stained with hematoxylin-eosin (HE), and scanned into image files using a slide scanner (VS200, Olympus). Histological analysis confirmed that muscle tissue had thickened and proliferated in the subcutaneous area surrounding the amputation site.

### [Example 32]

Figure 36 presents the results of an experiment in which the left upper limb of a nude mouse was repositioned dorsally, followed by a transverse amputation at the forearm, leaving the cross-section as an open wound, and continuing gene transduction post-amputation. Gene transduction was conducted using AAVDJ to transduce LEF1, PRRX1, HDAC2, PRDX2, PPARD, FGF10, FGF20, FGF2, BMP5, and SHH into the stump. For AAV, AAV vectors expressing LEF1, PRRX1, HDAC2, PRDX2, PPARD, FGF10, FGF20, FGF2, BMP5, and SHH under the CAG promoter, with an AAVDJ capsid, were prepared and used following the AAV concentration method described in Example 2. Four- to five-week-old BALB/cAJcl-nu/nu mice (obtained from the Central Institute for Experimental Animals) underwent dorsal repositioning of the left upper limb using the surgical technique described in Example 13. On the second day after repositioning, the amputation level was set proximal to a skin structure on the forearm surface that exhibited a mild protrusion and erythema. Following transverse amputation, the cross-section was left as an open wound, and from the day of amputation until the second day post-amputation, AAV solutions were injected ten times into the distal stump, containing 5 × 10¹⁰ GC of AAV expressing PRRX1, HDAC2, PRDX2, PPARD, FGF20, FGF2, and SHH, 5 × 10⁹ GC of AAV expressing FGF10, and 1 × 10¹¹ GC of AAV expressing BMP5, for a total volume of 25 µl. Photographs were taken weekly after amputation under a surgical microscope (MM100-YOH, Mitaka Kohki Co., Ltd.). Around two weeks post-amputation, the distal stump gradually enlarged and became rounded over time. On the 70th day post-amputation, photographs were taken before collecting forearm tissue. The samples were fixed overnight in 4% paraformaldehyde-phosphate buffered solution (pH 7.4), embedded in OCT compound, and frozen. Using Kawamoto's film method (SECTION-LAB, Cryofilm type 2C(9), 2.5 cm C-FP094), 10 µm thick frozen sections were collected at 200 µm intervals, stained with hematoxylin-eosin (HE), and scanned into image files using a slide scanner (VS200, Olympus). Histological analysis confirmed the proliferation of adipose tissue within the muscle tissue, extending from the subcutaneous layer to the deep fascia layer.

As demonstrated in Examples 18, 19, 20, 21, 22, 23, 24, 25, 26, 27, 28, 29, 30, 31, and 32, the surgical procedure described in Example 13 and the stump treatment method illustrated as a schematic in Figure 21 provide a research and development platform for developing methods for regenerating amputated limb sites in mammals.

**In** the present invention, interventions such as gene transduction of candidate genes (Table 3) identified through gene expression analyses of mammalian fetal development and amphibian limb regeneration, protein administration, and steroid administration are applied to the stump. As demonstrated in Example 18, an untreated amputation stump typically heals through cortical bone formation and contraction closure of the surrounding skin, without any significant tissue regeneration. However, the interventions of the present invention enable the formation of regenerative structures observed in amphibian limb regeneration, including:
- Formation of a blastema-like soft tissue mass, similar to those seen at the amputated limb stumps of amphibians with higher intrinsic regenerative capacity (Example 19)
- Newly formed bone tissue accompanied by a blastema-like soft tissue mass and abundant trabecular bone structures (Example 20)
- Flattening of the distal stump and the formation of a branched structure by bone marrow cells (Example 21)
- Elongation of the distal stump and the presence of axially aligned cellular structures within the elongated tissue (Example 22)
- Branched elongation of the distal stump with axially stratified cellular structures within the elongated tissue (Example 23)
- Elongation of the distal stump into a branched shape, with a tissue structure composed of cartilage-like cells resembling the terminal phalanges of fingers, accompanied by newly formed trabecular bone structures (Example 24)
- Formation of a pouch-like structure containing cartilage tissue and newly formed independent muscle bundles (Example 25)
These findings demonstrate the potential for regenerating branched, digit-like structures and functionally reconstructed limbs, including neuromuscular tissues. The present invention can be applied as a therapeutic approach for limb regeneration in cases of limb defects, as well as a research and development platform for regenerative therapy.

In the present invention, clear regeneration of cartilage tissue can be achieved, as demonstrated by:
- Protrusion and elongation of the distal stump accompanied by endochondral ossification-like structures resembling intra-articular cartilage (Example 26)
- Induction of continuous cartilage tissue from the cutaneous muscle layer near the stump (Example 27)
- A covering structure of cartilage-like tissue at the bone stump (Example 28)
- Axial elongation of the bone stump and induction of cartilage tissue accompanied by endochondral ossification-like structures resembling intra-articular cartilage at the bone stump (Example 29)
These findings indicate the potential for developing therapeutic approaches for age-related degeneration of articular cartilage and osteoarthritis, as well as methods for inducing endochondral ossification at the bone stump and promoting elongation of the region. Furthermore, the present invention can be applied as a therapeutic approach for limb bone elongation and as a research and development platform for future therapeutic advancements.

In the present invention, regeneration of muscle and adipose tissues can be achieved, as demonstrated by:
- Proliferation of muscle tissue in the subcutaneous area surrounding the amputation site (Examples 30 and 31)
- Proliferation of adipose tissue within muscle tissue, extending from the subcutaneous layer to the deep fascia layer (Example 32)
These findings indicate the potential for developing therapeutic approaches for age-related subcutaneous fat atrophy leading to cosmetic changes and muscle weakness, as well as various conditions resulting in cosmetic changes, such as trauma or malignant tumor resection, and muscle strength alterations in paralytic diseases. Furthermore, the present invention can be applied as a therapeutic approach and a research and development platform for future treatment advancements.

## Claims

1. A non-naturally modified adeno-associated virus (AAV) capsid protein, comprising a peptide insertion relative to its corresponding parent AAV capsid protein, wherein the peptide insertion includes the amino acid sequence EPKARAP (SEQ ID NO: 2), and the insertion site is between amino acid residues 589 and 590 of VP1 in AAVDJ or at the corresponding position in the capsid protein of another AAV serotype.

2. A non-naturally modified adeno-associated virus (AAV) capsid protein, comprising a peptide insertion relative to the parent AAV capsid protein AAVDJ, and having an amino acid sequence selected from SEQ ID NO: 5, SEQ ID NO: 14, SEQ ID NO: 15, SEQ ID NO: 16, or SEQ ID NO: 17.

3. A polynucleotide comprising a nucleotide sequence encoding the modified AAV capsid protein according to Claim 1 or 2.

4. An expression vector comprising the polynucleotide according to Claim 3, wherein the nucleotide sequence encoding the modified AAV capsid protein is operably linked to a promoter sequence.

5. A recombinant virus or viral vector comprising the modified AAV capsid protein according to Claim 1 or 2.

6. The recombinant virus or viral vector according to Claim 5, wherein the recombinant virus or viral vector is an AAV.

7. The recombinant virus or viral vector according to Claim 5 or 6, wherein the recombinant virus or viral vector comprises a nucleotide sequence encoding a therapeutic gene product.

8. The recombinant virus or viral vector according to any one of Claims 5 to 7, wherein the recombinant virus or viral vector exhibits higher infectivity in cells of a skin ulcer surface or epidermal cells.

9. A pharmaceutical composition comprising a pharmaceutically acceptable excipient and the recombinant virus or viral vector according to any one of Claims 5 to 8.

10. A method for treating or preventing a skin disease, skin disorder, or skin function impairment in a subject in need thereof, the method comprising administering the pharmaceutical composition according to Claim 9 to the subject via intradermal injection, subcutaneous injection, or parenteral administration, optionally intravenously, thereby treating or preventing the skin disease, skin disorder, or skin function impairment.

11. A promoter for tissue- or cell-specific expression of a target gene in mammalian cells, comprising a nucleotide sequence described in SEQ ID NO: 23 or SEQ ID NO: 25, or a nucleotide sequence having at least 95% or at least 99% homology to the nucleotide sequence described in SEQ ID NO: 23 or SEQ ID NO: 25.

12. A promoter for tissue- or cell-specific expression of a target gene in mammalian cells, comprising a nucleotide sequence described in SEQ ID NO: 26, or a nucleotide sequence having at least 95% or at least 99% homology to the nucleotide sequence described in SEQ ID NO: 26.

13. A recombinant vector comprising the promoter according to Claim 11 or 12.

14. A recombinant vector comprising the promoter according to Claim 11 or 12, and a nucleotide sequence encoding a therapeutic gene product.

15. The recombinant vector according to Claim 13 or 14, wherein the recombinant vector is an AAV.

16. The recombinant vector according to any one of Claims 13 to 15, wherein the recombinant vector exhibits high gene expression in epidermal cells or epidermal tissue.

17. A pharmaceutical composition comprising a pharmaceutically acceptable excipient and the recombinant vector according to any one of Claims 13 to 16.

18. A method for treating or preventing a skin disease, skin disorder, or skin function impairment in a subject in need thereof, the method comprising administering the pharmaceutical composition according to Claim 17 to the subject via intradermal injection, subcutaneous injection, or parenteral administration, optionally intravenously, thereby treating or preventing the skin disease, skin disorder, or skin function impairment.

19. A gene transduction vector for inducing skin tissue with skin appendages from a skin ulcer surface in vivo by transducing a gene encoding at least one of DNP63A, GRHL2, TFAP2A, cMYC, LEF1, SOX2, HOXC4, HOXC9, HOXC13, JARID2, HEY1, HEY2, FOXO1, FOXD1, EGR3, MEF2C, LHX2, PRRX1, PRRX2, CREB3, ETV1, TBX6, MSX2, PRDM1, or SHH.

20. A gene transduction vector for inducing skin tissue with skin appendages from a skin ulcer surface in vivo by transducing a gene encoding at least one of DNP63A, GRHL2, TFAP2A, cMYC, LEF1, FOXD1, PRDM1, or SHH.

21. A gene transduction vector for inducing the neogenesis of skin appendages or enhancing skin appendage function in vivo by transducing a gene encoding all or some of DNP63A, GRHL2, TFAP2A, cMYC, LEF1, FOXD1, PRDM1, or SHH.

22. A gene transduction vector for inducing the neogenesis of skin appendages or enhancing skin appendage function in vivo by transducing a gene encoding all or some of LEF1, FOXD1, PRDM1, or SHH.

23. A gene transduction vector for inducing the neogenesis of skin appendages or enhancing skin appendage function in vivo by transducing a gene encoding either or both of FOXD1 and PRDM1.

24. The gene transduction vector according to any one of Claims 19 to 23, wherein the gene transduction vector is an AAV or mRNA.

25. A pharmaceutical composition comprising a pharmaceutically acceptable excipient and the gene transduction vector according to any one of Claims 19 to 24.

26. A method for treating or preventing a skin disease, skin disorder, or skin function impairment in a subject in need thereof, the method comprising administering the pharmaceutical composition according to Claim 25 to the subject by ulcer surface application, intradermal injection, or subcutaneous injection, thereby treating or preventing the skin disease, skin disorder, or skin function impairment.

27. A gene transduction pharmaceutical comprising a gene transduction vector and an effective amount of a steroid for enhancing gene transduction efficiency or gene expression.

28. The gene transduction pharmaceutical according to Claim 27, wherein the steroid acts systemically or locally in the subject to be transduced.

29. The gene transduction pharmaceutical according to Claim 27 or 28, wherein the gene transduction vector is a viral vector, AAV, retroviral vector, mRNA processed with lipofection reagents, mRNA formulated into liposomes or lipid nanoparticles, or naked DNA.

30. The gene transduction pharmaceutical according to any one of Claims 27 to 29, wherein the gene transduction vector is a pharmaceutical composition comprising a pharmaceutically acceptable excipient.

31. A method for enhancing gene transduction efficiency, wherein a steroid is systemically administered to the subject prior to gene transduction.

32. A method for treating or preventing a skin disease, skin disorder, or skin function impairment in a subject in need thereof, the method comprising administering a gene transduction vector to the subject via ulcer surface application, intradermal injection, or subcutaneous injection, while systemically or locally administering a steroid to the subject prior to, simultaneously with, or shortly after gene transduction.

33. A method for treating or preventing a muscle disease, muscle disorder, or muscle function impairment in a subject in need thereof, the method comprising administering a gene transduction vector to the subject via intramuscular injection, while systemically or locally administering a steroid to the subject prior to, simultaneously with, or shortly after gene transduction.

34. A method for treating or preventing a bone and cartilage disease, bone and cartilage disorder, or bone and cartilage function impairment in a subject in need thereof, the method comprising administering a gene transduction vector to the subject via local injection, while systemically or locally administering a steroid to the subject prior to, simultaneously with, or shortly after gene transduction.

35. A method for producing an upper limb amputation model animal, comprising repositioning the upper limb of a non-human mammal and subsequently amputating the upper limb at the upper arm, forearm, hand, or finger level.

36. A method for producing an upper limb amputation model animal, comprising repositioning the upper limb of a non-human mammal and subsequently amputating the upper limb at the forearm level.

37. The method according to Claim 35 or 36, wherein the non-human mammal is a rabbit, dog, cat, guinea pig, hamster, rat, or mouse.

38. The method according to any one of Claims 35 to 37, wherein the repositioning of the upper limb is performed while preserving the major nerves, arteries, and veins of the upper limb.

39. The method according to any one of Claims 35 to 38, wherein the repositioning of the upper limb is performed at the shoulder joint.

40. The method according to any one of Claims 35 to 39, wherein the repositioning of the upper limb involves scapular resection.

41. An upper limb amputation model animal produced by the method according to any one of Claims 35 to 40.

42. A method for screening pharmaceutical substances, comprising applying a test substance to the upper limb amputation model animal according to Claim 41 and evaluating its effects on the amputation site.

43. The method for screening pharmaceutical substances according to Claim 42, wherein the pharmaceutical substance is a gene transduction vector for gene transduction.

44. The method for screening pharmaceutical substances according to Claim 42 or 43, wherein the pharmaceutical substance is a material used for the regeneration of amputated tissue.

45. A pharmaceutical composition for use as a research reagent or therapeutic agent to stimulate the regeneration of missing limbs or the regeneration of missing limb tissues in mammals, comprising:
(1) A vector for transducing or acting on at least one gene that is relatively highly expressed in cells possessing tissue regenerative capacity;
(2) A vector for transducing or acting on at least one gene that functions as a master regulator gene during cellular differentiation and development;
(3) A vector for transducing or acting on at least one cytokine gene that is highly active in environments where tissue regeneration occurs; or
(4) A research reagent or pharmaceutical composition comprising at least one protein encoded by any of the genes listed in (1) to (3).

46. The research reagent or pharmaceutical composition according to Claim 45, wherein the gene encodes FGF10, FGF20, FGF2, FGF8, SHH, or OCT4, or wherein the protein is FGF10, FGF20, FGF2, FGF8, SHH, or OCT4.

47. The research reagent or pharmaceutical composition according to Claim 45, wherein the gene encodes FGF10, FGF20, FGF2, FGF8, SHH, OCT4, or SOX2, or wherein the protein is FGF10, FGF20, FGF2, FGF8, SHH, OCT4, or SOX2.

48. The research reagent or pharmaceutical composition according to Claim 45, wherein the gene encodes FGF10, FGF20, FGF2, FGF8, SHH, OCT4, SOX2, or TBX6, or wherein the protein is FGF10, FGF20, FGF2, FGF8, SHH, OCT4, SOX2, or TBX6.

49. The research reagent or pharmaceutical composition according to Claim 45, comprising a gene encoding at least one selected from FGF10, FGF20, FGF2, FGF8, SHH, OCT4, SOX2, and TBX6, or comprising at least one protein selected from FGF10, FGF20, FGF2, FGF8, SHH, OCT4, SOX2, and TBX6.

50. The research reagent or pharmaceutical composition according to Claim 45, wherein the gene encodes FGF10, FGF20, FGF2, FGF8, SHH, LEF1, NFIB, PPARD, POLE4, PRRX1, or HDAC2, or wherein the protein is FGF10, FGF20, FGF2, FGF8, SHH, LEF1, NFIB, PPARD, POLE4, PRRX1, or HDAC2.

51. The research reagent or pharmaceutical composition according to Claim 45, comprising a gene encoding at least one selected from FGF10, FGF20, FGF2, FGF8, SHH, LEF1, NFIB, PPARD, POLE4, PRRX1, and HDAC2, or comprising at least one protein selected from FGF10, FGF20, FGF2, FGF8, SHH, LEF1, NFIB, PPARD, POLE4, PRRX1, and HDAC2.

52. The research reagent or pharmaceutical composition according to Claim 45, wherein the gene encodes FGF10, FGF20, FGF2, FGF8, SHH, LEF1, NFIB, PPARD, POLE4, PRRX1, HDAC2, or BMP5, or wherein the protein is FGF10, FGF20, FGF2, FGF8, SHH, LEF1, NFIB, PPARD, POLE4, PRRX1, HDAC2, or BMP5.

53. The research reagent or pharmaceutical composition according to Claim 45, comprising a gene encoding at least one selected from FGF10, FGF20, FGF2, FGF8, SHH, LEF1, NFIB, PPARD, POLE4, PRRX1, HDAC2, and BMP5, or comprising at least one protein selected from FGF10, FGF20, FGF2, FGF8, SHH, LEF1, NFIB, PPARD, POLE4, PRRX1, HDAC2, and BMP5.

54. The research reagent or pharmaceutical composition according to Claim 45, wherein the gene encodes FGF10, FGF20, FGF2, FGF8, SHH, NFIB, PPARD, or POLE4, or wherein the protein is FGF10, FGF20, FGF2, FGF8, SHH, NFIB, PPARD, or POLE4.

55. The research reagent or pharmaceutical composition according to Claim 45, comprising a gene encoding at least one selected from FGF10, FGF20, FGF2, FGF8, SHH, NFIB, PPARD, and POLE4, or comprising at least one protein selected from FGF10, FGF20, FGF2, FGF8, SHH, NFIB, PPARD, and POLE4.

56. A research reagent or pharmaceutical composition for use as a therapeutic agent to stimulate cartilage tissue regeneration in mammals, comprising:
(1) A vector for transducing or acting on at least one gene that is relatively highly expressed in cells possessing tissue regenerative capacity;
(2) A vector for transducing or acting on at least one gene that functions as a master regulator gene during cellular differentiation and development;
(3) A vector for transducing or acting on at least one cytokine gene that is highly active in environments where tissue regeneration occurs; or
(4) A research reagent or pharmaceutical composition comprising at least one protein encoded by any of the genes listed in (1) to (3).

57. The research reagent or pharmaceutical composition according to Claim 56, wherein the gene encodes FGF10, FGF2, SHH, HMGB3, DMNT1, HDAC2, or PRDX2, or wherein the protein is FGF10, FGF2, SHH, HMGB3, DMNT1, HDAC2, or PRDX2.

58. The research reagent or pharmaceutical composition according to Claim 56, wherein the gene encodes FGF10, FGF2, SHH, HMGB3, DMNT1, or HDAC2, or wherein the protein is FGF10, FGF2, SHH, HMGB3, DMNT1, or HDAC2.

59. The research reagent or pharmaceutical composition according to Claim 56, comprising a gene encoding at least one selected from FGF10, FGF2, SHH, HMGB3, DMNT1, HDAC2, and PRDX2, or comprising at least one protein selected from FGF10, FGF2, SHH, HMGB3, DMNT1, HDAC2, and PRDX2.

60. The research reagent or pharmaceutical composition according to Claim 56, comprising a gene encoding at least one selected from FGF10, FGF2, SHH, HMGB3, DMNT1, and HDAC2, or comprising at least one protein selected from FGF10, FGF2, SHH, HMGB3, DMNT1, and HDAC2.

61. The research reagent or pharmaceutical composition according to Claim 56, wherein the gene encodes FGF10, FGF2, FGF8, LEF1, SHH, MSX1, MSX2, LIN28A, or MEIS1, or wherein the protein is FGF10, FGF2, FGF8, LEF1, SHH, MSX1, MSX2, LIN28A, or MEIS1.

62. The research reagent or pharmaceutical composition according to Claim 56, comprising a gene encoding at least one selected from FGF10, FGF2, FGF8, LEF1, SHH, MSX1, MSX2, LIN28A, and MEIS1, or comprising at least one protein selected from FGF10, FGF2, FGF8, LEF1, SHH, MSX1, MSX2, LIN28A, and MEIS1.

63. The research reagent or pharmaceutical composition according to any one of Claims 57 to 62, wherein the pharmaceutical composition is for promoting endochondral ossification from articular cartilage or for promoting the growth of long bones.

64. The research reagent or pharmaceutical composition according to Claim 56, wherein the gene encodes FGF10 and SHH, or wherein the protein is FGF10 and SHH.

65. The research reagent or pharmaceutical composition according to Claim 56, wherein the gene encodes FGF10 or SHH, or wherein the protein is FGF10 or SHH.

66. A research reagent or pharmaceutical composition for use as a therapeutic agent to stimulate muscle tissue regeneration in mammals, comprising:
(1) A vector for transducing or acting on at least one gene that is relatively highly expressed in cells possessing tissue regenerative capacity;
(2) A vector for transducing or acting on at least one gene that functions as a master regulator gene during cellular differentiation and development;
(3) A vector for transducing or acting on at least one cytokine gene that is highly active in environments where tissue regeneration occurs; or
(4) A research reagent or pharmaceutical composition comprising at least one protein encoded by any of the genes listed in (1) to (3).

67. The research reagent or pharmaceutical composition according to Claim 66, wherein the gene encodes FGF10, FGF2, LEF1, SHH, MSX1, MSX2, LIN28A, or WNT7A, or wherein the protein is FGF10, FGF2, LEF1, SHH, MSX1, MSX2, LIN28A, or WNT7A.

68. The research reagent or pharmaceutical composition according to Claim 66, comprising a gene encoding at least one selected from FGF10, FGF2, LEF1, SHH, MSX1, MSX2, LIN28A, and WNT7A, or comprising at least one protein selected from FGF10, FGF2, LEF1, SHH, MSX1, MSX2, LIN28A, and WNT7A.

69. The research reagent or pharmaceutical composition according to Claim 66, wherein the gene encodes FGF10, FGF2, LEF1, SHH, HDAC2, PRDX2, or WNT7A, or wherein the protein is FGF10, FGF2, LEF1, SHH, HDAC2, PRDX2, or WNT7A.

70. The research reagent or pharmaceutical composition according to Claim 66, comprising a gene encoding at least one selected from FGF10, FGF2, LEF1, SHH, HDAC2, PRDX2, and WNT7A, or comprising at least one protein selected from FGF10, FGF2, LEF1, SHH, HDAC2, PRDX2, and WNT7A.

71. A research reagent or pharmaceutical composition for use as a therapeutic agent to stimulate adipose tissue regeneration in mammals, comprising:
(1) A vector for transducing or acting on at least one gene that is relatively highly expressed in cells possessing tissue regenerative capacity;
(2) A vector for transducing or acting on at least one gene that functions as a master regulator gene during cellular differentiation and development;
(3) A vector for transducing or acting on at least one cytokine gene that is highly active in environments where tissue regeneration occurs; or
(4) A research reagent or pharmaceutical composition comprising at least one protein encoded by any of the genes listed in (1) to (3).

72. The research reagent or pharmaceutical composition according to Claim 71, wherein the gene encodes FGF10, FGF20, FGF2, BMP5, LEF1, SHH, PRRX1, HDAC2, PRDX2, or PPARD, or wherein the protein is FGF10, FGF20, FGF2, BMP5, LEF1, SHH, PRRX1, HDAC2, PRDX2, or PPARD.

73. The research reagent or pharmaceutical composition according to Claim 71, comprising a gene encoding at least one selected from FGF10, FGF20, FGF2, BMP5, LEF1, SHH, PRRX1, HDAC2, PRDX2, and PPARD, or comprising at least one protein selected from FGF10, FGF20, FGF2, BMP5, LEF1, SHH, PRRX1, HDAC2, PRDX2, and PPARD.

74. The research reagent or pharmaceutical composition according to any one of Claims 45 to 73, wherein the mammal is a human, primate, rat, or mouse.

75. The research reagent or pharmaceutical composition according to Claim 74, wherein the mammal is a mouse.
